# EUROPEAN PATENT SPECIFICATION

(11) **EP 3 055 333 B1**
(45) Date of publication and mention of the grant of the patent: **04.12.2019**
(21) Application number: 14802527.3
(22) Date of filing: 10.10.2014
(51) Int. Cl.: C07K 16/40, A61K 39/395, A61P 3/06

(54) **USE OF A PCSK9 INHIBITOR TO TREAT HYPERLIPIDEMIA**
VERWENDUNG VON PCSK9-INHIBITOREN ZUR BEHANDLUNG VON HYPERLIPIDÄMIE
UTILISATION D'INHIBITEUR DE PCSK9 POUR TRAITER L'HYPERLIPIDÉMIE

(30) Priority: 11.10.2013 US 201361890154 P; 02.01.2014 US 201461923103 P; 19.03.2014 US 201461955514 P; 29.05.2014 US 201462004620 P; 16.07.2014 US 201462025104 P; 31.07.2014 EP 14306221; 24.09.2014 US 201462054571 P; 09.10.2014 EP 14306584
(43) Date of publication of application: 17.08.2016
(73) Proprietor: Sanofi Biotechnology, 75008 Paris (FR); Regeneron Pharmaceuticals, Inc., Tarrytown, NY 10591 (US)
(72) Inventor: BACCARA-DINET, Marie, F-75008 Paris (FR); BESSAC, Laurence, F-75008 Paris (FR); CHAUDHARI, Umesh, Bridgewater, NJ 08807 (US); HANOTIN, Corinne, F-75008 Paris (FR); PORDY, Robert, C., Tarrytown, NY 10591 (US); SASIELA, William, J., Tarrytown, NY 10591 (US); SCHWEMMER GIPE, Daniel A.,, Tarrytown, NY 10591 (US)
(74) Representative: Zwicker, Jörk
(86) International application number: PCT/US2014/060109
(87) International publication number: WO 2015/054619

(56) References cited:
- WO-A1-2010/077854
- WO-A1-2014/194111
- DAVID SULLIVAN ET AL: "Effect of a Monoclonal Antibody to PCSK9 on Low-Density Lipoprotein Cholesterol Levels in Statin-Intolerant Patients", JAMA, vol. 308, no. 23, 19 December 2012 (2012-12-19), page 2497, XP55136453, ISSN: 0098-7484, DOI: 10.1001/jama.2012.25790
- COSTET P: "PCSK9 inhibitors as LDL cholesterol-lowering agents: Rationale, concerns and preliminary outcomes", DRUGS OF THE FUTURE, PROUS SCIENCE, ES, vol. 37, no. 5, 1 May 2012 (2012-05-01), pages 331-341, XP009163740, ISSN: 0377-8282, DOI: 10.1358/DOF.2012.37.5.1790302

## Description

The present invention relates to the field of therapeutic treatments of diseases and disorders which are associated with elevated levels of lipids and lipoproteins. More specifically, the invention relates to the use of anti-PCSK9 antibodies or antigen-binding fragments thereof to treat patients with hyperlipidemia who are not on statin therapy, including patients who are statin non-responsive, poorly controlled with statin therapy, intolerant to statins, or who have a history of adverse reactions to statin therapy.

### BACKGROUND

Hypercholesterolemia, particularly an increase in low-density lipoprotein (LDL) cholesterol (LDL-C) levels, constitutes a major risk for the development of atherosclerosis and coronary heart disease (CHD) (Sharrett et al., 2001, Circulation 104:1108-1113). Low-density lipoprotein cholesterol is identified as the primary target of cholesterol lowering therapy and is accepted as a valid surrogate therapeutic endpoint. Numerous studies have demonstrated that reducing LDL-C levels reduces the risk of CHD with a strong direct relationship between LDL-C levels and CHD events; for each 1 mmol/L (∼40 mg/dL) reduction in LDL-C, cardiovascular disease (CVD) mortality and morbidity is lowered by 22%. Greater reductions in LDL-C produce greater reduction in events, and comparative data of intensive versus standard statin treatment suggest that the lower the LDL-C level, the greater the benefit in patients at very high cardiovascular (CV) risk.

Current LDL-C lowering medications include statins, cholesterol absorption inhibitors (e.g., ezetimibe [EZE]), fibrates, niacin, and bile acid sequestrants. While modifications in lifestyle and conventional drug treatment are often successful in reducing cholesterol levels, not all patients are able to achieve the recommended target cholesterol levels with such approaches. Various conditions, such as familial hypercholesterolemia (FH), appear to be resistant to lowering of LDL-C levels in spite of aggressive use of conventional therapy. Specifically, treatment with statins, which reduce LDL-C by inhibiting cholesterol synthesis and upregulating the hepatic LDL receptor, may have little effect in patients whose LDL receptors are non-existent or defective. Moreover, many patients are statin non-responsive, poorly controlled with statin therapy, cannot tolerate statins, and/or do not adhere to their prescribed therapeutic statin regimen due to statin-related side effects. Because hypercholesterolemia is largely asymptomatic, any unpleasant effects of pharmacologic agents used to manage this disorder can undermine patient compliance. In several cohort studies, the reported rate of adherence to statin therapy at 1 year ranged from 26% to 85%, with a rapid decline in adherence rates typically observed within the first few months. PCSK9 inhibition may be a promising strategy in lowering cholesterol associated with LDL to prevent cardiovascular diseases or attenuate their progression and damage (Costet P, "PCSK9 inhibitors as LDL cholesterol-lowering agents: Rationale, concerns and preliminary outcomes", Drugs of the Future. Vol. 37, No. 5, pages 331-341, May 1, 2012). The effect of a monoclonal antibody to PCSK9 in combination with ezetimibe on LDL-C levels in statin intolerant patients is evaluated in clinical trials, e.g. the GAUSS randomized trial (Sullivan et al. "Effect of a Monoclonal Antibody to PCSK9 on Low-Density Lipoprotein Cholesterol Levels in Statin-intolerant Patients", JAMA, Vol. 308, No. 23, Page 2497, December 19, 2012).

Accordingly, there exists a need in the art for alternative options for lowering LDL-C in patients.

### BRIEF SUMMARY OF THE INVENTION

The present invention provides a pharmaceutical composition comprising 75 mg or 150 mg of an antibody or antigen-binding fragment thereof which specifically binds human PCSK9 for use in the treatment of hypercholesterolemia in a patient who is intolerant to statins or who has a history of adverse reactions to statin therapy, wherein the antibody or antigen binding fragment thereof comprises the heavy and light chain CDRs having SEQ ID NOs:2, 3, 4, 7, 8 and 10, and wherein the antibody or antigen-binding fragment thereof is administered in the absence of statin therapy at a frequency of once every two or four weeks at a dose of 75 mg or of once every two or four weeks at a dose of 150 mg. Examples of adverse reactions to statin therapy include, *e.g.,* skeletal muscle pain, discomfort, weakness and/or cramping. Thus, according to certain embodiments, the present invention provides methods for reducing serum LDL-C levels in a patient without inducing skeletal muscle pain, discomfort, weakness or cramping, *e.g.,* by discontinuing the patient's statin therapeutic regimen and administering an anti-PCSK9 antibody or antigen-binding fragment thereof to the patient.

The present invention provides a pharmaceutical composition comprising 75 mg or 150 mg of an antibody or antigen-binding fragment thereof which specifically binds human PCSK9 for use in the treatment of hypercholesterolemia in a patient, wherein the patient has a moderate, high, or very high cardiovascular risk; and is intolerant to statins or has a history of adverse reactions to statin therapy, wherein one or more initial doses of the pharmaceutical composition comprising 75 mg of the antibody or antigen-binding fragment thereof is administered about every two weeks, and wherein (a) one or more further doses of the pharmaceutical composition comprising 75 mg of the antibody or antigen-binding fragment thereof is administered about every two weeks if the LDL-C level of the patient after the initial doses is lower than 70 mg/dL; or (b) one or more antigen-binding fragment thereof is administered about every two weeks if the LDL-C level of further doses of the pharmaceutical composition comprising 150 mg of the antibody or the patient after the initial doses is great than or equal to 70 mg/dL, wherein the antibody or antigen-binding fragment thereof comprises the heavy and light chain CDRs having SEQ ID NOs:2, 3, 4, 7, 8 and 10, and wherein after about 24 weeks of treatment with the antibody or antigen-binding fragment thereof, the improvement in the serum level of one or more lipid components is selected from the group consisting of:
(a) reduction of the patient's low density lipoprotein cholesterol (LDL-C) by at least 35%;
(b) reduction of the patient's apolipoprotein B (ApoB) by at least 25%;
(c) reduction of the patient's non-high density lipoproprotein cholesterol (non-HDL-C) by at least 30%;
(d) reduction of the patient's total cholesterol by at least 20%; and
(e) reduction of the patient's lipoprotein a (Lp(a)) by at least 15%.

According to one aspect, patients with hyperlipidemia include patients with non-familial hypercholesterolemia, heterozygous or homozygous familial hypercholesterolemia, or mixed dyslipidemia. In certain aspects, a patient with hyperlipidemia also has type 2 diabetes mellitus.

In some embodiments, the anti-PCSK9 antibody or antigen-binding fragment is administered to a patient as a monotherapy, in the absence of any other lipid modifying therapy. In some embodiments, the anti-PCSK9 antibody or antigen binding fragment is administered to a patient in combination with other non-statin lipid modifying therapy.

The present invention also provides methods for treating hypercholesterolemia in a patient who is intolerant to statins or who has a history of adverse reactions to statin therapy by selecting a patient with moderate, high, or very high cardiovascular risk who has previously experienced skeletal muscle-related symptoms that began or increased while on a daily therapeutic statin regimen and administering one or more doses of an anti-PCSK9 antibody or antigen-binding fragments thereof to the patient. According to certain embodiments, the patient is selected on the basis of having previously experienced skeletal muscle-related symptoms that began or increased while on at least two separate daily therapeutic statin regimens (e.g., wherein at least one of the daily therapeutic statin regimens is the lowest approved daily dose of a statin).

The present invention also provides pharmaceutical compositions comprising an anti-PCSK9 antibody or antigen-binding fragment thereof for use in treating patients with hypercholesterolemia who are not on statin therapy.

One embodiment provides a pharmaceutical composition for use in treating hypercholesterolemia in a patient in need thereof, comprising: (a) selecting a patient who is intolerant to statins or who has a history of adverse reactions to statin therapy; and (b) administering one or more doses of an anti-PCSK9 antibody or antigen-binding fragments thereof to the patient.

Another embodiment provides a pharmaceutical composition for use in reducing serum LDL-C levels in a patient without inducing skeletal muscle pain, discomfort, weakness or cramping, comprising: (a) selecting a patient who has experienced a skeletal muscle-related symptom that began or increased while taking a lowest approved daily dose of one or more statin; and (b) administering one or more doses of an anti-PCSK9 antibody or antigen-binding fragments thereof to the patient; thereby reducing serum LDL-C levels in the patient without inducing skeletal muscle pain, discomfort weakness or cramping.

Another embodiment provides a pharmaceutical composition for use in reducing serum LDL-C levels in a hypercholesterolemic, statin intolerant patient, comprising: (a) selecting a patient with moderate, high, or very high cardiovascular risk who is intolerant to statins or who has a history of adverse reactions to statin therapy; and (b) administering one or more doses of an anti-PCSK9 antibody or antigen-binding fragments thereof to the patient.

Another embodiment provides a pharmaceutical composition for use in treating hypercholesterolemia in a patient who is intolerant to statins or who has a history of adverse reactions to statin therapy, comprising: (a) selecting a patient with moderate, high, or very high cardiovascular risk who has previously experienced skeletal muscle-related symptoms that began or increased while on a daily therapeutic statin regimen; and (b) administering one or more doses of an anti-PCSK9 antibody or antigen-binding fragment thereof to the patient.

In some embodiments, the patient previously experienced skeletal muscle-related symptoms that began or increased while on at least two separate daily therapeutic statin regimens. In some embodiments, at least one of the daily therapeutic statin regimens is the lowest approved daily dose of a statin. In some embodiments, at least one of the daily therapeutic statin regimens is selected from the group consisting of: 5 mg rosuvastatin daily, 10 mg atorvastatin daily, 10 mg simvastatin daily, 20 mg lovastatin daily, 40 mg pravastatin daily, 40 mg fluvastatin daily, and 2 mg pitavastatin daily. In some embodiments, the anti-PCSK9 antibody or antigen-binding fragment thereof is administered to the patient in the absence of statin therapy.

One embodiment provides a pharmaceutical composition for use for eliminating statin usage in a hypercholesterolemic patient who is intolerant to statins while lowering the patient's serum LDL-C levels, the method comprising: (a) selecting a patient who is or was on a daily therapeutic statin regimen and who is intolerant to statins or who has a history of adverse reactions to statin therapy; (b) discontinuing the patient's daily therapeutic statin regimen; and (c) administering one or more doses of an anti-PCSK9 antibody or antigen-binding fragment thereof to the patient.

In some embodiments, the patient, prior to or at the time of administration of the anti-PCSK9 antibody or antigen-binding fragments thereof, exhibits hypercholesterolemia defined as a serum low-density lipoprotein cholesterol (LDL-C) level of greater than about 70 mg/dL. In some embodiments, the patient, prior to or at the time of administration of the anti-PCSK9 antibody or antigen-binding fragment thereof, exhibits hypercholesterolemia defined as a serum low-density lipoprotein cholesterol (LDL-C) level of greater than about 100 mg/dL.

In some embodiments, the patient has heterozygous Familial Hypercholesterolemia (heFH). In some embodiments, the patient has a form of hypercholesterolemia that is not Familial Hypercholesterolemia (non-FH). In some embodiments, the patient, prior to or at the time of administration of the anti-PCSK9 antibody or antigen-binding fragment thereof, has moderate cardiovascular risk defined as a calculated 1 0-year fatal cardiovascular disease risk SCORE greater than or equal to 1% and less than 5%. In some embodiments, the patient, prior to or at the time of administration of the anti-PCSK9 antibody or antigen-binding fragment thereof, has high cardiovascular risk defined as a calculated 10-year fatal cardiovascular disease risk SCORE greater than or equal to 5% along with one or more of: (i) moderate chronic kidney disease, (ii) type 1 diabetes mellitus without target organ damage, (iii) type 2 diabetes mellitus without target organ damage, and/or (iv) heFH. In some embodiments, the patient, prior to or at the time of administration of the anti-PCSK9 antibody or antigen-binding fragment thereof, has very high cardiovascular risk defined as one or more of: (i) documented coronary heart disease; (ii) ischemic stroke; (iii) peripheral stroke; (iv) peripheral arterial disease (PAD); (v) transient ischemic attack (TIA); (vi) abdominal aortic aneurysm; (vii) carotid artery occlusion >50% without symptoms; (viii) carotid endarterectomy; (ix) carotid artery stent procedure; (x) renal artery stenosis; (xi) renal artery stent procedure; (xii) type 1 diabetes mellitus with target organ damage; and/or (xiii) type 2 diabetes mellitus with target organ damage.

The anti-PCSK9 antibody or antigen-binding fragments thereof is an antibody or antigen-binding fragment thereof comprising heavy and light chain CDR amino acid sequences having SEQ ID NOs:2, 3, 4, 7, 8 and 10. In some embodiments, the antibody or antigen-binding fragment thereof comprises an HCVR having the amino acid sequence of SEQ ID NO:1 and an LCVR having the amino acid sequence of SEQ ID NO:6.

In some embodiments, the antibody or antigen-binding protein that specifically binds PCSK9 is administered to the patient at a dose of about 75 mg at a frequency of once every two weeks. In some embodiments, the about 75 mg dose is maintained if the patient's LDL-C measured after five or more doses is <70 mg/dL. In some embodiments, the about 75 mg dose is discontinued if the patient's LDL-C measured after five or more doses remains ≥70 mg/dL, and the antibody or antigen-binding fragment thereof that specifically binds PCSK9 is subsequently administered to the patient at a dose of about 150 mg at a frequency of once every two weeks. In some embodiments, the antibody or antigen-binding protein that specifically binds PCSK9 is administered to the patient at a dose of about 150 mg at a frequency of once every two weeks.

In some embodiments, the anti-PCSK9 antibody or antigen-binding fragment thereof is administered to the patient in combination with a non-statin lipid modifying therapy. In some embodiments, the non-statin lipid modifying therapy comprises a therapeutic agent selected from the group consisting of ezetimibe, a fibrate, niacin, an omega-3 fatty acid, and a bile acid resin.

In some embodiments, the pharmaceutical composition for use improves the serum levels of one or more lipid components selected from the group consisting of: (a) reduction of the patient's low density lipoprotein cholesterol (LDL-C) by at least 35%; (b) reduction of the patient's apolipoprotein B (ApoB) by at least 25%; (c) reduction of the patient's non-high density lipoproprotein cholesterol (non-HDL-C) by at least 30%; (d) reduction of the patient's total cholesterol by at least 20%; and (e) reduction of the patient's lipoprotein a (Lp(a)) by at least 15%.

One embodiment provides a pharmaceutical composition for use in improving the serum level of one or more lipid components in a hypercholesterolemic, statin intolerant patient, the method comprising: (a) selecting a patient with moderate, high, or very high cardiovascular risk who is intolerant to statins or who has a history of adverse reactions to statin therapy; and (b) administering multiple doses of an anti-PCSK9 antibody to the patient at a dosing amount of about 75 to 150 mg per dose, and a dosing frequency of about once every two weeks, wherein after about 24 weeks of treatment with the anti-PCSK9 antibody, the improvement in the serum level of one or more lipid components is selected from the group consisting of: (a) reduction of the patient's low density lipoprotein cholesterol (LDL-C) by at least 35%; (b) reduction of the patient's apolipoprotein B (ApoB) by at least 25%; (c) reduction of the patient's non-high density lipoproprotein cholesterol (non-HDL-C) by at least 30%; (d) reduction of the patient's total cholesterol by at least 20%; and (e) reduction of the patient's lipoprotein a (Lp(a)) by at least 15%.

Another embodiment of the invention provides pharmaceutical compositions for use in treating hypercholesterolemia in a patient in need thereof, comprising administering to the patient as a monotherapy a pharmaceutical composition comprising a anti-PCSK9 antibody or antigen-binding fragments thereof, wherein the composition is administered every two weeks and the patient is not concurrently taking another lipid modifying therapy, thereby treating the hypercholesterolemia in the patient.

Another embodiment of the invention provides pharmaceutical compositions for use in for reducing low-density lipoprotein cholesterol (LDL-C) in a patient in need thereof, comprising administering to the patient as a monotherapy a pharmaceutical composition comprising an anti-PCSK9 antibody or antigen-binding fragment thereof, wherein the composition is administered every two weeks and the patient is not concurrently taking another lipid modifying therapy, thereby reducing the LDL-C in the patient.

Another embodiment of the invention provides pharmaceutical compositions for use in maintaining constant low-density lipoprotein cholesterol (LDL-C) levels in a patient comprising administering to the patient as a monotherapy a pharmaceutical composition comprising an anti-PCSK9 antibody or antigen-binding fragment thereof at an initial dose of about 75 mg, wherein the composition is administered every two weeks, and wherein the patient is not concurrently taking another lipid lowering therapy, thereby maintaining constant LDL-C levels in the patient. In some embodiments, the anti-PCSK9 antibody or antigen-binding fragment thereof is administered to the patient for at least 24 weeks, and the patient's LDL-C levels are maintained constant for 20 weeks.

In some embodiments providing administration of an anti-PCSK9 antibody or antigen-binding fragment thereof as a monotherapy, the patient is intolerant to statins or has a history of adverse reactions to statin therapy. In some embodiments, the patient previously experienced skeletal muscle-related symptoms that began or increased while on at least two separate daily therapeutic statin regimens. In some embodiments, at least one of the daily therapeutic statin regimens is the lowest approved daily dose of a statin. In some embodiments, at least one of the daily therapeutic statin regimens is selected from the group consisting of: 5 mg rosuvastatin daily, 10 mg atorvastatin daily, 10 mg simvastatin daily, 20 mg lovastatin daily, 40 mg pravastatin daily, 40 mg fluvastatin daily, and 2 mg pitavastatin daily.

In some embodiments providing administration of an anti-PCSK9 antibody or antigen-binding fragment thereof as a monotherapy, the patient, prior to or at the time of administration of the anti-PCSK9 antibody or antigen-binding fragment thereof, exhibits hypercholesterolemia defined as a serum low-density lipoprotein cholesterol (LDL-C) level of greater than about 70 mg/dL.

In some embodiments providing administration of a anti-PCSK9 antibody or antigen-binding fragment thereof as a monotherapy, the patient, prior to or at the time of administration of the anti-PCSK9 antibody or antigen-binding fragment thereof, exhibits hypercholesterolemia defined as a serum low-density lipoprotein cholesterol (LDL-C) level of greater than about 100 mg/dL.

In some embodiments providing administration of an anti-PCSK9 antibody or antigen-binding fragment thereof as a monotherapy, the patient has heterozygous Familial Hypercholesterolemia (heFH). In some embodiments providing administration of an anti-PCSK9 antibody or antigen-binding fragment thereof as a monotherapy, the patient has a form of hypercholesterolemia that is not Familial Hypercholesterolemia (non-FH). In some embodiments, the patient, prior to or at the time of administration of the anti-PCSK9 antibody or antigen-binding fragment thereof, has moderate cardiovascular risk defined as a calculated 10-year fatal cardiovascular disease risk SCORE greater than or equal to 1% and less than 5%. In some embodiments, the patient, prior to or at the time of administration of the anti-PCSK9 antibody or antigen-binding fragment thereof, has high cardiovascular risk defined as a calculated 10-year fatal cardiovascular disease risk SCORE greater than or equal to 5% along with one or more of: (i) moderate chronic kidney disease, (ii) type 1 diabetes mellitus without target organ damage, (iii) type 2 diabetes mellitus without target organ damage, and/or (iv) heFH. In some embodiments, the patient, prior to or at the time of administration of the anti-PCSK9 antibody or antigen-binding fragment thereof, has very high cardiovascular risk defined as one or more of: (i) documented coronary heart disease; (ii) ischemic stroke; (iii) peripheral stroke; (iv) peripheral arterial disease (PAD); (v) transient ischemic attack (TIA); (vi) abdominal aortic aneurysm; (vii) carotid artery occlusion >50% without symptoms; (viii) carotid endarterectomy; (ix) carotid artery stent procedure; (x) renal artery stenosis; (xi) renal artery stent procedure; (xii) type 1 diabetes mellitus with target organ damage; and/or (xiii) type 2 diabetes mellitus with target organ damage.

In some embodiments providing administration of an anti-PCSK9 antibody or antigen-binding fragment thereof as a monotherapy, the anti-PCSK9 antibody or antigen-binding fragment thereof is an antibody or antigen-binding fragment thereof comprising a heavy and light chain CDR amino acid sequences having SEQ ID NOs:2, 3, 4, 7, 8 and 10. In some embodiments the antibody or antigen-binding fragment thereof comprises an HCVR having the amino acid sequence of SEQ ID NO:1 and an LCVR having the amino acid sequence of SEQ ID NO:6.

In some embodiments providing administration of an anti-PCSK9 antibody or antigen-binding fragment thereof as a monotherapy, the antibody or antigen-binding protein that specifically binds PCSK9 is administered to the patient at a dose of about 75 mg at a frequency of once every two weeks. In some embodiments the about 75 mg dose is maintained if the patient's LDL-C measured after five or more doses is <70 mg/dL. In some embodiments the about 75 mg dose is discontinued if the patient's LDL-C measured after five or more doses remains ≥70 mg/dL, and the antibody or antigen-binding fragment thereof that specifically binds PCSK9 is subsequently administered to the patient at a dose of about 150 mg at a frequency of once every two weeks. In some embodiments the antibody or antigen-binding protein that specifically binds PCSK9 is administered to the patient at a dose of about 150 mg at a frequency of once every two weeks.

In some embodiments providing administration of an anti-PCSK9 antibody or antigen-binding fragment thereof as a monotherapy, the method improves the serum levels of one or more lipid components selected from the group consisting of: (a) reduction of the patient's low density lipoprotein cholesterol (LDL-C) by at least 35%; (b) reduction of the patient's apolipoprotein B (ApoB) by at least 25%; (c) reduction of the patient's non-high density lipoproprotein cholesterol (non-HDL-C) by at least 30%; (d) reduction of the patient's total cholesterol by at least 20%; and (e) reduction of the patient's lipoprotein a (Lp(a)) by at least 15%.

One embodiment provides a pharmaceutical composition for use in reducing free PCSK9 levels in a patient comprising administering to the patient as a monotherapy a pharmaceutical composition comprising an anti-PCSK9 antibody or antigen-binding protein at a dose of about 75 mg, wherein the composition is administered every two weeks, and wherein the patient is not concurrently taking another lipid lowering therapy, thereby reducing free PCSK9 levels in the patient.

One embodiment provides a pharmaceutical compositions for use in improving the serum level of one or more lipid components in a patient in need thereof comprising administering multiple doses of an anti-PCSK9 antibody as a monotherapy to the patient at a dosing amount of about 75 to 150 mg per dose, at a dosing frequency of about once every two weeks, wherein the patient is not concurrently taking another lipid modifying therapy and wherein after about 24 weeks of treatment with the anti-PCSK9 antibody the improvement in the serum level of one or more lipid components is selected from the group consisting of: (a) reduction of the patient's low density lipoprotein cholesterol (LDL-C) by at least 35%; (b) reduction of the patient's apolipoprotein B (ApoB) by at least 25%; (c) reduction of the patient's non-high density lipoproprotein cholesterol (non-HDL-C) by at least 30%; (d) reduction of the patient's total cholesterol by at least 20%; and (e) reduction of the patient's lipoprotein a (Lp(a)) by at least 15%.

Other embodiments of the present invention will become apparent from a review of the ensuing detailed description.

### BRIEF DESCRIPTION OF THE FIGURES

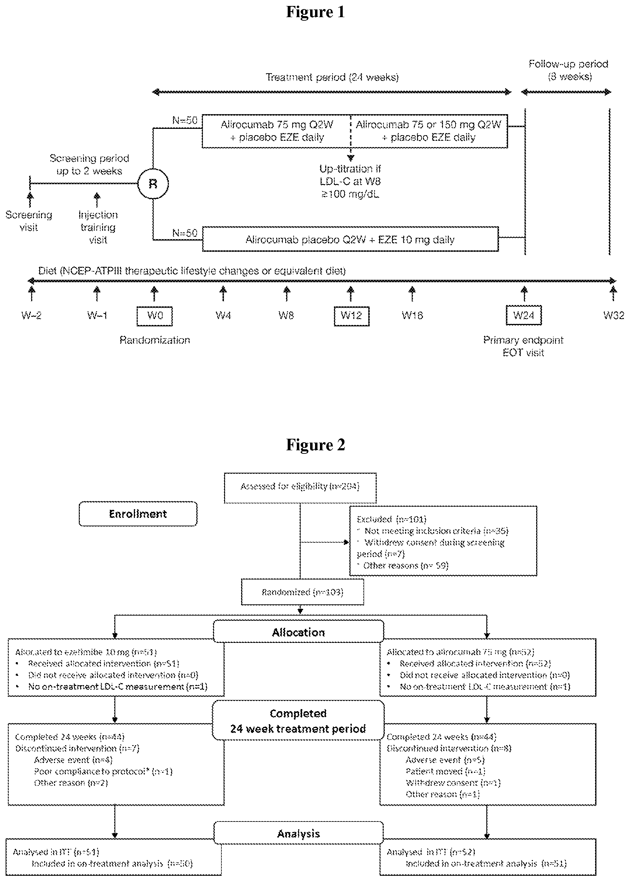

**Figure 1** shows the study design of the clinical trial described in Example 2. Although the protocol called for an LDL-C threshold of ≥100 mg/dL for up-titration, a threshold of ≥70 mg/dL was applied in a blinded manner in this study. Arrows along the bottom of the figure indicate assessment times. EOT, end of treatment; EZE, ezetimibe; LDL-C, low-density lipoprotein cholesterol; NCEP ATP III TCP, National Cholesterol Education Program Adult Treatment Panel III; Q2W, every 2 weeks; W, week.
**Figure 2** shows the patient disposition of the clinical trial described in Example 2. *Life events made continuing too difficult. ITT, intent-to-treat.

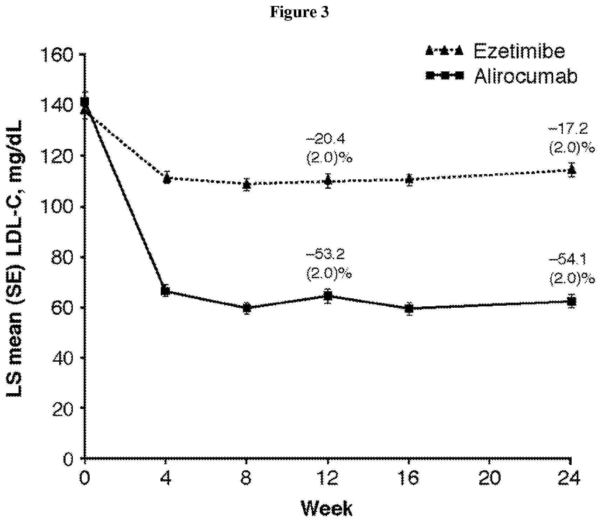

**Figure 3** is a graph that shows the LDL-C levels (mg/dL) versus study time point (on-treatment analysis) for the clinical trial described in Example 2. Values above week 12 and week 24 data points indicate LS mean (SE) % change from baseline. LDL-C, low-density lipoprotein cholesterol; LS, least squares; SE, standard error.

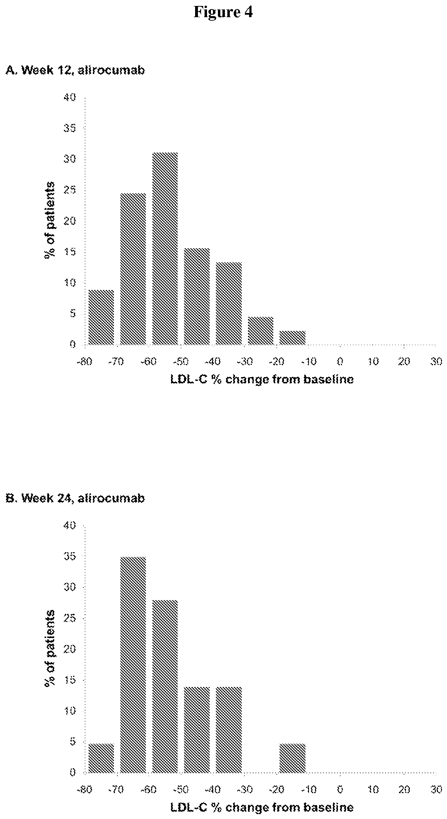

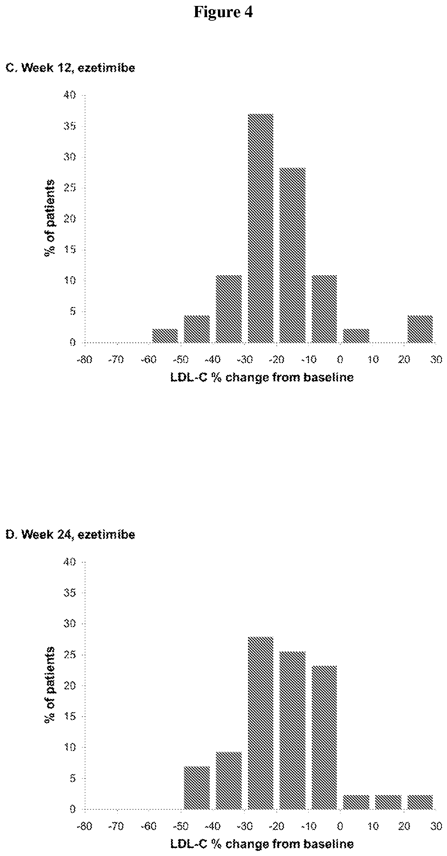

**Figure 4** is a group of four graphs showing the distribution of percentage changes in LDL-C from baseline to Week 12 **(****Figure 4A****)** and Week 24 **(****Figure 4B****)** in patients treated with mAb316P (Alirocumab), and Week 12 **(****Figure 4C****)** and Week 24 **(****Figure 4D****)** in patients treated with ezetimibe (On-Treatment Population) in the clinical trial described in Example 2.

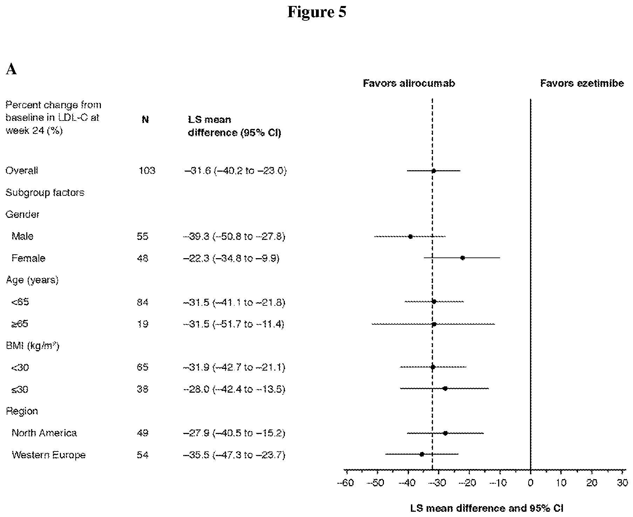

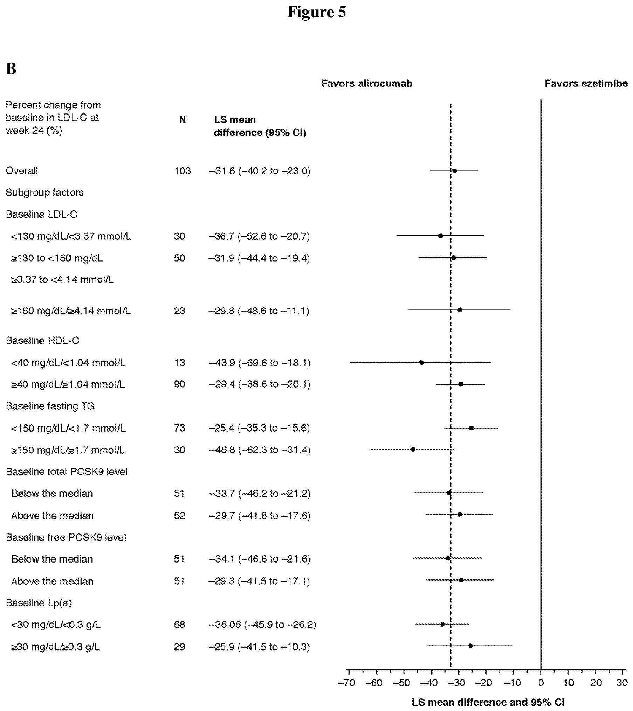

**Figure 5** is a group of two charts showing subgroup analyses of percent change from baseline in LDL-C at Week 24 according to demographics **(****Figure 5A****)** and other baseline characteristics **(****Figure 5B****)** (ITT Population) for the clinical trial described in Example 2.

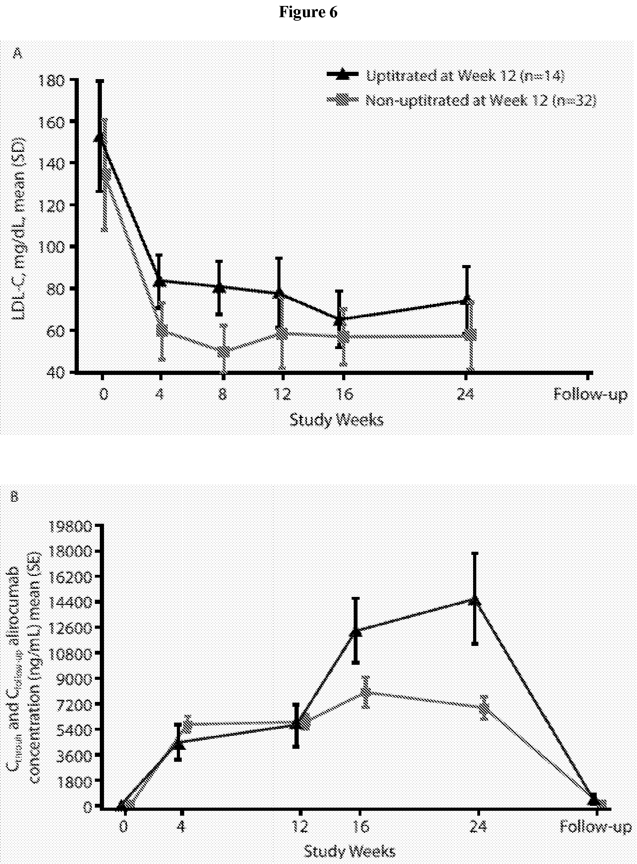

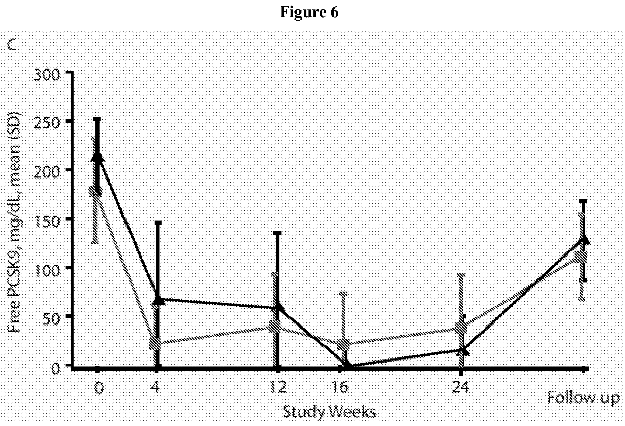

**Figure 6** shows a series of graphs that illustrate the mean LDL-C levels **(****Figure 6A****),** C_{trough} and C_{follow-up} mAb316P (Alirocumab) concentrations **(****Figure 6B****),** and free PCSK9 levels **(****Figure 6C****)** in mAb316P treated patients according to uptitration status in the clinical trial described in Example 2. C_{trough} values were taken 14±6 days after previous injection; C_{follow-up} values were taken > 21 days after last injection. Triangles represent the uptitrated group at Week 12. Squares represent the non-uptitrated group at Week 12.

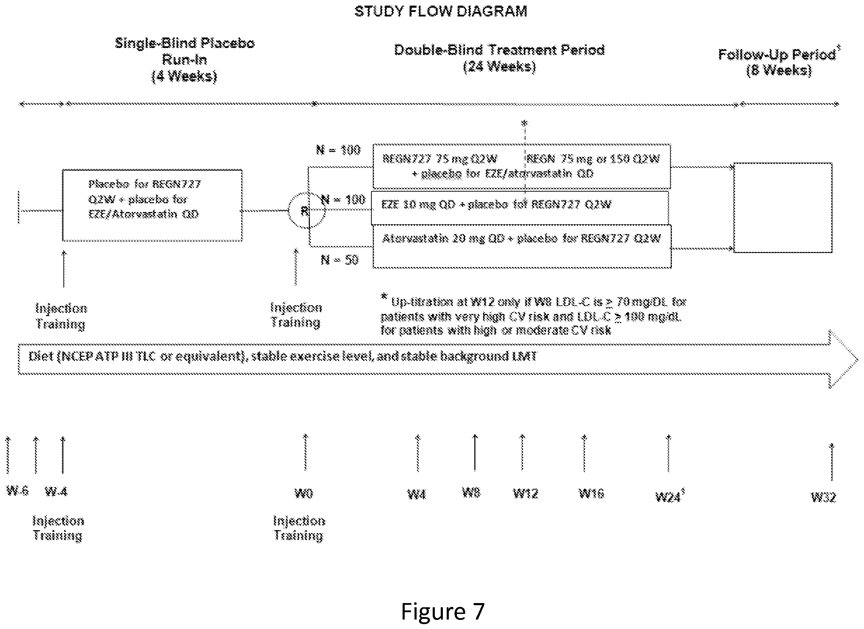

**Figure 7** is a Study Flow Diagram illustrating the clinical trial described in Example 3 herein. "REGN727" is a designation for the antibody referred to herein as alirocumab or mAb316P.

### DETAILED DESCRIPTION

Before the present invention is described, it is to be understood that this invention is not limited to particular methods and experimental conditions described, as such methods and conditions may vary. It is also to be understood that the terminology used herein is for the purpose of describing particular embodiments only, and is not intended to be limiting, since the scope of the present invention will be limited only by the appended claims.

### Definitions

Unless defined otherwise, all technical and scientific terms used herein have the same meaning as is commonly understood by one of ordinary skill in the art.

It is noted here that as used in this specification and the appended claims, the singular forms "a", "an", and "the" also include plural reference, unless the context clearly dictates otherwise.

The term "about" or "approximately," when used in reference to a particular recited numerical value, means that the value may vary from the recited value by no more than 1%. For example, as used herein, the expression "about 100" includes 99 and 101 and all values in between (*e.g.,* 99.1, 99.2, 99.3, 99.4, etc.).

The terms "administer" or "administration" refer to the act of injecting or otherwise physically delivering a substance as it exists outside the body (*e.g.,* a formulation of the invention) into a patient, such as by mucosal, intradermal, intravenous, subcutaneous, intramuscular delivery and/or any other method of physical delivery described herein or known in the art. When a disease, or a symptom thereof, is being treated, administration of the substance typically occurs after the onset of the disease or symptoms thereof. When a disease or symptoms thereof, are being prevented, administration of the substance typically occurs before the onset of the disease or symptoms thereof.

The terms "composition" and "formulation" are intended to encompass a product containing the specified ingredients (e.g., an anti-PCSK9 antibody) in, optionally, the specified amounts, as well as any product which results, directly or indirectly, from the combination of the specified ingredients in, optionally, the specified amounts.

The term "excipients" refers to inert substances that are commonly used as a diluent, vehicle, preservative, binder, stabilizing agent, etc. for drugs and includes, but is not limited to, proteins (e.g., serum albumin, etc.), amino acids *(e.g.,* aspartic acid, glutamic acid, lysine, arginine, glycine, histidine, etc.), fatty acids and phospholipids (*e.g.,* alkyl sulfonates, caprylate, etc.), surfactants (*e.g.,* SDS, polysorbate, nonionic surfactant, etc.), saccharides (*e.g.,* sucrose, maltose, trehalose, etc.) and polyols (*e.g.,* mannitol, sorbitol, etc.). See, also, Remington's Pharmaceutical Sciences (1990) Mack Publishing Co., Easton, Pa.

In the context of a peptide or polypeptide, the term "fragment" refers to a peptide or polypeptide that comprises less than the full length amino acid sequence. Such a fragment may arise, for example, from a truncation at the amino terminus, a truncation at the carboxy terminus, and/or an internal deletion of a residue(s) from the amino acid sequence. Fragments may, for example, result from alternative RNA splicing or from *in vivo* protease activity. In certain embodiments, PCSK9 fragments include polypeptides comprising an amino acid sequence of at least 50, at 100 amino acid residues, at least 125 contiguous amino acid residues, at least 150 contiguous amino acid residues, at least 175 contiguous amino acid residues, at least 200 contiguous amino acid residues, or at least 250 contiguous amino acid residues of the amino acid sequence of a PCSK9 polypeptide. In a specific embodiment, a fragment of a PCSK9 polypeptide or an antibody that specifically binds to a PCSK9 antigen retains at least 1, at least 2, or at least 3 functions of the full-length polypeptide or antibody.

The term "pharmaceutically acceptable" means being approved by a regulatory agency of the Federal or a state government, or listed in the U.S. Pharmacopeia, European Pharmacopeia or other generally recognized Pharmacopeia for use in animals, and more particularly in humans.

The terms "prevent", "preventing", and "prevention" refer to the total or partial inhibition of the development, recurrence, onset or spread of a PCSK9-mediated disease and/or symptom related thereto, resulting from the administration of a therapy or combination of therapies provided herein (*e.g.,* a combination of prophylactic or therapeutic agents).

The term "PCSK9 antigen" refers to that portion of a PCSK9 polypeptide to which an antibody specifically binds. A PCSK9 antigen also refers to an analog or derivative of a PCSK9 polypeptide or fragment thereof to which an antibody specifically binds. In some embodiments, a PCSK9 antigen is a monomeric PCSK9 antigen or a trimeric PCSK9 antigen. A region of a PCSK9 polypeptide contributing to an epitope may be contiguous amino acids of the polypeptide, or the epitope may come together from two or more non-contiguous regions of the polypeptide. The epitope may or may not be a three-dimensional surface feature of the antigen. A localized region on the surface of a PCSK9 antigen that is capable of eliciting an immune response is a PCSK9 epitope. The epitope may or may not be a three-dimensional surface feature of the antigen.

The term "human PCSK9," "hPCSK9" or "hPCSK9 polypeptide" and similar terms refer to the polypeptides ("polypeptides," "peptides" and "proteins" are used interchangeably herein) comprising the amino acid sequence of SEQ ID NO:198 and related polypeptides, including SNP variants thereof. Related polypeptides include allelic variants (*e.g.,* SNP variants); splice variants; fragments; derivatives; substitution, deletion, and insertion variants; fusion polypeptides; and interspecies homologs, preferably, which retain PCSK9 activity and/or are sufficient to generate an anti-PCSK9 immune response. Also encompassed are soluble forms of PCSK9 that are sufficient to generate an anti-PCSK9 immunological response. As those skilled in the art will appreciate, an anti-PCSK9 antibody can bind to a PCSK9 polypeptide, polypeptide fragment, antigen, and/or epitope, as an epitope is part of the larger antigen, which is part of the larger polypeptide fragment, which, in turn, is part of the larger polypeptide. hPCSK9 can exist in a trimeric (native) or monomeric (denatured) form.

The terms "PCSK9-mediated disease," "PCSK9-mediated condition," and "PCSK9-mediated disorder" are used interchangeably and refer to any disease that is completely or partially caused by or is the result of PCSK9, e.g., hPCSK9. In certain embodiments, PCSK9 is aberrantly (*e.g.,* highly) expressed. In some embodiments, PCSK9 may be aberrantly upregulated. In other embodiments, normal, aberrant, or excessive cell signaling is caused by binding of PCSK9 to a PCSK9 ligand. In certain embodiments, the PCSK9 ligand is a PCSK9 receptor. In certain embodiments, the PCSK9-mediated disease or condition is selected from the group consisting of: elevated total cholesterol levels; elevated low-density lipoprotein cholesterol (LDL-C) levels; hyperlipidemia; dyslipidemia; hypercholesterolemia, particularly hypercholesterolemia uncontrolled by statins, hypercholesterolemia, such as familial hypercholesterolemia or non-familial hypercholesterolemia, and hypercholesterolemia uncontrolled by statins; atherosclerosis; and cardiovascular diseases.

The terms "subject" and "patient" are used interchangeably. As used herein, a subject is preferably a mammal, such as a non-primate (*e.g.,* cows, pigs, horses, cats, dogs, rats, etc.) or a primate (*e.g.,* monkey and human), most preferably a human. In one embodiment, the subject is a mammal, preferably a human, having a PCSK9-mediated disease. In another embodiment, the subject is a mammal, preferably a human, at risk of developing a PCSK9-mediated disease.

The term "therapeutic agent" refers to any agent that can be used in the treatment, management or amelioration of a PCSK9-mediated disease and/or a symptom related thereto. In certain embodiments, the term "therapeutic agent" refers to a PCSK9 antibody for use of the invention. In certain other embodiments, the term "therapeutic agent" refers to an agent other than a PCSK9 antibody for use of the invention. Preferably, a therapeutic agent is an agent that is known to be useful for, or has been or is currently being used for the treatment, management or amelioration of a PCSK9-mediated disease or one or more symptoms related thereto.

The term "therapy" refers to any protocol, method, and/or agent that can be used in the prevention, management, treatment, and/or amelioration of a PCSK9-mediated disease (*e.g.,* atherosclerosis or hypercholesterolemia). In certain embodiments, the terms "therapies" and "therapy" refer to a biological therapy, supportive therapy, and/or other therapies useful in the prevention, management, treatment, and/or amelioration of a PCSK9-mediated disease known to one of skill in the art, such as medical personnel.

The terms "treat", "treatment", and "treating" refer to the reduction or amelioration of the progression, severity, and/or duration of a PCSK9-mediated disease (*e.g.,* atherosclerosis) resulting from the administration of one or more therapies (including, but not limited to, the administration of one or more prophylactic or therapeutic agents). In specific embodiments, such terms refer to the reduction or inhibition of the binding of PCSK9 to a PCSK9 ligand.

Although any methods and materials similar or equivalent to those described herein can be used in the practice of the present invention, the preferred methods and materials are now described.

### Patient Selection

The present invention includes pharmaceutical compositions for use, *inter alia,* for treating patients who have hypercholesterolemia who are not on statin therapy, including patients who are statin non-responsive, poorly controlled with statin therapy, intolerant to statins, or who have a history of adverse reactions to statin therapy.

The present invention comprise selecting patients that have, or are at risk of developing hypercholesterolemia and administering to these patients a pharmaceutical composition comprising an anti PCSK9 antibody or antigen-binding fragment thereof. For example, a patient may be selected for treatment with the methods of the present invention if the patient is diagnosed with or identified as being at risk of developing a hyperlipidemia condition such as, *e.g.,* heterozygous Familial Hypercholesterolemia (heFH), homozygous Familial Hypercholesterolemia (hoFH), Autosomal Dominant Hypercholesterolemia (ADH, *e.g.,* ADH associated with one or more gain-of-function mutations in the PCSK9 gene), non-Familial Hypercholesterolemia (nonFH), dyslipidemia, and mixed dyslipidemia. In certain aspects, the patient to be treated is indicated for LDL apheresis. In certain aspects, a patient with hyperlipidemia also has type 2 diabetes mellitus. In certain aspects, the patient to be treated is diagnosed with hypercholesterolemia and is statin intolerant, statin non-responsive, or statin uncontrolled. As used herein, hyperlipidemia includes primary hyperlipidemia, secondary hyperlipidemia, and Fredrickson phenotype classes I-V.

As used herein, the expression "a patient in need thereof' means a human or non-human animal that exhibits one or more symptoms or indicia of hyperlipidemia or who has been diagnosed with hyperlipidemia, or who otherwise would benefit from a reduction in total serum cholesterol, LDL, triglycerides, VLDL, lipoprotein(a) [Lp(a)], or who would benefit from an increase in HDL.

The present invention comprise selecting patients who are not currently on statin therapy. As used herein, a statin therapy is an inhibitor of HMG-CoA reductase and includes but is not limited to atorvastatin, cerivastatin, fluvastatin, lovastatin, mevastatin, pitavastatin, pravastatin, rosuvastatin, simvastatin, etc. In some embodiments, doses of an anti-PCSK9 antibody or antigen-binding fragment thereof are administered to a patient whose previous (or "background") statin therapy is discontinued prior to or concurrent with the administration of the first dose of the anti-PCSK9 antibody or antigen-binding fragment thereof.

According to certain embodiments, the patient may be selected on the basis of having moderate, high, or very high CV risk. Degree of CV risk may be assessed and expressed in terms of a calculated 10-year fatal cardiovascular disease (CVD) risk SCORE value, as defined by The Task Force for the Management of Dislipidaemias of the European Society of Cardiology (ESC) and the European Atherosclerosis Society (EAS), as set forth in the ESC/EAS Guidelines for the Management of Dislipidaemias, European Heart Journal, 2100; 32:1769-1818 (referred to herein as "ESC/EAS 2011"). As used herein, "moderate CV risk" means a calculated 1 0-year fatal CVD risk SCORE greater than or equal to 1% and less than 5%. As used herein, "high CV risk" means a calculated 1 0-year fatal CVD risk SCORE greater than or equal to 5%, and/or moderate kidney disease (CKD), and/or type 1 or type 2 diabetes mellitus without target organ damage, and/or heFH. As used herein, "very high CV risk" means a history of documented coronary heart disease (CHD), ischemic stroke, peripheral arterial disease (PAD), transient ischemic attack (TIA), abdominal aortic aneurysm, carotid artery occlusion greater than 50% without symptoms, carotid endarterectomy or carotid artery stent procedure, renal artery stenosis, renal artery stent procedure, and/or type 1 or type 2 diabetes mellitus with target organ damage.

According to certain embodiments, the patient may be selected on the basis of having a history of coronary heart disease (CHD). As used herein, a "history of CHD" (or "documented history of CHD") includes one or more of: (i) acute myocardial infarction (MI); (ii) silent MI; (iii) unstable angina; (iv) coronary revascularization procedure (e.g., percutaneous coronary intervention [PCI] or coronary artery bypass graft surgery [CABG]); and/or (v) clinically significant CHD diagnosed by invasive or non-invasive testing (such as coronary angiography, stress test using treadmill, stress echocardiography or nuclear imaging).

According to certain embodiments, the patient may be selected on the basis of having one or more additional risk factors selected from the group consisting of age (e.g., older than 40, 45, 50, 55, 60, 65, 70, 75, or 80 years), race, national origin, gender (male or female), exercise habits (e.g., regular exerciser, non-exerciser), other preexisting medical conditions (e.g., type-II diabetes, high blood pressure, etc.), and current medication status (e.g., currently taking beta blockers, niacin, ezetimibe, fibrates, omega-3 fatty acids, bile acid resins, etc.).

The present invention comprise administering one or more doses of an anti-PCSK9 antibody or antigen-binding fragment thereof to a patient who is not on statin therapy. Patients with hypercholesterolemia may not be on statin therapy because they are statin non-responsive, poorly controlled with statin therapy, intolerant to statins, have a history of adverse reactions to statin therapy, or for any other reason.

While modifications in lifestyle and conventional drug treatment are often successful in reducing cholesterol levels, not all patients are able to achieve the recommended target cholesterol levels with such approaches. Various conditions, such as familial hypercholesterolemia (FH), appear to be resistant to lowering of LDL-C levels in spite of aggressive use of conventional therapy. Homozygous and heterozygous familial hypercholesterolemia (hoFH, heFH) are conditions associated with premature atherosclerotic vascular disease. However, patients diagnosed with hoFH are largely unresponsive to conventional drug therapy and have limited treatment options. Specifically, treatment with statins, which reduce LDL-C by inhibiting cholesterol synthesis and upregulating the hepatic LDL receptor, may have little effect in patients whose LDL receptors are non-existent or defective. A mean LDL-C reduction of only less than about 20% has been recently reported in patients with genotype-confirmed hoFH treated with the maximal dose of statins. The addition of ezetimibe 10 mg/day to this regimen resulted in a total reduction of LDL-C levels of 27%, which is still far from optimal. Likewise, many patients are statin non-responsive or poorly controlled with statin therapy.

In some aspects, the present invention comprise administering one or more doses of an anti-PCSK9 antibody or antigen-binding fragment thereof to a patient who is "statin intolerant" or "intolerant to statins." As used herein, a patient is regarded as "statin intolerant" or "intolerant to statins" if the patient has a history of experiencing one or more adverse reactions that began or increased while on a daily statin therapeutic regimen and stopped when statin therapy was discontinued. In certain embodiments, the adverse reactions are musculoskeletal in nature, *e.g.,* skeletal muscle pain, aches, weakness or cramping *(e.g.,* myalgia, myopathy, rhabdomyolysis, etc.). In certain embodiments, the adverse reactions are skeletal muscle pain or aches that occur or are intensified following exercise or exertion. Statin-related adverse reactions also include hepatic, gastrointestinal and psychiatric symptoms that correlate with statin administration. According to certain embodiments, a patient is deemed "statin intolerant" or "intolerant to statins" if the patient has a history of skeletal muscle-related symptoms associated with at least two different and separate daily statin therapeutic regimens. According to certain embodiments, a patient is "statin intolerant" or "intolerant to statins" if the patient exhibits one or more statin-related adverse reaction(s) to the lowest approved daily doses of one or more statins. In certain embodiments, a patient is "statin intolerant" or "intolerant to statins" if the patient is unable to tolerate a cumulative weekly statin dose of seven times the lowest approved tablet size. According to other embodiments of the present invention, a patient is "statin intolerant" or "intolerant to statins" if the patient is able to tolerate a low dose statin therapy but develops symptoms when the dose is increased (e.g., to achieve a targeted LDL-C level).

According to the present invention, "a history of skeletal muscle-related symptoms associated with taking at least two different and separate statins" includes skeletal muscle-related pain, aches, weakness and/or cramping, that began or increased during statin therapy and stopped when statin therapy was discontinued. In the context of the present invention, exemplary statin therapies associated with statin intolerance may include daily therapeutic statin regimens selected from the group consisting of: 5 mg rosuvastatin daily, 10 mg atorvastatin daily, 10 mg simvastatin daily, 20 mg lovastatin daily, 40 mg pravastatin daily, 40 mg fluvastatin daily, and 2 mg pitavastatin daily.

Patients who may be treated with the pharmaceutical composition of the present invention may be receiving one or more other non-statin lipid modifying therapies. Alternatively, they may be receiving no other lipid modifying therapy; in this instance, the administration of the anti-PCSK9 antibody or antigen-binding fragment thereof may be described as a monotherapy. As used herein, the use of the anti-PCSK9 antibody or antigen-binding fragment thereof as a "monotherapy" means in the absence of any other concurrent lipid modifying therapy.

### Methods for Treating Hyperlipidemia and Reducing Serum LDL-C Levels

According to certain embodiments, the patient who is treatable by the methods of the present invention has hypercholesterolemia (sometimes referred to herein as "a hypercholesterolemic patient"). "Hypercholesterolemia," as used herein, includes a serum LDL-C concentration of greater than or equal to 70 mg/dL, or a serum LDL-C concentration greater than or equal to 100 mg/dL, depending on the patient's cardiovascular risk ("CV risk"). For example, for patients with a very high CV risk (as defined elsewhere herein), the patient is regarded as having hypercholesterolemia if the patient's serum LDL-C concentration is greater than or equal to about 70 mg/dL. For patients with moderate or high CV risk (as defined elsewhere herein), the patient is regarded as having hypercholesterolemia if the patient's serum LDL-C concentration is greater than or equal to about 100 mg/dL.

Hypercholesterolemia, for purposes of the present invention, includes heterozygous Familial Hypercholesterolemia (heFH), homozygous Familial Hypercholesterolemia (hoFH), Autosomal Dominant Hypercholesterolemia (ADH, *e.g.,* ADH associated with one or more gain-of-function mutations in the PCSK9 gene), as well as incidences of hypercholesterolemia that are distinct from Familial Hypercholesterolemia (nonFH).

The present invention includes pharmaceutical compositions for reducing serum LDL-C levels in a patient. The patient may be a hypercholesterolemic, statin intolerant patient, or any other patient for whom a reduction in serum LDL-C is deemed beneficial or desirable. Similarly, the present invention includes pharmaceutical compositions for reducing serum LDL-C levels in a patient without inducing skeletal muscle pain, discomfort, weakness, or cramping. As used in this context, "reducing serum LDL-C levels" means causing the patient's serum LDL-C level to decrease by at least 10% (*e.g.,* at least 15%, 20%, 25%, 30%, 35%, 40%, 45%, 50%, 55%, 60%, 65%, 70%, 75% or more).

### Methods for Eliminating or Reducing Statin Usage

The present invention includes a pharmaceutical composition for use, *inter alia,* for eliminating or reducing statin usage in a patient with hypercholesterolemia, including a hypercholesterolemic patient, *e.g.,* a hypercholesterolemic patient who is intolerant to statins. The methods according to this aspect of the invention comprise: (a) selecting a patient who is or was on a daily therapeutic statin regimen and who is intolerant to statins or who has a history of adverse reactions to statin therapy; and (b) discontinuing or reducing the patient's daily therapeutic statin regimen; and (c) administering one or more doses of an anti-PCSK9 antibody or antigen-binding fragment thereof to the patient. According to certain embodiments of this aspect of the invention, the patient's daily therapeutic statin regimen may be completely discontinued at the time of or just prior to commencement of a therapeutic course of treatment comprising administration of one or more doses of an anti-PCSK9 antibody or antigen-binding fragment thereof to the patient. In other embodiments, the patient's daily therapeutic statin regimen may be gradually reduced at the time of or just prior to commencement of a therapeutic course of treatment comprising administration of one or more doses of an anti-PCSK9 antibody or antigen-binding fragment thereof to the patient. Gradual reduction of a statin regimen, in the context of this aspect of the invention, may comprise reducing the quantity of statin administered to a patient, and/or decreasing the frequency of administration of statin to the patient. Gradual reduction of a statin regimen, according to this aspect of the invention, may result in complete elimination of statin usage by the patient while the patient is receiving an anti-PCSK9 antibody or antigen-binding fragment thereof in place of the statin. In this respect, the adverse effects of statins on a patient are reduced or eliminated by reducing or eliminating statin usage by the patient, while still permitting adequate treatment of hypercholesterolemia in the patient by administration of an anti-PCSK9 antibody or antigen-binding fragment thereof.

### Therapeutic Efficacy

The pharmaceutical composition of the present invention result in the reduction in serum levels of one or more lipid component selected from the group consisting of LDL-C, ApoB100, non-HDL-C, total cholesterol, VLDL-C, triglycerides, Lp(a) and/or remnant cholesterol, and increasing ApoA-1.

According to certain embodiments of the present invention, administration of a pharmaceutical composition comprising an anti-PCSK9 antibody or antigen-binding fragment thereof to a patient with hypercholesterolemia as a monotherapy, in the absence of any other lipid modifying therapy, will result in a mean percent reduction from baseline in serum low density lipoprotein cholesterol (LDL-C) of at least about 25%, 30%, 40%, 45%, 50%, 60%, or greater; a mean percent reduction from baseline in ApoB100 of at least about 25%, 30%, 40%, 50%, 60%, or greater; a mean percent reduction from baseline in non-HDL-C of at least about 25%, 30%, 40%, 50%, 60%, or greater; a mean percent reduction from baseline in total cholesterol of at least about 10%, 15%, 20%, 25%, 30%, 35%, or greater; and/or a mean percent increase from baseline in ApoA-1 of at least about 2%, 3%, 4%, 5%, 6%, 7%, 8%, 9%, 10%, or greater. The percent reductions in the various lipid parameters as set forth above may be achieved at 1, 2, 3, 4, 5, 6, 7, 8, 9, 10, 11, 12, 14, 16, 18, 20, 22, 24, 26, 28, 30, or more weeks after the commencement of a therapeutic regimen comprising the administration of an anti-PCSK9 antibody or antigen-binding fragment thereof as disclosed herein (*e.g.,* 75 mg or 150 mg mAb316P administered once every two weeks, or other similar administration regimens; see, *e.g.,* Example 2 herein).

According to certain specific embodiments, the present invention includes a pharmaceutical composition for reducing serum LDL-C levels in a hypercholesterolemic patient in the absence of any other lipid modifying therapy. The pharmaceutical compositions used according to this aspect of the invention comprise: (a) selecting a patient with LDL-C between 100 mg/dL (2.59 mmol/L) and 190 mg/dL (4.9 mmol/L) who is at moderate cardiovascular risk and is not receiving any other lipid modifying therapy ; and (b) administering multiple doses of an anti-PCSK9 antibody to the patient at a dosing amount of about 75 to 150 mg per dose, and a dosing frequency of about once every two weeks, wherein after about 24 weeks of treatment with the anti-PCSK9 antibody, the patient exhibits one or more lipid parameter improvements selected from the group consisting of: a reduction in LDL-C level from baseline of about 47%, a reduction in non-HDL-C level from baseline of about 41%, a reduction in Apo B level from baseline of about 37%, and/or a reduction in total cholesterol level from baseline of about 30%. Pharmaceutical compositions for use according to this aspect of the invention may comprise discontinuing the patient's background lipid modifying therapy prior to or concurrent with commencement of treatment with the anti-PCSK9 antibody.

Pharmaceutical compositions for use of the present invention result in the reduction in serum levels of one or more lipid component selected from the group consisting of LDL-C, ApoB100, non-HDL-C, total cholesterol, VLDL-C, triglycerides, Lp(a) and/or remnant cholesterol. For example, according to certain embodiments of the present invention, administration of a pharmaceutical composition comprising an anti-PCSK9 antibody or antigen-binding fragment thereof to a patient with hypercholesterolemia who is intolerant to statins or who has a history of adverse reactions to statin therapy (also referred to herein as a "hypercholesterolemic, statin-intolerant patient") will result in a mean percent reduction from baseline in serum low density lipoprotein cholesterol (LDL-C) of at least about 25%, 30%, 40%, 45%, 50%, 60%, or greater; a mean percent reduction from baseline in ApoB100 of at least about 25%, 30%, 40%, 50%, 60%, or greater; a mean percent reduction from baseline in non-HDL-C of at feast about 25%, 30%, 40%, 50%, 60%, or greater; a mean percent reduction from baseline in total cholesterol of at least about 10%, 15%, 20%, 25%, 30%, 35%, or greater; a mean percent reduction from baseline in VLDL-C of at least about 5%, 10%, 15%, 20%, 25%, 30%, or greater; a mean percent reduction from baseline in triglycerides (*e.g.,* fasting triglycerides) of at least about 5%, 10%, 15%, 20%, 25%, 30%, 35% or greater; and/or a mean percent reduction from baseline in Lp(a) of at least about 5%, 10%, 15%, 20%, 25%, or greater. The percent reductions in the various lipid parameters as set forth above may be achieved at 1, 2, 3, 4, 5, 6, 7, 8, 9, 10, 11, 12, 14, 16, 18, 20, 22, 24, 26, 28, 30, or more weeks after the commencement of a therapeutic regimen comprising the administration of an anti-PCSK9 antibody or antigen-binding fragment thereof as disclosed herein (*e.g*., 75 mg or 150 mg mAb316P administered once every two weeks, or other similar administration regimens; *see, e.g.,* Example 3 herein).

According to certain specific embodiments, the present invention includes pharmaceutical compositions for use in reducing serum LDL-C levels in a hypercholesterolemic, statin intolerant patient. The pharmaceutical compositions for use according to this aspect of the invention comprise: (a) selecting a patient with moderate, high, or very high cardiovascular risk who is intolerant to statins or who has a history of adverse reactions to statin therapy; and (b) administering multiple doses of an anti-PCSK9 antibody to the patient at a dosing amount of about 75 to 150 mg per dose, and a dosing frequency of about once every two weeks, wherein after about 24 weeks of treatment with the anti-PCSK9 antibody, the patient exhibits one or more lipid parameter improvements selected from the group consisting of: a reduction in LDL-C level from baseline of about 45%, a reduction in non-HDL-C level from baseline of about 40%, a reduction in Apo B level from baseline of about 36%, and/or a reduction in Lp(a) level from baseline of about 26%. Pharmaceutical compositions for use according to this aspect of the invention may comprise discontinuing the patient's background statin therapy prior to or concurrent with commencement of treatment with the anti-PCSK9 antibody.

### PCSK9 Antibodies

The pharmaceutical compositions for use of the present invention comprise administering to a patient a therapeutic composition comprising an anti-PCSK9 antibody or antigen-binding fragment thereof. As used herein, an "anti-PCSK9 antibody" is an agent which binds to or interacts with human PCSK9 and inhibits the normal biological function of PCSK9 *in vitro* or *in vivo.* Examples of anti-PCSK9 antibodies or antigen-binding fragments thereof include antibodies or antigen-binding fragments of antibodies that specifically bind human PCSK9.

The term "human proprotein convertase subtilisin/kexin type 9" or "human PCSK9" or "hPCSK9", as used herein, refers to PCSK9 encoded by the nucleic acid sequence shown in SEQ ID NO:197 and comprising the amino acid sequence of SEQ ID NO:198, or a biologically active fragment thereof.

The term "antibody", as used herein, is intended to refer to immunoglobulin molecules comprising four polypeptide chains, two heavy (H) chains and two light (L) chains inter-connected by disulfide bonds, as well as multimers thereof (*e.g*., IgM). Each heavy chain comprises a heavy chain variable region (abbreviated herein as HCVR or V_{H}) and a heavy chain constant region. The heavy chain constant region comprises three domains, C_{H}1, C_{H}2 and C_{H}3. Each light chain comprises a light chain variable region (abbreviated herein as LCVR or V_{L}) and a light chain constant region. The light chain constant region comprises one domain (C_{L}1). The V_{H} and V_{L} regions can be further subdivided into regions of hypervariability, termed complementarity determining regions (CDRs), interspersed with regions that are more conserved, termed framework regions (FR). Each V_{H} and V_{L} is composed of three CDRs and four FRs, arranged from amino-terminus to carboxy-terminus in the following order: FR1, CDR1, FR2, CDR2, FR3, CDR3, FR4. In different embodiments of the invention, the FRs of the anti-PCSK9 antibody (or antigen-binding portion thereof) may be identical to the human germline sequences, or may be naturally or artificially modified. An amino acid consensus sequence may be defined based on a side-by-side analysis of two or more CDRs.

The term "antibody," as used herein, also includes antigen-binding fragments of full antibody molecules. The terms "antigen-binding portion" of an antibody, "antigen-binding fragment" of an antibody, and the like, as used herein, include any naturally occurring, enzymatically obtainable, synthetic, or genetically engineered polypeptide or glycoprotein that specifically binds an antigen to form a complex. Antigen-binding fragments of an antibody may be derived, e.g., from full antibody molecules using any suitable standard techniques such as proteolytic digestion or recombinant genetic engineering techniques involving the manipulation and expression of DNA encoding antibody variable and optionally constant domains. Such DNA is known and/or is readily available from, *e.g.,* commercial sources, DNA libraries (including, *e.g.,* phage-antibody libraries), or can be synthesized. The DNA may be sequenced and manipulated chemically or by using molecular biology techniques, for example, to arrange one or more variable and/or constant domains into a suitable configuration, or to introduce codons, create cysteine residues, modify, add or delete amino acids, etc.

Non-limiting examples of antigen-binding fragments include: (i) Fab fragments; (ii) F(ab')2 fragments; (iii) Fd fragments; (iv) Fv fragments; (v) single-chain Fv (scFv) molecules; (vi) dAb fragments; and (vii) minimal recognition units consisting of the amino acid residues that mimic the hypervariable region of an antibody (e.g., an isolated complementarity determining region (CDR) such as a CDR3 peptide), or a constrained FR3-CDR3-FR4 peptide. Other engineered molecules, such as domain-specific antibodies, single domain antibodies, domain-deleted antibodies, chimeric antibodies, CDR-grafted antibodies, diabodies, triabodies, tetrabodies, minibodies, nanobodies (e.g. monovalent nanobodies, bivalent nanobodies, etc.), small modular immunopharmaceuticals (SMIPs), and shark variable IgNAR domains, are also encompassed within the expression "antigen-binding fragment," as used herein.

An antigen-binding fragment of an antibody will typically comprise at least one variable domain. The variable domain may be of any size or amino acid composition and will generally comprise at least one CDR which is adjacent to or in frame with one or more framework sequences. In antigen-binding fragments having a V_{H} domain associated with a V_{L} domain, the V_{H} and V_{L} domains may be situated relative to one another in any suitable arrangement. For example, the variable region may be dimeric and contain V_{H}-V_{H}, V_{H}-V_{L} or V_{L}-V_{L} dimers. Alternatively, the antigen-binding fragment of an antibody may contain a monomeric V_{H} or V_{L} domain.

In certain embodiments, an antigen-binding fragment of an antibody may contain at least one variable domain covalently linked to at least one constant domain. Non-limiting, exemplary configurations of variable and constant domains that may be found within an antigen-binding fragment of an antibody of the present invention include: (i) V_{H}-C_{H}1; (ii) V_{H}-C_{H}2; (iii) V_{H}-C_{H}3; (iv) V_{H}-C_{H}1-C_{H}2; (v) V_{H}-C_{H}1-C_{H}2-C_{H}3; (vi) V_{H}-C_{H}2-C_{H}3; (vii) V_{H}-C_{L}; (viii) V_{L}-C_{H}1; (ix) V_{L}-C_{H}2; (x) V_{L-}C_{H}3; (xi) V_{L}-C_{H}1-C_{H}2; (xii) V_{L}-C_{H}1-C_{H}2-C_{H}3; (xiii) V_{L}-C_{H}2-C_{H}3; and (xiv) V_{L}-C_{L}. In any configuration of variable and constant domains, including any of the exemplary configurations listed above, the variable and constant domains may be either directly linked to one another or may be linked by a full or partial hinge or linker region. A hinge region may consist of at least 2 (*e.g.,* 5, 10, 15, 20, 40, 60 or more) amino acids which result in a flexible or semi-flexible linkage between adjacent variable and/or constant domains in a single polypeptide molecule. Moreover, an antigen-binding fragment of an antibody of the present invention may comprise a homo-dimer or hetero-dimer (or other multimer) of any of the variable and constant domain configurations listed above in non-covalent association with one another and/or with one or more monomeric V_{H} or V_{L} domain (*e.g.,* by disulfide bond(s)).

As with full antibody molecules, antigen-binding fragments may be monospecific or multispecific (*e.g.,* bispecific). A multispecific antigen-binding fragment of an antibody will typically comprise at least two different variable domains, wherein each variable domain is capable of specifically binding to a separate antigen or to a different epitope on the same antigen. Any multispecific antibody format, including the exemplary bispecific antibody formats disclosed herein, may be adapted for use in the context of an antigen-binding fragment of an antibody for use in the present invention using routine techniques available in the art.

The constant region of an antibody is important in the ability of an antibody to fix complement and mediate cell-dependent cytotoxicity. Thus, the isotype of an antibody may be selected on the basis of whether it is desirable for the antibody to mediate cytotoxicity.

The term "human antibody", as used herein, is intended to include antibodies having variable and constant regions derived from human germline immunoglobulin sequences. The human antibodies for use in the invention may nonetheless include amino acid residues not encoded by human germline immunoglobulin sequences (e.g., mutations introduced by random or site-specific mutagenesis *in vitro* or by somatic mutation *in vivo*)*,* for example in the CDRs and in particular CDR3. However, the term "human antibody", as used herein, is not intended to include antibodies in which CDR sequences derived from the germline of another mammalian species, such as a mouse, have been grafted onto human framework sequences.

The term "recombinant human antibody", as used herein, is intended to include all human antibodies that are prepared, expressed, created or isolated by recombinant means, such as antibodies expressed using a recombinant expression vector transfected into a host cell (described further below), antibodies isolated from a recombinant, combinatorial human antibody library (described further below), antibodies isolated from an animal (e.g., a mouse) that is transgenic for human immunoglobulin genes (see e.g., Taylor et al. (1992) Nucl. Acids Res. 20:6287-6295) or antibodies prepared, expressed, created or isolated by any other means that involves splicing of human immunoglobulin gene sequences to other DNA sequences. Such recombinant human antibodies have variable and constant regions derived from human germline immunoglobulin sequences. In certain embodiments, however, such recombinant human antibodies are subjected to *in vitro* mutagenesis (or, when an animal transgenic for human Ig sequences is used, *in vivo* somatic mutagenesis) and thus the amino acid sequences of the V_{H} and V_{L} regions of the recombinant antibodies are sequences that, while derived from and related to human germline V_{H} and V_{L} sequences, may not naturally exist within the human antibody germline repertoire *in vivo.*

Human antibodies can exist in two forms that are associated with hinge heterogeneity. In one form, an immunoglobulin molecule comprises a stable four chain construct of approximately 150-160 kDa in which the dimers are held together by an interchain heavy chain disulfide bond. In a second form, the dimers are not linked via inter-chain disulfide bonds and a molecule of about 75-80 kDa is formed composed of a covalently coupled light and heavy chain (half-antibody). These forms have been extremely difficult to separate, even after affinity purification.

The frequency of appearance of the second form in various intact IgG isotypes is due to, but not limited to, structural differences associated with the hinge region isotype of the antibody. A single amino acid substitution in the hinge region of the human IgG4 hinge can significantly reduce the appearance of the second form (Angal et al. (1993) Molecular Immunology 30:105) to levels typically observed using a human IgG1 hinge. The instant invention encompasses antibodies having one or more mutations in the hinge, C_{H}2 or C_{H}3 region which may be desirable, for example, in production, to improve the yield of the desired antibody form.

An "isolated antibody," as used herein, means an antibody that has been identified and separated and/or recovered from at least one component of its natural environment. For example, an antibody that has been separated or removed from at least one component of an organism, or from a tissue or cell in which the antibody naturally exists or is naturally produced, is an "isolated antibody" for purposes of the present invention. An isolated antibody also includes an antibody *in situ* within a recombinant cell. Isolated antibodies are antibodies that have been subjected to at least one purification or isolation step. According to certain embodiments, an isolated antibody may be substantially free of other cellular material and/or chemicals.

The term "specifically binds," or the like, means that an antibody or antigen-binding fragment thereof forms a complex with an antigen that is relatively stable under physiologic conditions. Methods for determining whether an antibody specifically binds to an antigen are well known in the art and include, for example, equilibrium dialysis, surface plasmon resonance, and the like. For example, an antibody that "specifically binds" PCSK9, as used in the context of the present invention, includes antibodies that bind PCSK9 or portion thereof with a K_{D} of less than about 1000 nM, less than about 500 nM, less than about 300 nM, less than about 200 nM, less than about 100 nM, less than about 90 nM, less than about 80 nM, less than about 70 nM, less than about 60 nM, less than about 50 nM, less than about 40 nM, less than about 30 nM, less than about 20 nM, less than about 10 nM, less than about 5 nM, less than about 4 nM, less than about 3 nM, less than about 2 nM, less than about 1 nM or less than about 0.5 nM, as measured in a surface plasmon resonance assay. An isolated antibody that specifically binds human PCSK9, however, have cross-reactivity to other antigens, such as PCSK9 molecules from other (non-human) species.

The anti-PCSK9 antibodies comprised in the pharmaceutical compositions for use in the present invention may comprise one or more amino acid substitutions, insertions and/or deletions in the framework and/or CDR regions of the heavy and light chain variable domains as compared to the corresponding germline sequences from which the antibodies were derived. Such mutations can be readily ascertained by comparing the amino acid sequences disclosed herein to germline sequences available from, for example, public antibody sequence databases. The present invention includes pharmaceutical compositions for use comprising antibodies, and antigen-binding fragments thereof, which are derived from any of the amino acid sequences disclosed herein, wherein one or more amino acids within one or more framework and/or CDR regions are mutated to the corresponding residue(s) of the germline sequence from which the antibody was derived, or to the corresponding residue(s) of another human germline sequence, or to a conservative amino acid substitution of the corresponding germline residue(s) (such sequence changes are referred to herein collectively as "germline mutations"). A person of ordinary skill in the art, starting with the heavy and light chain variable region sequences disclosed herein, can easily produce numerous antibodies and antigen-binding fragments which comprise one or more individual germline mutations or combinations thereof. In certain embodiments, all of the framework and/or CDR residues within the V_{H} and/or V_{L} domains are mutated back to the residues found in the original germline sequence from which the antibody was derived. In other embodiments, only certain residues are mutated back to the original germline sequence, e.g., only the mutated residues found within the first 8 amino acids of FR1 or within the last 8 amino acids of FR4, or only the mutated residues found within CDR1, CDR2 or CDR3. In other embodiments, one or more of the framework and/or CDR residue(s) are mutated to the corresponding residue(s) of a different germline sequence (*i.e.,* a germline sequence that is different from the germline sequence from which the antibody was originally derived). Furthermore, the antibodies for use in the present invention may contain any combination of two or more germline mutations within the framework and/or CDR regions, *e.g.,* wherein certain individual residues are mutated to the corresponding residue of a particular germline sequence while certain other residues that differ from the original germline sequence are maintained or are mutated to the corresponding residue of a different germline sequence. Once obtained, antibodies and antigen-binding fragments that contain one or more germline mutations can be easily tested for one or more desired property such as, improved binding specificity, increased binding affinity, improved or enhanced antagonistic or agonistic biological properties (as the case may be), reduced immunogenicity, etc. The use of pharmaceutical compositions comprising antibodies and antigen-binding fragments obtained in this general manner are encompassed within the present invention.

The present invention also includes pharmaceutical compositions for use comprising anti-PCSK9 antibodies comprising variants of any of the HCVR, LCVR, and/or CDR amino acid sequences disclosed herein having one or more conservative substitutions. For example, the present invention includes the pharmaceutical compositions for use comprising anti-PCSK9 antibodies having HCVR, LCVR, and/or CDR amino acid sequences with, e.g., 10 or fewer, 8 or fewer, 6 or fewer, 4 or fewer, etc. conservative amino acid substitutions relative to any of the HCVR, LCVR, and/or CDR amino acid sequences disclosed herein.

The term "surface plasmon resonance", as used herein, refers to an optical phenomenon that allows for the analysis of real-time interactions by detection of alterations in protein concentrations within a biosensor matrix, for example using the BIAcore™ system (Biacore Life Sciences division of GE Healthcare, Piscataway, NJ).

The term "K_{D}", as used herein, is intended to refer to the equilibrium dissociation constant of a particular antibody-antigen interaction.

The term "epitope" refers to an antigenic determinant that interacts with a specific antigen binding site in the variable region of an antibody molecule known as a paratope. A single antigen may have more than one epitope. Thus, different antibodies may bind to different areas on an antigen and may have different biological effects. Epitopes may be either conformational or linear. A conformational epitope is produced by spatially juxtaposed amino acids from different segments of the linear polypeptide chain. A linear epitope is one produced by adjacent amino acid residues in a polypeptide chain. In certain circumstance, an epitope may include moieties of saccharides, phosphoryl groups, or sulfonyl groups on the antigen.

According to certain embodiments, the anti-PCSK9 antibody comprised in the pharmaceutical compositions for use in the present invention is an antibody with pH-dependent binding characteristics. As used herein, the expression "pH-dependent binding" means that the antibody or antigen-binding fragment thereof exhibits "reduced binding to PCSK9 at acidic pH as compared to neutral pH" (for purposes of the present disclosure, both expressions may be used interchangeably). For the example, antibodies "with pH-dependent binding characteristics" includes antibodies and antigen-binding fragments thereof that bind PCSK9 with higher affinity at neutral pH than at acidic pH. In certain embodiments, the antibodies and antigen-binding fragments of the present invention bind PCSK9 with at least 3, 5, 10, 15, 20, 25, 30, 35, 40, 45, 50, 55, 60, 65, 70, 75, 80, 85, 90, 95, 100, or more times higher affinity at neutral pH than at acidic pH.

According to this aspect of the invention, the pharmaceutical compositions for use comprising anti-PCSK9 antibodies with pH-dependent binding characteristics may possess one or more amino acid variations relative to the parental anti-PCSK9 antibody. For example, an anti-PCSK9 antibody with pH-dependent binding characteristics may contain one or more histidine substitutions or insertions, *e.g.,* in one or more CDRs of a parental anti-PCSK9 antibody. Thus, according to certain embodiments of the present invention, pharmaceutical compositions for use are provided comprising administering an anti-PCSK9 antibody which comprises CDR amino acid sequences (*e.g.,* heavy and light chain CDRs) which are identical to the CDR amino acid sequences of a parental anti-PCSK9 antibody, except for the substitution of one or more amino acids of one or more CDRs of the parental antibody with a histidine residue. The anti-PCSK9 antibodies with pH-dependent binding may possess, *e.g.,* 1, 2, 3, 4, 5, 6, 7, 8, 9, or more histidine substitutions, either within a single CDR of a parental antibody or distributed throughout multiple (*e.g.,* 2, 3, 4, 5, or 6) CDRs of a parental anti-PCSK9 antibody. For example, the present invention includes pharmaceutical compositions for use comprising anti-PCSK9 antibodies with pH-dependent binding comprising one or more histidine substitutions in HCDR1, one or more histidine substitutions in HCDR2, one or more histidine substitutions in HCDR3, one or more histidine substitutions in LCDR1, one or more histidine substitutions in LCDR2, and/or one or more histidine substitutions in LCDR3, of a parental anti-PCSK9 antibody.

As used herein, the expression "acidic pH" means a pH of 6.0 or less (*e.g.,* less than about 6.0, less than about 5.5, less than about 5.0, etc.). The expression "acidic pH" includes pH values of about 6.0, 5.95, 5.90, 5.85, 5.8, 5.75, 5.7, 5.65, 5.6, 5.55, 5.5, 5.45, 5.4, 5.35, 5.3, 5.25, 5.2, 5.15, 5.1, 5.05, 5.0, or less. As used herein, the expression "neutral pH" means a pH of about 7.0 to about 7.4. The expression "neutral pH" includes pH values of about 7.0, 7.05, 7.1, 7.15, 7.2, 7.25, 7.3, 7.35, and 7.4.

Non-limiting examples of anti-PCSK9 antibodies that can be used in the context of the present invention include alirocumab or antigen-binding portions thereof.

### Preparation of Human Antibodies

Methods for generating human antibodies in transgenic mice are known in the art. Any such known methods can be used in the context of the present invention to make human antibodies that specifically bind to human PCSK9.

Using VELOCIMMUNE™ technology (see, for example, US Patent No: 6,596,541B2, Regeneron Pharmaceuticals) or any other known method for generating monoclonal antibodies, high affinity chimeric antibodies to PCSK9 are initially isolated having a human variable region and a mouse constant region. The VELOCIMMUNE® technology involves generation of a transgenic mouse having a genome comprising human heavy and light chain variable regions operably linked to endogenous mouse constant region loci such that the mouse produces an antibody comprising a human variable region and a mouse constant region in response to antigenic stimulation. The DNA encoding the variable regions of the heavy and light chains of the antibody are isolated and operably linked to DNA encoding the human heavy and light chain constant regions. The DNA is then expressed in a cell capable of expressing the fully human antibody.

Generally, a VELOCIMMUNE® mouse is challenged with the antigen of interest, and lymphatic cells (such as B-cells) are recovered from the mice that express antibodies. The lymphatic cells may be fused with a myeloma cell line to prepare immortal hybridoma cell lines, and such hybridoma cell lines are screened and selected to identify hybridoma cell lines that produce antibodies specific to the antigen of interest. DNA encoding the variable regions of the heavy chain and light chain may be isolated and linked to desirable isotypic constant regions of the heavy chain and light chain. Such an antibody protein may be produced in a cell, such as a CHO cell. Alternatively, DNA encoding the antigen-specific chimeric antibodies or the variable domains of the light and heavy chains may be isolated directly from antigen-specific lymphocytes.

Initially, high affinity chimeric antibodies are isolated having a human variable region and a mouse constant region. The antibodies are characterized and selected for desirable characteristics, including affinity, selectivity, epitope, etc, using standard procedures known to those skilled in the art. The mouse constant regions are replaced with a desired human constant region to generate the fully human antibody comprised in the pharmaceutical compositions for use in the invention, for example wild-type or modified IgG1 or IgG4. While the constant region selected may vary according to specific use, high affinity antigen-binding and target specificity characteristics reside in the variable region.

In general, the antibodies that can be used in the pharmaceutical compositions of the present invention possess high affinities, as described above, when measured by binding to antigen either immobilized on solid phase or in solution phase. The mouse constant regions are replaced with desired human constant regions to generate the fully human antibodies comprised in the pharmaceutical compositions of the invention. While the constant region selected may vary according to specific use, high affinity antigen-binding and target specificity characteristics reside in the variable region.

Specific examples of human antibodies or antigen-binding fragments of antibodies that specifically bind PCSK9 which can be used in the context of the pharmaceutical compositions for use in the present invention include any antibody or antigen-binding fragment which comprises the three heavy chain CDRs (HCDR1, HCDR2 and HCDR3) contained within a heavy chain variable region (HCVR) having an amino acid sequence selected from the group consisting of SEQ ID NOs:1 and 11, or a substantially similar sequence thereof having at least 90%, at least 95%, at least 98% or at least 99% sequence identity. Alternatively, specific examples of human antibodies or antigen-binding fragments of antibodies that specifically bind PCSK9 which can be used in the context of the pharmaceutical compositions for use in the present invention include any antibody or antigen-binding fragment which comprises the three heavy chain CDRs (HCDR1, HCDR2 and HCDR3) contained within a heavy chain variable region (HCVR) having an amino acid sequence selected from the group consisting of SEQ ID NOs 37, 45, 53, 61, 69, 77, 85, 93, 101, 109, 117, 125, 133, 141, 149, 157, 165, 173, 181, and 189, or a substantially similar sequence thereof having at least 90%, at least 95%, at least 98% or at least 99% sequence identity. The antibody or antigen-binding fragment may comprise the three light chain CDRs (LCVR1, LCVR2, LCVR3) contained within a light chain variable region (LCVR) having an amino acid sequence selected from the group consisting of SEQ ID NOs 6 and 15, or a substantially similar sequence thereof having at least 90%, at least 95%, at least 98% or at least 99% sequence identity. Alternatively, the antibody or antigen-binding fragment may comprise the three light chain CDRs (LCVR1, LCVR2, LCVR3) contained within a light chain variable region (LCVR) having an amino acid sequence selected from the group consisting of SEQ ID NOs 41, 49, 57, 65, 73, 81, 89, 97, 105, 113, 121, 129, 137, 145, 153, 161, 169, 177, 185, and 193, or a substantially similar sequence thereof having at least 90%, at least 95%, at least 98% or at least 99% sequence identity.

Sequence identity between two amino acids sequences is determined over the entire length of the reference amino acid sequence, i.e. the amino acid sequence identified with a SEQ ID NO, using the best sequence alignment and/or over the region of the best sequence alignment between the two amino acid sequences, wherein the best sequence alignment can be obtained with art known tools, e.g. Align, using standard settings, preferably EMBOSS::needle, Matrix: Blosum62, Gap Open 10.0, Gap Extend 0.5.

In certain embodiments of the present invention, the antibody or antigen-binding protein used comprises the six CDRs (HCDR1, HCDR2, HCDR3, LCDR1, LCDR2 and LCDR3) from the heavy and light chain variable region amino acid sequence pairs (HCVR/LCVR) selected from the group consisting of SEQ ID NOs:1/6 and 11/15. Alternatively, in certain embodiments of the present invention, the antibody or antigen-binding protein used comprises the six CDRs (HCDR1, HCDR2, HCDR3, LCDR1, LCDR2 and LCDR3) from the heavy and light chain variable region amino acid sequence pairs (HCVR/LCVR) selected from the group consisting of SEQ ID NOs:37/41, 45/49, 53/57, 61/65, 69/73, 77/81, 85/89, 93/97, 101/105, 109/113, 117/121, 125/129, 133/137, 141/145, 149/153, 157/161, 165/169, 173/177, 181/185, and 189/193.

In certain embodiments of the present invention, the anti-PCSK9 antibody, or antigen-binding protein, that can be used in the pharmaceutical compositions for use in the present invention has HCDR1/HCDR2/HCDR3/LCDR1/LCDR2/LCDR3 amino acid sequences selected from SEQ ID NOs: 2/3/4/7/8/10 (mAb316P [also referred to as "REGN727," or "alirocumab"]) and 12/13/14/16/17/18 (mAb300N) (See US Patent Application Publication No. 2010/0166768) and 12/13/14/16/17/18, wherein SEQ ID NO:16 comprises a substitution of histidine for leucine at amino acid residue 30 (L30H).

In certain embodiments of the present invention, the antibody or antigen-binding protein used comprises HCVR/LCVR amino acid sequence pairs selected from the group consisting of SEQ ID NOs:1/6 and 11/15. In certain exemplary embodiments, the antibody or antigen-binding protein comprises an HCVR amino acid sequence of SEQ ID NO:1 and an LCVR amino acid sequence of SEQ ID NO:6. In certain exemplary embodiments, the antibody or antigen-binding protein comprises an HCVR amino acid sequence of SEQ ID NO:11 and an LCVR amino acid sequence of SEQ ID NO:15. In certain exemplary embodiments, the antibody or antigen-binding protein comprises an HCVR amino acid sequence of SEQ ID NO:11 and an LCVR amino acid sequence of SEQ ID NO:15 comprising a substitution of histidine for leucine at amino acid residue 30 (L30H).

### Pharmaceutical Compositions and Methods of Administration

The present invention includes pharmaceutical compositions for use which comprise administering an anti-PCSK9 antibody or antigen-binding fragment thereof to a patient, wherein the anti-PCSK9 antibody or antigen-binding fragment thereof is contained within the pharmaceutical composition. The pharmaceutical compositions for use in the invention are formulated with suitable carriers, excipients, and other agents that provide suitable transfer, delivery, tolerance, and the like. A multitude of appropriate formulations can be found in the formulary known to all pharmaceutical chemists: Remington's Pharmaceutical Sciences, Mack Publishing Company, Easton, PA. These formulations include, for example, powders, pastes, ointments, jellies, waxes, oils, lipids, lipid (cationic or anionic) containing vesicles (such as LIPOFECTIN™), DNA conjugates, anhydrous absorption pastes, oil-in-water and water-in-oil emulsions, emulsions carbowax (polyethylene glycols of various molecular weights), semi-solid gels, and semi-solid mixtures containing carbowax. See also Powell et al. "Compendium of excipients for parenteral formulations" PDA (1998) J Pharm Sci Technol 52:238-311.

Various delivery systems are known and can be used to administer the pharmaceutical composition for use in the invention, *e.g.,* encapsulation in liposomes, microparticles, microcapsules, recombinant cells capable of expressing the mutant viruses, receptor mediated endocytosis (see, e.g., Wu et al., 1987, J. Biol. Chem. 262:4429-4432). Methods of administration include, but are not limited to, intradermal, intramuscular, intraperitoneal, intravenous, subcutaneous, intranasal, epidural, and oral routes. The composition may be administered by any convenient route, for example by infusion or bolus injection, by absorption through epithelial or mucocutaneous linings (e.g., oral mucosa, rectal and intestinal mucosa, etc.) and may be administered together with other biologically active agents.

A pharmaceutical composition for use in the present invention can be delivered subcutaneously or intravenously with a standard needle and syringe. In addition, with respect to subcutaneous delivery, a pen delivery device readily has applications in delivering a pharmaceutical composition for use in the present invention. Such a pen delivery device can be reusable or disposable. A reusable pen delivery device generally utilizes a replaceable cartridge that contains a pharmaceutical composition. Once all of the pharmaceutical composition within the cartridge has been administered and the cartridge is empty, the empty cartridge can readily be discarded and replaced with a new cartridge that contains the pharmaceutical composition. The pen delivery device can then be reused. In a disposable pen delivery device, there is no replaceable cartridge. Rather, the disposable pen delivery device comes prefilled with the pharmaceutical composition held in a reservoir within the device. Once the reservoir is emptied of the pharmaceutical composition, the entire device is discarded.

Numerous reusable pen and autoinjector delivery devices have applications in the subcutaneous delivery of a pharmaceutical composition for use in the present invention. Examples include, but are not limited to AUTOPEN™ (Owen Mumford, Inc., Woodstock, UK), DISETRONIC™ pen (Disetronic Medical Systems, Bergdorf, Switzerland), HUMALOG MIX 75/25™ pen, HUMALOG™ pen, HUMALIN 70/30™ pen (Eli Lilly and Co., Indianapolis, IN), NOVOPEN™ I, II and III (Novo Nordisk, Copenhagen, Denmark), NOVOPEN JUNIOR™ (Novo Nordisk, Copenhagen, Denmark), BD™ pen (Becton Dickinson, Franklin Lakes, NJ), OPTIPEN™, OPTIPEN PRO™, OPTIPEN STARLET™, and OPTICLIK™ (sanofi-aventis, Frankfurt, Germany), to name only a few. Examples of disposable pen delivery devices having applications in subcutaneous delivery of a pharmaceutical composition for use in the present invention include, but are not limited to the SOLOSTAR™ pen (sanofi-aventis), the FLEXPEN™ (Novo Nordisk), and the KWIKPEN™ (Eli Lilly), the SURECLICK™ Autoinjector (Amgen, Thousand Oaks, CA), the PENLET™ (Haselmeier, Stuttgart, Germany), the EPIPEN (Dey, L.P.), and the HUMIRA™ Pen (Abbott Labs, Abbott Park IL), to name only a few.

In certain situations, the pharmaceutical composition can be delivered in a controlled release system. In one embodiment, a pump may be used (see Langer, supra; Sefton, 1987, CRC Crit. Ref. Biomed. Eng. 14:201). In another embodiment, polymeric materials can be used; see, Medical Applications of Controlled Release, Langer and Wise (eds.), 1974, CRC Pres., Boca Raton, Florida. In yet another embodiment, a controlled release system can be placed in proximity of the composition's target, thus requiring only a fraction of the systemic dose (see, *e.g.,* Goodson, 1984, in Medical Applications of Controlled Release, supra, vol. 2, pp. 115-138). Other controlled release systems are discussed in the review by Langer, 1990, Science 249:1527-1533.

The injectable preparations may include dosage forms for intravenous, subcutaneous, intracutaneous and intramuscular injections, drip infusions, etc. These injectable preparations may be prepared by known methods. For example, the injectable preparations may be prepared, *e.g.,* by dissolving, suspending or emulsifying the antibody or its salt described above in a sterile aqueous medium or an oily medium conventionally used for injections. As the aqueous medium for injections, there are, for example, physiological saline, an isotonic solution containing glucose and other auxiliary agents, etc., which may be used in combination with an appropriate solubilizing agent such as an alcohol (e.g., ethanol), a polyalcohol (e.g., propylene glycol, polyethylene glycol), a nonionic surfactant [e.g., polysorbate 80, HCO-50 (polyoxyethylene (50 mol) adduct of hydrogenated castor oil)], etc. As the oily medium, there are employed, e.g., sesame oil, soybean oil, etc., which may be used in combination with a solubilizing agent such as benzyl benzoate, benzyl alcohol, etc. The injection thus prepared is preferably filled in an appropriate ampoule.

Advantageously, the pharmaceutical compositions for oral or parenteral use described above are prepared into dosage forms in a unit dose suited to fit a dose of the active ingredients. Such dosage forms in a unit dose include, for example, tablets, pills, capsules, injections (ampoules), suppositories, etc.

Exemplary pharmaceutical formulations comprising an anti-PCSK9 antibody that can be used in the context of the pharmaceutical compositions for use in the present invention are set forth, *e.g.,* in US Patent Application Publication 2013/0189277.

### Dosage

The amount of an anti-PCSK9 antibody or antigen-binding fragment thereof administered to a patient according to the pharmaceutical compositions for use in the present invention is, generally, a therapeutically effective amount. As used herein, the phrase "therapeutically effective amount" means a dose of an anti-PCSK9 antibody or antigen-binding fragment thereof that results in a detectable reduction (at least about 5%, 10%, 15%, 20%, 25%, 30%, 35%, 40%, 45%, 50%, 55%, 60%, 65%, 70%, 75%, or more from baseline) in one or more parameters selected from the group consisting of LDL-C, ApoB100, non-HDL-C, total cholesterol, VLDL-C, triglycerides, Lp(a) and remnant cholesterol.

For an anti-PCSK9 antibody, a therapeutically effective amount can be from about 0.05 mg to about 600 mg, *e.g.,* about 0.05 mg, about 0.1 mg, about 1.0 mg, about 1.5 mg, about 2.0 mg, about 10 mg, about 20 mg, about 30 mg, about 40 mg, about 50 mg, about 60 mg, about 70 mg, about 75 mg, about 80 mg, about 90 mg, about 100 mg, about 110 mg, about 120 mg, about 130 mg, about 140 mg, about 150 mg, about 160 mg, about 170 mg, about 180 mg, about 190 mg, about 200 mg, about 210 mg, about 220 mg, about 230 mg, about 240 mg, about 250 mg, about 260 mg, about 270 mg, about 280 mg, about 290 mg, about 300 mg, about 310 mg, about 320 mg, about 330 mg, about 340 mg, about 350 mg, about 360 mg, about 370 mg, about 380 mg, about 390 mg, about 400 mg, about 410 mg, about 420 mg, about 430 mg, about 440 mg, about 450 mg, about 460 mg, about 470 mg, about 480 mg, about 490 mg, about 500 mg, about 510 mg, about 520 mg, about 530 mg, about 540 mg, about 550 mg, about 560 mg, about 570 mg, about 580 mg, about 590 mg, or about 600 mg, of the anti-PCSK9 antibody. In certain embodiments, the therapeutically effective amount is 75 mg of the anti-PCSK9 antibody. In certain embodiments, the therapeutically effective amount is 150 mg of the anti-PCSK9 antibody.

The amount of anti-PCSK9 antibody contained within the individual doses may be expressed in terms of milligrams of antibody per kilogram of patient body weight (*i.e*., mg/kg). For example, the anti-PCSK9 antibody may be administered to a patient at a dose of about 0.0001 to about 10 mg/kg of patient body weight.

### Combination Therapies

According to certain embodiments of the present invention, one or more non-statin lipid modifying therapies may be administered to the patient in combination with the pharmaceutical compositions for use comprising an anti-PCSK9 antibody or antigen-binding fragment thereof. Examples of such non-statin lipid modifying therapies include *e.g.,* (1) an agent which inhibits cholesterol absorption inhibitors (*e.g.,* ezetimibe); (2) an agent which increases lipoprotein catabolism (such as nicotinic acid, including niacin and slow-release niacins); (3) fibric acid, (4) a bile acid sequestrant and/or (5) an activator of the LXR transcription factor that play a role in cholesterol elimination (such as 22-hydroxycholesterol).

### Administration Regimens

According to certain embodiments of the present invention, multiple doses of an anti-PCSK9 antibody or antigen-binding fragment thereof (*i.e.,* a pharmaceutical composition for use comprising an anti-PCSK9 antibody or antigen-binding fragment thereof) may be administered to a patient over a defined time course (*e.g.,* in place of of a daily therapeutic statin regimen). The pharmaceutical compositions for use according to this aspect of the invention comprise sequentially administering to a patient multiple doses of an anti-PCSK9 antibody or antigen-binding fragment thereof. As used herein, "sequentially administering" means that each dose of Anti-PCSK9 antibody or antigen-binding fragment thereof is administered to the patient at a different point in time, *e.g.,* on different days separated by a predetermined interval (*e.g.,* hours, days, weeks or months). The present invention includes pharmaceutical compositions for use which comprise sequentially administering to the patient a single initial dose of an anti-PCSK9 antibody or antigen-binding fragment thereof, followed by one or more secondary doses of the anti-PCSK9 antibody or antigen-binding fragment thereof, and optionally followed by one or more tertiary doses of the anti-PCSK9 antibody or antigen-binding fragment thereof.

The terms "initial dose," "secondary doses," and "tertiary doses," refer to the temporal sequence of administration of the individual doses of a pharmaceutical composition comprising an anti-PCSK9 antibody or antigen-binding fragment thereof. Thus, the "initial dose" is the dose which is administered at the beginning of the treatment regimen (also referred to as the "baseline dose"); the "secondary doses" are the doses which are administered after the initial dose; and the "tertiary doses" are the doses which are administered after the secondary doses. The initial, secondary, and tertiary doses may all contain the same amount of the anti-PCSK9 antibody or antigen-binding fragment thereof, but generally may differ from one another in terms of frequency of administration. In certain embodiments, however, the amount of anti-PCSK9 antibody or antigen-binding fragment thereof contained in the initial, secondary and/or tertiary doses varies from one another (*e.g.,* adjusted up or down as appropriate) during the course of treatment. In certain embodiments, two or more (*e.g.,* 2, 3, 4, or 5) doses are administered at the beginning of the treatment regimen as "loading doses" followed by subsequent doses that are administered on a less frequent basis (*e.g.,* "maintenance doses").

According to exemplary embodiments of the present invention, each secondary and/or tertiary dose is administered 1 to 26 (*e.g.,* 1, 1½, 2, 2½, 3, 3½, 4, 4½, 5, 5½, 6, 6½, 7, 7½, 8, 8½, 9, 9½, 10, 10½, 11, 11½, 12, 12½, 13, 13½, 14, 14½, 15, 15½, 16, 16½, 17, 17½, 18, 18½, 19, 19½, 20, 20½, 21, 21½, 22, 22½, 23, 23½, 24, 24½, 25, 25½, 26, 26½, or more) weeks after the immediately preceding dose. The phrase "the immediately preceding dose," as used herein, means, in a sequence of multiple administrations, the dose of antigen-binding molecule which is administered to a patient prior to the administration of the very next dose in the sequence with no intervening doses.

The pharmaceutical composition for use according to this aspect of the invention may comprise administering to a patient any number of secondary and/or tertiary doses of an anti-PCSK9 antibody or antigen-binding fragment thereof. For example, in certain embodiments, only a single secondary dose is administered to the patient. In other embodiments, two or more (*e.g.,* 2, 3, 4, 5, 6, 7, 8, or more) secondary doses are administered to the patient. Likewise, in certain embodiments, only a single tertiary dose is administered to the patient. In other embodiments, two or more (*e.g.,* 2, 3, 4, 5, 6, 7, 8, or more) tertiary doses are administered to the patient.

In embodiments involving multiple secondary doses, each secondary dose may be administered at the same frequency as the other secondary doses. For example, each secondary dose may be administered to the patient 1 to 2, 4, 6, 8 or more weeks after the immediately preceding dose. Similarly, in embodiments involving multiple tertiary doses, each tertiary dose may be administered at the same frequency as the other tertiary doses. For example, each tertiary dose may be administered to the patient 1 to 2, 4, 6, 8 or more weeks after the immediately preceding dose. Alternatively, the frequency at which the secondary and/or tertiary doses are administered to a patient can vary over the course of the treatment regimen. The frequency of administration may also be adjusted during the course of treatment by a physician depending on the needs of the individual patient following clinical examination.

According to certain embodiments of the present invention, multiple doses of a pharmaceutical composition comprising about 75 mg of anti-PCSK9 antibody are administered to a patient at a frequency of once every two weeks.

According to certain embodiments of the present invention, multiple doses of a pharmaceutical composition comprising about 150 mg of anti-PCSK9 antibody are administered to a patient at a frequency of once every two weeks.

According to certain embodiments of the present invention, multiple doses of a pharmaceutical composition comprising about 75 mg of anti-PCSK9 antibody are administered to a patient at a frequency of once every four weeks.

According to certain embodiments of the present invention, multiple doses of a pharmaceutical composition comprising about 150 mg of anti-PCSK9 antibody are administered to a patient at a frequency of once every four weeks.

The present invention includes administration regimens comprising an up-titration option (also referred to herein as "dose modification"). As used herein, an "up-titration option" means that, after receiving a particular number of doses of an anti-PCSK9 antibody, if a patient has not achieved a specified reduction in one or more defined therapeutic parameters, the dose of the anti-PCSK9 antibody or antigen-binding fragment thereof is thereafter increased. For example, in the case of a therapeutic regimen comprising administration of 75 mg doses of an anti-PCSK9 antibody to a patient at a frequency of once every two weeks, if after 8 weeks (i.e., 5 doses administered at Week 0, Week 2 and Week 4, Week 6 and Week 8), the patient has not achieved a serum LDL-C concentration of less than 70 mg/dL, then the dose of anti-PCSK9 antibody is increased to *e.g.,* 150 mg administered once every two weeks thereafter *(e.g.,* starting at Week 10 or Week 12, or later).

In certain embodiments, the anti-PCSK9 antibody is administered to a patient at a dose of about 75 mg every two weeks, for example for at least three doses.

In certain embodiments, the anti-PCSK9 antibody is administered to a patient at a dose of about 150 mg every two weeks, for example for at least three doses.

In some embodiments, the antibody is administered to a patient at a dose of about 75 mg every two weeks for 12 weeks, and the dose remains at 75 mg every two weeks if, at week 8, the patient's LDL-C value was less than 100 mg/dl and a 30% reduction of LDL-C.

In other embodiments, the antibody is administered to a patient at a dose of about 75 mg every two weeks for 12 weeks, and the dose is titrated up to about 150 mg every two weeks if, at week 8, the patient's LDL-C value was greater than or equal to 100 mg/dl.

In some embodiments, the antibody is administered to a patient at a dose of about 75 mg every two weeks for 12 weeks, and the dose remains at 75 mg every two weeks if, at week 8, the patient's LDL-C value was less than 70 mg/dl and a 30% reduction of LDL-C.

In another embodiment, the antibody is administered to a patient at a dose of about 300 mg every four weeks.

In a further embodiment, the antibody is administered to a patient at a dose of about 300 mg every four weeks for a total of three doses, and the dose is changed to 150 mg every two weeks for another 36 weeks if, at week 8, the patient did not achieve a pre-determined treatment goal or the patient did not have at least a 30% reduction of LDL-C from baseline.

In certain embodiments, the anti-PCSK9 antibody is administered to a patient at a dose of about 150 mg every four weeks for at least three doses.

In some embodiments, the antibody is administered to a patient at a dose of about 150 mg every four weeks for 12 weeks, and the dose remains at 150 mg every four weeks if, at week 8, the patient's LDL-C value was less than 100 mg/dl and a 30% reduction of LDL-C.

In other embodiments, the antibody is administered to a patient at a dose of about 150 mg every four weeks for 12 weeks, and the dose is titrated up to about 300 mg every two weeks if, at week 8, the patient's LDL-C value was greater than or equal to 100 mg/dl.

In some embodiments, the antibody is administered to a patient at a dose of about 150 mg every four weeks for 12 weeks, and the dose remains at 150 mg every four weeks for another 12 weeks if, at week 8, the patient's LDL-C value was less than 70 mg/dl and a 30% reduction of LDL-C.

In another embodiment, the antibody is administered to a patient at a dose of about 300 mg every four weeks.

In a further embodiment, the antibody is administered to a patient at a dose of about 300 mg every four weeks for a total of three doses, and the dose is changed to 150 mg every two weeks for another 36 weeks if, at week 8, the patient did not achieve a pre-determined treatment goal or the patient did not have at least a 30% reduction of LDL-C from baseline.

### EXAMPLES

The following examples are put forth so as to provide those of ordinary skill in the art with a complete disclosure and description of how to make the pharmaceutical compositions for use in the invention, and are not intended to limit the scope of what the inventors regard as their invention. Efforts have been made to ensure accuracy with respect to numbers used (e.g., amounts, temperature, etc.) but some experimental errors and deviations should be accounted for. Unless indicated otherwise, parts are parts by weight, molecular weight is average molecular weight, temperature is in degrees Centigrade, and pressure is at or near atmospheric.

### Example 1. Generation of Human Antibodies to Human PCSK9

Human anti-PCSK9 antibodies were generated as described in US Patent No. 8,062,640. The exemplary anti-PCSK9 antibody used in the following Example is the human anti-PCSK9 antibody designated "mAb316P," also known as "REGN727," or "alirocumab." mAb316P has the following amino acid sequence characteristics: a heavy chain comprising SEQ ID NO:5 and a light chain comprising SEQ ID NO:9; a heavy chain variable region (HCVR) comprising SEQ ID NO:1 and a light chain variable domain (LCVR) comprising SEQ ID NO:6; a heavy chain complementarity determining region 1 (HCDR1) comprising SEQ ID NO:2, a HCDR2 comprising SEQ ID NO:3, a HCDR3 comprising SEQ ID NO:4, a light chain complementarity determining region 1 (LCDR1) comprising SEQ ID NO:7, a LCDR2 comprising SEQ ID NO:8 and a LCDR3 comprising SEQ ID NO:10.

### Example 2: Monotherapy with an anti-PCSK9 Antibody ("mAb316P") Versus Ezetimibe in Patients with Hypercholesterolemia: Results of a 24 Week, Double-Blind, Randomized Phase 3 Trial

### BACKGROUND

Hypercholesterolemia, particularly an increase in low-density lipoprotein cholesterol (LDL-C) levels, constitutes a major risk for the development of atherosclerosis and CHD, the leading cause of death and disability in the Western world. LDL-C is identified as the primary target of cholesterol lowering therapy and is accepted as a valid surrogate endpoint. Numerous studies have demonstrated that reducing LDL-C levels mainly via 3-hydroxy-3-methyl-glutaryl-CoA reductase (HMG CoA) inhibition with statin, reduces the risk of CHD, with a strong direct relationship between LDL-C levels and CHD events; for each 1 mmol/L (-40 mg/dL) reduction in LDL-C, cardiovascular disease (CVD) mortality and morbidity is lowered by 22%.

Three Phase 1 studies have been conducted with mAb316P and evaluated the safety, tolerability and PK/PD profile. Two studies were single dose administration (one study with IV administration of doses from 0.3 mg to 12 mg/kg and another study with SC administration of doses from 50 mg to 250 mg) conducted in healthy subjects with LDL-C >100 mg/dL for whom statin therapy was not indicated. The third study was conducted in hypercholesterolemic patients

(familial or non-familial) with single to multiple SC administration of 50 mg, 100 mg, 150 mg and 200 mg either as add-on to stable doses of atorvastatin from 10 mg to 40 mg/day or as monotherapy.

Results of these Phase 1 studies showed that mAb316P administered to healthy subjects and patients either by IV or SC administration was generally well tolerated at all doses; treatment emergent adverse events (TEAEs) did not display a dose relationship. No pattern of adverse events (AEs) related to the drug was identified. In all these Phase 1 studies, administration of MAb316P induced rapid, substantial, and sustained reductions from baseline in LDL-C, up to 60%. The magnitude and duration of these reductions were positively related to the dose administered. It should also be noted that in the third study, results were similar in the familial and non-familial hypercholesterolemic patients. Overall, a total of 109 subjects were exposed to at least 1 dose of mAb316P in these 3 Phase 1 studies.

Three Phase 2 studies have also been conducted. The results of these studies have been previously reported.

### INTRODUCTION

In the present example, a phase 3 clinical trial was conducted to evaluate the efficacy and safety of mAb316P when administered as a monotherapy.

The aim of this study was to provide information on the magnitude of effect and safety profile when the investigational product mAb316P is used as monotherapy. Obtaining data on the singular efficacy and safety of mAb316P is important to put in perspective with data obtained when it is used as add on to statin.

Another aim of this study was to provide monotherapy data supporting the assessment of mAb316P in statin-intolerant patients. Current LDL-C-lowering medications that can be used as monotherapy when statins are considered inappropriate or not tolerated include ezetimibe, niacin, and bile acid sequestrants. Those options that might be used in monotherapy are associated with about 20% LDL-C reduction.

The control arm that was selected for this study was ezetimibe 10 mg PO daily. This allowed for a study comparing mAb316P against a treatment option (i.e., ezetimibe) which is available in routine clinical practice.

This specific study was undertaken to demonstrate in patients with moderate CV risk and with LDL-C between 100 mg/dL (2.59 mmol/L) and 190 mg/dL (4.9 mmol/L) that mAb316P 75 mg and/or 150 mg Q2W as monotherapy causes a statistically significant and clinically meaningful reduction in LDL-C compared to ezetimibe.

### Study population

The study population for the monotherapy study was patients with LDL-C between 100 mg/dL (2.59 mmol/L) and 190 mg/dL (4.9 mmol/L).

This study included patients with moderate CV risk, as defined by a 10-year risk of CVD death ≥1% and <5% based on the SCORE chart, and without established CHD or CHD risk equivalents. Risk charts such as SCORE are intended to facilitate risk estimation in apparently healthy persons with no signs of clinical or preclinical disease. SCORE measures the 10-year risk of CV death based on total cholesterol, age, gender, smoking, and systolic BP. This level of risk was considered appropriate in the context of a monotherapy study with a nonstatin active comparator.

The sample size of 100 patients (50 patients per group) with a double-blind study treatment duration of 24 weeks was intended to detect a treatment difference of 20% in means percent change in LDL-C from baseline to Week 24, with a 0.05 two-sided significance level and assuming a common SD of 25% and 5% non-evaluable primary endpoint.

### Selection of the dose

All patients were initially treated with 75 mg Q2W, and only those patients whose LDL-C levels remained equal to or higher than 100 mg/dL after 8 weeks of treatment were up-titrated to 150 mg Q2W at week 12 onwards.

The selection of doses, dosing frequency and up-titration approach is based on the LDL-C reduction needed to provide the best benefit in terms of CV disease reduction, and potential safety considerations regarding low LDL-C values. Based on the results of the 2 dose finding studies, the Q2W dosing regimen is expected to maintain constant LDL-C lowering throughout the interdosing interval, with the maximum efficacy at 12 weeks provided by the 150 mg Q2W dosing. However, for many patients, the magnitude of effect observed with the 150 mg Q2W dose may not be needed to achieve the target LDL-C goal, and starting with a lower dose may be undertaken. Using a dose response model, 75 mg Q2W was selected to provide approximately 50% decrease in LDL-C from baseline: all patients were initially treated with 75 mg Q2W, and only those patients whose LDL-C levels remain equal to or higher than 100 mg/dL after 8 weeks of treatment were dose up-titrated to 150 mg Q2W (at week 12). With this treatment scheme, most patients with primary hypercholesterolemia were expected to achieve their target LDL-C level, with few patients reaching a level below 25 mg/dL.

Preliminary PK data from the Phase 2 studies showed that exposure to mAb316P declined during the 8-week follow-up period that followed the double-blind treatment period, with serum total concentrations of mAb316P still detectable, but at very low levels. Therefore to ensure sufficient low, noneffective serum mAb316P concentrations, patients were followed during a follow-up period of 8 weeks (ie, 10 weeks after last dosing). Pharmacokinetic results in this trial are important since there is no statin background to attenuate the effect of mAb316P.

### STUDY OBJECTIVES

The primary objective of the monotherapy study was to demonstrate the reduction of low density lipoprotein cholesterol (LDL-C) by mAb316P every 2 weeks (Q2W) as monotherapy in comparison with ezetimibe (EZE) 10 mg daily after 24 weeks of treatment in patients with hypercholesterolemia at moderate cardiovascular (CV) risk.

The secondary objectives of the monotherapy study were as follows. (1) To evaluate the effect of mAb316P 75 mg in comparison with EZE on LDL-C after 12 weeks of treatment. (2) To evaluate the effect of mAb316P on other lipid parameters (i.e., Apo B, non-HDL-C, total-C, Lp (a), HDL-C, TG levels, and Apo A-1 levels). (3) To evaluate the safety and tolerability of MAb316P. (4) To evaluate the development of anti- MAb316P antibodies. (5) To evaluate the pharmacokinetics (PK) of MAb316P.

### STUDY DESIGN

This was a randomized, double-blind, parallel-group, double dummy, ezetimibe-controlled, balanced (1:1, mAb316P: ezetimibe), multi-center, multi-national study to assess the efficacy and the safety of mAb316P in patients with hypercholesterolemia and a 10 year risk score (SCORE) 21% and < 5%. Randomization was stratified according to DM status. After randomization, patients received double-blind study treatment (either mAb316P or placebo) every 2 weeks and ezetimibe or placebo for ezetimibe PO daily over a period of 24 weeks. A dose up-titration depending on Week 8 LDL-C levels may have occurred at Week 12 for patients randomized to mAb316P. Patients were followed for 8 weeks after the last visit of the Double Blind Treatment Period (DBTP). The study design is shown in **Figure 1****.**

### Description of the protocol

The study consisted of 3 periods: screening, double-blind treatment, and follow up.

Screening period - up to 2 weeks in duration including an intermediate visit during which the patient (or another designated person such as spouse, relative, etc.) was trained to self-inject/inject with placebo for mAb316P. Eligibility assessments were performed to permit the randomization of the patients into the study. Investigators had the option for providing a second training kit of placebo for mAb316P for patients who required additional self-injection training prior to randomization visit. The patient or Investigator could elect to have the patient inject at home or at study site.

Double-blind treatment period (DBTP) - A randomized, double-blind double dummy study treatment period of 24 weeks. The first injection during the double-blind period was done at the site on the day of randomization (Week 0 [D1] -V3) and as soon as possible after the call to IVRS/IWRS for randomization into the study. The subsequent injections were done by the patient (self-injection) or another designated person (such as spouse, relative, etc.) at a patient-preferred location (home, etc.). Patients randomized to mAb316P received a dose of 75 mg of mAb316P from randomization (V3) up to Week 12 (V6) (ie, Weeks 0, 2, 4, 6, 8, and 10) + placebo for ezetemibe PO daily. At the Week 12 visit (V6) these patients, in a blinded manner, either: (1) continued mAb316P 75 mg every 2 weeks (from week 12 onwards until the last injection at week 22), if the week 8 LDL-C was <100 mg/dL (1.81 mmol/L), or (2) dose up-titrate to mAb316P 150 mg every 2 weeks (from week 12 onwards until the last injection at week 22), if the week 8 LDL-C was 2100 mg/dL (1.81 mmol/L). Patients randomized to ezetimibe received mAb316P placebo injected every 2 weeks + ezetimibe 10 mg PO daily from randomization (V3) up to week 24 (V8).

Follow-up period - A period of 8 weeks after the end of the double-blind treatment period.

### Duration of study participation

The study duration included a screening period of up to 2-weeks, a 24-week double blind treatment period for efficacy and safety assessment and an 8-week post-treatment follow-up period for all patients after the last visit of the DBTP. Thus the study duration per patient was about 34 weeks.

### PATIENT SELECTION

The target population for this study was patients with hypercholesterolemia at moderate cardiovascular (CV) risk defined with a 10-year risk score ≥1% and <5%, based on the Systematic Coronary Risk Estimation (SCORE), and included a signed written informed consent.

Patients who met all the above inclusion criteria were screened for the following exclusion criteria, which were sorted and numbered in the following 3 subsections:
A. Exclusion criteria related to study methodology
   1. LDL-C <100 mg/dL or >190 mg/dL (<2.59 mmol/L or >4.9 mmol/L, respectively) at Week-2 (screening, V1).
   2. History of established CHD or CHD risk equivalents as defined as: **(A).** Documented history of CHD (includes one or more of the following): (1) Acute myocardial infarction (MI); (2) Silent MI; (3) Unstable angina; (4) Coronary revascularization procedure (eg, percutaneous coronary intervention [PCI] or coronary artery bypass graft surgery [CABG]); (5) Clinically significant CHD diagnosed by invasive or non-invasive testing (such as coronary angiography, stress test using treadmill, stress echocardiography or nuclear imaging) **(B)** CHD risk equivalents include clinical manifestations of noncoronary forms of atherosclerotic disease: (1) Symptomatic peripheral arterial disease; (2) Abdominal aortic aneurysm; or (3) Transient ischemic attacks or ischemic stroke and "clinically significant carotid artery obstruction by invasive or non-invasive testing (such as angiography or ultrasound)".
   3. Patients with DM associated with a risk SCORE ≥5% or with any additional risk factor (as listed below): (1) documented history of ankle-brachial index ≤0.90; (2) documented history of microalbuminuria or macroalbuminuria(30) OR dipstick urinalysis at screening visit (Week-2) with >2+ protein; or (3) documented history of pre-proliferative or proliferative retinopathy or laser treatment for retinopathy.
   4. Use of a statin, nicotinic acid, a bile acid-binding sequestrant, an intestinal cholesterol absorption (ICA) blocker (ie, ezetimibe), or omega-3 fatty acids at doses >1000 mg daily within 4 weeks of the screening visit (Week -2, V1) or between screening and randomization visits.
   5. Use of a fibrate within 6 weeks of the screening visit (Week -2, V1) or between screening and randomization visits.
   6. Use of nutraceutical products or over-the-counter therapies that may affect lipids which have not been at a stable dose/amount for at least 4 weeks prior to the screening visit (Week -2) or between screening and randomization visits
   7. Use of red yeast rice products within 4 weeks of the screening visit (Week-2) or between screening and randomization visits.
   8. Planned to undergo scheduled PCI, CABG, carotid or peripheral revascularization during the study.
   9. Systolic blood pressure (BP) >160 mmHg or diastolic BP >100 mmHg at screening (Week-2, V1) or randomization (Week 0) visits.
   10. History of New York Heart Association (NYHA) Class III or IV heart failure within the past 12 months.
   11. Known history of hemorrhagic stroke
   12. Age <18 years or legal age of majority at the screening visit (Week-2), whichever is greater.
   13. Patients not previously instructed on a cholesterol-lowering diet prior to the screening visit (Week-2)
   14. Newly diagnosed (within 3 months prior to randomization visit [Week 0]) or poorly controlled (HbA1_{c} > 8.5% at the screening visit [Week -2]) diabetes
   15. Presence of any clinically significant uncontrolled endocrine disease known to influence serum lipids or lipoproteins. *Note:* Patients on thyroid replacement therapy can be included if the dosage has been stable for at least 12 weeks prior to screening and TSH level is within the normal range of the Central Laboratory at the screening visit.
   16. History of bariatric surgery within 12 months prior to the screening visit (Week-2)
   17. Unstable weight defined by a variation >5 kg within 2 months prior to the screening visit (Week-2)
   18. Known history of homozygous or heterozygous familial hypercholesterolemia
   19. Known history of loss of function of PCSK9 (ie, genetic mutation or sequence variation)
   20. Use of systemic corticosteroids, unless used as replacement therapy for pituitary/adrenal disease with a stable regimen for at least 6 weeks prior to randomization visit (Week 0) *Note:* Topical, intra-articular, nasal, inhaled and ophthalmic steroid therapies are not considered as 'systemic' and are allowed.
   21. Use of continuous estrogen or testosterone hormone replacement therapy unless the regimen has been stable in the past 6 weeks prior to the Screening visit (Week-2) and no plans to change the regimen during the study.
   22. History of cancer within the past 5 years, except for adequately treated basal cell skin cancer, squamous cell skin cancer, or in situ cervical cancer
   23. Known history of HIV positivity
   24. Patient who has taken any investigational drugs other than the MAb316P training placebo kits within 1 month or 5 half lives, whichever is longer
   25. Patient who has previously participated in any clinical trial of MAb316P or any other anti-PCSK9 therapy.
   26. Patient who withdraws consent during the screening period (patient who is not willing to continue or fails to return).
   27. Conditions/situations such as: (1)aAny clinically significant abnormality identified at the time of screening that in the judgment of the Investigator or any sub-Investigator would preclude safe completion of the study or constrain endpoints assessment such as major systemic diseases, patients with short life expectancy; or (2) patients considered by the Investigator or any sub-Investigator as inappropriate for this study for any reason, eg: (a) deemed unable to meet specific protocol requirements, such as scheduled visits; (b) deemed unable to administer or tolerate long term injections as per the patient or the Investigator; (c) investigator or any sub-Investigator, pharmacist, study coordinator, other study staff or relative thereof directly involved in the conduct of the protocol, etc.; or (d) presence of any other conditions (eg, geographic, social) either actual or anticipated, that the Investigator feels would restrict or limit the patient's participation for the duration of the study.
   28. Laboratory findings during the screening period (not including randomization Week 0 labs): (1) Positive test for Hepatitis B surface antigen or Hepatitis C antibody (confirmed by reflexive testing); (2) Positive serum beta-hCG or urine pregnancy test (including Week 0) in women of childbearing potential; (3) Triglycerides >400 mg/dL (>4.52 mmol/L) (1 repeat lab is allowed); (4) eGFR <60 mL/min/1.73 m2 according to 4-variable MDRD Study equation (calculated by central lab); (5) ALT or AST >3 x ULN (1 repeat lab is allowed); (6) CPK >3 x ULN (1 repeat lab is allowed); or (7) TSH <LLN or >ULN.
B. Exclusion criteria related to background therapy
   29. All contraindications to the active comparator (ezetimibe) or warning/precaution of use (when appropriate) as displayed in the respective National Product Labeling.
C. Exclusion criteria related to MAb316P
   30. Known hypersensitivity to monoclonal antibody therapeutics
   31. Pregnant or breast-feeding women
   32. Women of childbearing potential with no effective contraceptive method of birth control and/or who are unwilling or unable to be tested for pregnancy
Note: Women of childbearing potential must have a confirmed negative pregnancy test at screening and randomization visits. They must use an effective contraceptive method throughout the study, and agree to repeat urine pregnancy test at designated visits. The applied methods of contraception have to meet the criteria for a highly effective method of birth control according to the Note for guidance on non-clinical safety studies for the conduct of human clinical trials and marketing authorization for pharmaceuticals. Postmenopausal women must be amenorrheic for at least 12 months.

### STUDY TREATMENTS

### Investigation Medicinal Product (IMP) and Administration

Sterile mAb316P drug product was supplied at a concentration of 75 mg/mL and 150 mg/mL both as 1 mL volume in an auto-injector. Sterile Placebo for mAb316P was prepared in the same formulation as mAb316P without the addition of protein as 1 mL volume in an auto-injector.

Ezetimibe 10 mg tablets over-encapsulated. Placebo for ezetimibe capsules.

The mAb316P IMP could be administered by self-injection or by another designated person (such as a spouse, relative, etc.). The used auto-injector was discarded in a sharps container which was provided to patients.

Patients were asked to store the mAb316P IMP in a refrigerator. Prior to administration, the IMP was to be set outside in a safe location at room temperature for about 30 to 40 minutes. Thereafter, the IMP should be administered as soon as possible.

During the double-blind treatment period, mAb316P or placebo for mAb316P was administered subcutaneously every 2 weeks, starting at week 0 continuing up to the last injection (week 22) 2 weeks before the end of the double blind treatment period.

MAb316P IMP injection was ideally administered every 2 weeks subcutaneously at approximately the same time of the day; however it was acceptable to have a window period of ± 3 days.

Ezetimibe 10 mg or placebo for ezetimibe capsules were taken orally once daily at approximately the same time of the day, with or without food.

### STUDY ENDPOINTS

### Primary efficacy endpoint

The primary efficacy endpoint was the percent change in calculated LDL-C from baseline to week 24, which is defined as: 100x (calculated LDL-C value at Week 24 - calculated LDL-C value at baseline) / calculated LDL-C value at baseline.

The baseline calculated LDL-C value was the last LDL-C level obtained before the first double-blind IMP, defined as the earliest between the first double-blind injection date and the first capsule intake.

The calculated LDL-C at week 24 was the LDL-C level obtained within the week 24 time window and during the main efficacy period. The main efficacy period was defined as the time from the first double-blind IMP up to 21 days after the last double-blind IMP injection or up to the upper limit of the Week 24 analysis window whichever comes first.

All calculated LDL-C values (scheduled or unscheduled, fasting or not fasting) may be used to provide a value for the primary efficacy endpoint if appropriate according to above definition.

### Secondary efficacy endpoints

### Key secondary efficacy endpoints

(1) The percent change in calculated LDL-C from baseline to week 12: similar definition and rules as above except that the calculated LDL-C at week 12 was the LDL-C level obtained within the week 12 analysis window and during the 12-week efficacy period. The 12-week efficacy period was defined as the time from the first double-blind IMP up to the Visit 6 re-supply IVRS contact or up to 21 days after the last double-blind IMP injection, whichever came first. Blood sampling collected the day of the Visit 6 re-supply IVRS contact was considered as before titration.
(2) The percent change in Apo B from baseline to Week 24. Same definition and rules as for the primary endpoint.
(3) The percent change in non-HDL-C from baseline to Week 24. Same definition and rules as for the primary endpoint.
(4) The percent change in total-C from baseline to Week 24. Same definition and rules as for the primary endpoint.
(5) The percent change in Apo B from baseline to Week 12. Same definition and rules as for the percent change in calculated LDL-C from baseline to Week 12.
(6) The percent change in non-HDL-C from baseline to Week 12. Same definition and rules as for the percent change in calculated LDL-C from baseline to Week 12.
(7) The percent change in total-C from baseline to Week 12. Same definition and rules as for the percent change in calculated LDL-C from baseline to Week 12.
(8) The proportion of patients reaching LDL-C goal <100 mg/dl (2.59 mmol/L) at Week 24, using definition and rules used for the primary endpoint.
(9) The proportion of patients reaching LDL-C goal <70 mg/dl (1.81 mmol/L) at Week 24, using definition and rules used for the primary endpoint.
(10) The percent change in Lp(a) from baseline to Week 24. Same definition and rules as for the primary endpoint.
(11) The percent change in HDL-C from baseline to Week 24. Same definition and rules as for the primary endpoint.
(12) The percent change in HDL-C from baseline to Week 12. Same definition and rules as for the percent change in calculated LDL-C from baseline to Week 12.
(13) The percent change in Lp(a) from baseline to Week 12. Same definition and rules as for the percent change in calculated LDL-C from baseline to Week 12.
(14) The percent change in fasting TG from baseline to Week 24. Same definition and rules as for the primary endpoint.
(15) The percent change in fasting TG from baseline to Week 12. Same definition and rules as for the percent change in calculated LDL-C from baseline to Week 12.
(16) The percent change in Apo A-1 from baseline to Week 24. Same definition and rules as for the primary endpoint.
(17) The percent change in Apo A-1 from baseline to Week 12. Same definition and rules as for the percent change in calculated LDL-C from baseline to Week 12.

### Other secondary efficacy endpoints

(18) The proportion of patients reaching LDL-C <100 mg/dL (2.59 mmol/L) at Week 12.
(19) The proportion of patients reaching LDL-C <70 mg/dL (1.81 mmol/L) at Week 12.
(20) The absolute change in LDL-C (mg/dL and mmol/L) from baseline to Weeks 12 and 24.
(21) The change in ratio Apo B/Apo A-1 from baseline to Weeks 12 to Week 24.
(22) The proportion of patients with Apo B <80 mg/dL (0.8 g/L) at Week 12 and Week 24.
(23) The proportion of patients with non-HDL-C <100 mg/dL (2.59mmol/L) at Weeks 12 and 24.
(24) The proportion of patients with LDL-C <70 mg/dL (1.81mmol/L) and / or ?50% reduction in LDL-C (if LDL-C ?70 mg/dL [1.81mmol/L]) at Weeks 12 and 24.

### Efficacy assessment method

### Lipid parameters

Total-C, HDL-C, TG, Apo B, Apo A-1, and Lp (a) were directly measured. LDL-C was calculated using the Friedewald formula at all visits (except Week -1 and Follow Up visit). If TG values exceeded 400 mg/dL (4.52 mmol/L) then the central lab reflexively measured (via the beta quantification method) the LDL-C rather than calculating it. Non-HDL-C was calculated by subtracting HDL-C from the total-C. Ratio Apo B/Apo A-1 was calculated.

### Safety endpoints - Observation period

The observation of safety data was as follows:

PRETREATMENT period: The PRETREATMENT observation period was defined from the signed informed consent up to the first dose of double-blind IMP.

TEAE period: The TEAE observation period was defined as the time from the first dose of double-blind IMP to the last dose of double-blind IMP injection + 70 days (10 weeks) as residual effect of MAb316P is expected until 10 weeks after the stop of double-blind IMP injection.

POST-TREATMENT period: The POST-TREATMENT observation period was defined as the time starting the day after the end of the TEAE period up to the end of the study

### Safety endpoints - Safety laboratory

The clinical laboratory data consisted of urinalysis and blood analysis, hematology (RBC count, red blood cell distribution width (RDW), reticulocyte count, hemoglobin, hematocrit, platelets, WBC count with differential blood count), standard chemistry (glucose, sodium, potassium, chloride, bicarbonate, calcium, phosphorous, urea nitrogen, creatinine, uric acid, total protein, LDH, albumin, γ Glutamyl Transferase [yGT]), Hepatitis C antibody, liver panel (ALT, AST, ALP, and total bilirubin), and CPK.

Safety endpoints - Vital signs measurement: Vital signs included: HR, systolic and diastolic BP in sitting position.

Other endpoints Anti-MAb316P Antibody Assessments: Anti-mAb316P antibodies included the antibody status (positive/negative) and antibody titers.

Sampling time: Serum samples for anti-mAb316P antibody determination were drawn periodically throughout the study. The first scheduled sample at randomization visit was obtained before IMP injection (predose).

Patients who have a titer at or above 240 for anti-mAb316P antibody at follow-up visit had an additional antibody sample(s), at 6 to 12 months after the last dose and thereafter about every 3 to 6 months until titer returned below 240. In order to maintain the blind of the study, the requests for sample collection of poststudy anti-mAb316P antibodies were made on patients with titers below 240 at the follow-up visit.

Sampling procedure: Five (5) ml blood volume was collected for each anti-mAb316P antibody sample.

Bioanalytical method: All anti-mAb316P antibody (ADA; anti-drug antibody) samples were analyzed.

Anti-mAb316P antibody samples were analyzed using a validated non-quantitative, titer-based bridging immunoassay. It involved an initial screen, a confirmation assay based on drug specificity, and a measurement of the titer of anti-MAb316P antibodies in the sample. The lower limit of detection was approximately 1.5 ng/mL.

Samples that were positive in the ADA assay were assessed for neutralizing antibodies using a validated, non-quantitative, competitive ligand binding assay. The lower limit of detection based on a monoclonal positive control neutralizing antibody is 390 ng/mL.

hs-CRP: The percent change in hs-CRP from baseline to Week 12 and Week 24.

HbA1C: The absolute change in HbA1c (%) from baseline to Week 12 and Week 24.

EQ-5D Patient Questionnaire: EQ-5D is a standardized measure of health status developed by the EuroQol Group in order to provide a simple, generic measure of health for clinical and economic appraisal. The EQ-5D as a measure of health related quality of life, defines health in terms of 5 dimensions: mobility, self-care, usual activities, pain/discomfort, anxiety/depression. Each dimension can take one of three responses (3 ordinal levels of severity): 'no problem' (1) "some problems" (2) "severe problems" (3). Overall health state is defined as a 5-digit number. Health states defined by the 5-dimensional classification can be converted into corresponding index scores that quantify health status, where 0 represents 'death' and 1 represents "perfect health". If response to one or more dimension was missing, the index score will be missing.

EQ-5D variables included response of each EQ-5D items, index score and change of index score from baseline.

Pharmacokinetics: Pharmacokinetic variables included total serum mAb316P concentration. If needed, total and free PCSK9 concentrations could be measured from the same PK sample.

Sampling time: Serum samples for total mAb316P concentration were collected before IMP (predose) at week 0 (randomization visit) and then at several visits until the end of the follow-up period. To collect information on the absorption phase, an optional PK sample was collected at 5 days (± 2) after the week 22 IMP injection or at 5 days (± 2) after any subsequent IMP injection while the subject was on treatment.

Sampling procedure: Five (5) ml blood volume was collected for each PK sample.

Bioanalytical method: All PK samples were analyzed for the determination of total mAb316P concentrations (ie, free mAb316P and mAb316P present in PCSK9:mAb316P complexes) using a validated enzyme-linked immunosorbent assay (ELISA). The lower limit of quantification (LLQ) for this assay is 0.078 µg/mL.

If needed, PK samples could be analyzed for the determination of the total and free PCSK9 levels using validated ELISA. The LLQ is 0.156 µg/mL for the total PCSK9 assay and 0.0312 µg/mL for the free PCSK9 assay.

### STUDY PROCEDURES

For all visits after Day 1/Week 0 (randomization visit), a timeframe of a certain number of days was allowed. The window period for visits at Weeks 12 and 24 was ± 3 days and for all other site visits it was ± 7 days during the double-blind treatment period, and follow-up period. A window period of + 3 days was allowed for the randomization visit (Day1/Week 0) and for the screening visit for injection training (Week -1).

Blood samplings: The blood sampling for determination of lipid parameters (ie, total-C, LDL-C, HDL-C, TG, non-HDL-C, Apo B, Apo A-1, ratio Apo B/Apo A-1, Lp [a]) should be performed in the morning, in fasting condition (ie overnight, at least 10 to 12 hours fast and refrain from smoking) for all site visits throughout the study. Alcohol consumption within 48 hours and intense physical exercise within 24 hours preceding the blood sampling were discouraged.

Laboratory tests: The laboratory data were collected and forwarded to the central laboratory, including hematology; chemistry; liver panel (in case of total bilirubin values above the normal range, differentiation into conjugated and non-conjugated bilirubin will occur automatically); creatine phosphokinase (CPK); Hepatitis B surface antigen; Hepatitis C antibody (positive tests were confirmed with reflexive testing); and serum pregnancy test.

Urine samplings: Urinalysis - dipstick was performed at the Central lab and assessed for pH, specific gravity, and for the presence of blood, protein, glucose, ketones, nitrates, leukocyte esterase, uro-bilinogen and bilirubin. If the dipstick was abnormal then standard microscopy will be conducted.

Other endpoints assessment methods: All other blood parameters were measured by a Central Laboratory during the study. Alcohol consumption within 48 hours and intense physical exercise within 24 hours preceding the blood sampling were discouraged. Glycemic parameters (HbA1c and serum glucose) was measured by a Central laboratory, periodically throughout the study. The blood sampling for inflammatory parameter, hs-CRP was collected periodically throughout the study.

Pharmacokinetic samples: Serum samples for assessment of mAb316P concentration were obtained periodically throughout the study.

Physical examination: A general physical examination should have been performed.

Blood pressure (BP)/heart rate: BP should be measured in sitting position under standardized conditions, approximately at the same time of the day, on the same arm, with the same apparatus (after the patient has rested comfortably in sitting position for at least 5 minutes). Values were to be recorded in the e-CRF; both systolic BP and diastolic BP should be recorded. At the first screening visit, BP should be measured in both arms. The arm with the highest diastolic pressure will be determined at this visit, and BP should be measured on this arm throughout the study. This highest value will be recorded in the e-CRF. Heart rate will be measured at the time of the measurement of BP.

Electrocardiogram: The 12-lead ECGs should be performed after at least 10 minutes rest and in the supine position.

Body weight and height: Body weight should be obtained with the patient wearing undergarments or very light clothing and no shoes, and with an empty bladder.

### STATISTICAL ANALYSIS

A sample size of 45 patients per treatment arm was calculated to have 95% power to detect a mean difference between mAb316P and ezetimibe of 20% in LDL-C percent change from baseline to week 24 using a 2-sided *t*-test with 5% significance, assuming a common standard deviation (SD) of 25% based on a previous mAb316P trial (McKenney et al., "Safety and efficacy of a monoclonal antibody to proprotein convertase subtilisin/kexin type 9 serine protease, SAR236553/REGN727, in patients with primary hypercholesterolemia receiving ongoing stable atorvastatin therapy", J Am Coll Cardiol, vol. 59, pp. 2344-2353 (2012)) and with an expected rate of exclusion of 5%.

The primary endpoint was assessed in the intent-to-treat (ITT) population, which included all randomized patients who had at least 1 calculated LDL-C value at baseline and at one of the planned time points from weeks 4 to 24. An on-treatment analysis (corresponding to the modified ITT or mITT) was also carried out which included all randomized and treated patients who had at least 1 calculated LDL-C value at baseline and at one of the planned time points from weeks 4 to 24 on-treatment, defined as the period between the first dose of study treatment and up to 21 days after last injection or 3 days after last capsule intake, whichever came first.

Missing data were accounted for by means of a mixed effect model with repeated measures (MMRM) approach (Siddiqui et al., MMRM vs. LOCF: a comprehensive comparison based on simulation study and 25 NDA datasets", J Biopharm Stat, vol. 19, pp. 227-246 (2009); National Research Council, "The Prevention and Treatment of Missing Data in Clinical Trials" Panel on Handling Missing Data in Clinical Trials. Committee on National Statistics, Division of Behavioral and Social Sciences and Education. 2010. Washington, DC: The National Academies Press; Andersen et al., "On the practical application of mixed effects models for repeated measures to clinical trial data", Pharm Stat., vol. 12, pp.7-16 (2013)). For the ITT analysis, all available measurements at planned time points from weeks 4 to 24 (meaning week 4, 8, 12, 16, and 24), whatever their status on or off-treatment, were used in the MMRM. One MMRM model was used for LDL-C to provide least-squares means estimates and comparison between treatment arms at weeks 24 and 12. Further details on the model are given below. For the on-treatment analysis, all available on-treatment measurements (i.e. up to 21 days after last injection/3 days after last capsule, whichever comes first) at planned time points from weeks 4 to 24 were used in the MMRM model. In the same way as for the ITT analysis, one model was used to provide estimates and comparison at week 24 and week 12 for the on-treatment analysis. Continuous key secondary endpoints other than Lp(a) and triglycerides were analyzed in a similar fashion as the primary endpoint and description of the statistical methodology for secondary endpoints and subgroup analysis is provided below.

The safety analysis included all randomized and treated patients. Safety data were analyzed by descriptive statistics. All statistical analyses were conducted using SAS® version 9.2 or higher (SAS Institute Inc., Gary, NC).

### Mixed effect model with repeated measures (MMRM)

The MMRM included fixed categorical effects of treatment group (mAb316P versus ezetimibe), time point (weeks 4, 8, 12, 16, and 24) and treatment-by-time point interaction, as well as the continuous fixed covariates of baseline low-density lipoprotein cholesterol (LDL-C) value and baseline value-by-time point interaction. Outputs from the model were the baseline-adjusted least-squares (LS) mean estimates at week 24 for both treatment groups with their corresponding standard error (SE). Appropriate contrast statement was used to test the difference between these estimates at the 2-sided 5% alpha level. To assess the robustness of the primary analysis and to compare on-treatment results between groups, the MMRM model was also applied to LDL-C values collected on-treatment.

### Subgroup analysis

To assess the homogeneity of the treatment effect across various subgroups, treatment-by-subgroup factor, time point-by-subgroup factor and treatment-by time point-by subgroup factor interaction terms and a subgroup factor term were added to the primary MMRM model. Subgroups of interest included body mass index (BMI) ≥30 kg/m²; gender; region (North America, Western Europe); age ≥65 years; baseline LDL-C (≥130 or ≥160 mg/dL); baseline high-density lipoprotein cholesterol (HDL-C) <40 mg/dL; baseline fasting triglycerides ≥150 mg/dL; baseline lipoprotein(a) [Lp(a)] ≥30 mg/dL; and baseline free proprotein convertase subtilisin/kexin type 9 (PCSK9) levels (below/above the median).

### Statistical analysis of key secondary endpoints

To analyze the key secondary endpoints, a hierarchical procedure was used to control type I error and handle multiplicity. Secondary efficacy endpoints were tested sequentially using the order given above, in the intent-to-treat population. Continuous key secondary endpoints (including secondary endpoints at Week 12), except Lp(a) and triglycerides, were analyzed using the same MMRM model as for the primary endpoint with fixed categorical effects of treatment group, planned time points up to week 24 and treatment-by-time point interaction, as well as the continuous fixed covariates of corresponding baseline value and baseline value-by-time point interaction. Lp(a) and triglycerides (which have a non-Gaussian distribution) and the binary endpoints (proportion of patients with LDL-C <100 mg/dL and <70 mg/dL) were analyzed using a multiple imputation approach for handling of missing values. For Lp(a) and triglycerides, multiple imputation was followed by robust regression model with treatment groups and corresponding baseline value as effects. For binary endpoints, multiple imputation was followed by logistic regression with treatment group as effect and corresponding baseline value as covariate. A sensitivity analysis of key secondary endpoints was applied using the same statistical approach as described above using on-treatment values.

### RESULTS

Of 204 patients screened, 103 met the eligibility criteria for the study and were randomized (52 to the mAb316P arm and 51 to the ezetimibe arm; **Figure 2**). Baseline characteristics and lipid parameters were generally evenly distributed across the 2 study arms **(Table 1).** A total of 4 patients were identified as having diabetes mellitus at screening (3 in the mAb316P arm and 1 in the ezetimibe arm). Mean baseline LDL-C levels were 141.1 mg/dL (3.65 mmol/L) in the mAb316P arm and 138.3 mg/dL (3.58 mmol/L) in the ezetimibe arm **(Table 1).**

Fourteen patients in the mAb316P arm were up-titrated in a blinded manner at week 12 to the 150 mg Q2W dosing regimen because their week 8 LDL-C was ≥70 mg/dL; only one of these patients had LDL-C >100 mg/dL. Mean baseline LDL-C values were 153.2 mg/dL (3.96 mmol/L) in patients who were up-titrated to mAb316P 150 mg Q2W and 134.7 mg/dL (3.48 mmol/L) in patients who were not up-titrated. Baseline values of other lipid values according to whether patients were up-titrated or not are shown in **Table 2.**

Overall, 44/52 (85%) patients in the mAb316P arm and 44/51 (86%) patients in the ezetimibe arm completed the 24-week treatment period **(****Figure 2****).** The main reason for study treatment discontinuation was TEAEs in both treatment arms **(****Figure 2****).** Of the 15 patients who prematurely discontinued treatment, 3 (6%) patients in the mAb316P arm and 5 (10%) patients in the ezetimibe arm did not have a calculated LDL-C value at week 24.

Forty-eight patients in each arm self-injected for all injections (94% in the ezetimibe arm, 92% in the mAb316P arm). Three patients in the ezetimibe arm and 4 in the mAb316P arm self-injected for some of the injections and requested another person to do so for the other injections. No patients asked another person to perform all their injections.

All randomized patients received at least 1 dose of their allocated drug and were included in the intention-to-treat (ITT) and safety populations **(****Figure 2****).** One patient from each treatment arm withdrew from treatment before any post-randomization LDL-C measurements were made and so were excluded from the on-treatment analysis. However, they continued the study and had LDL-C measurements taken while off-treatment but before end of 24-week study period, so were included in the ITT analysis.

**Table 1. Baseline Characteristics (All Randomized Patients)**

| Characteristic (mean [SD] unless otherwise stated) | Ezetimibe 10 mg | MAb316P 75 mg Q2W |
|---|---|---|
| | (N=51) | (N=52) |
| Age, years | 59.6 (5.3) | 60.8 (4.6) |
| ≥65 years, n (%) | 8 (15.7) | 11 (21.2) |
| Male gender, n (%) | 27 (52.9) | 28 (53.8) |
| Race, n (%) | | |
| White | 47 (92.2) | 46 (88.5) |
| Black or African American | 4 (7.8) | 6 (11.5) |
| BMI, kg/m² | 28.4 (6.7) | 30.1 (5.9) |
| HbA1c, % | 5.6 (0.4) | 5.7 (0.5) |
| Fasting blood glucose, mg/dL | 97.4 (9.0) | 101.4 (14.3) |
| Time from hypercholesterolemia diagnosis, years | 4.5 (7.8) | 4.0 (4.7) |
| 10-yr risk of fatal CVD (SCORE), % | 2.68 (1.14) | 2.97 (1.29) |
| Lipid parameters, mg/dL | | |
| LDL-C | 138.3 (24.5) | 141.1 (27.1) |
| Range (min : max) | 73 : 186 | 77 : 207 |
| Apolipoprotein B | 104.3 (19.1) | 104.3 (18.4) |
| Total cholesterol | 223.9 (30.2) | 221.7 (33.7) |
| Non-HDL-C | 164.0 (29.7) | 167.4 (30.3) |
| Lipoprotein(a), median (IQR) | 16.0 (6.0:34.0) | 13.0 (4.0:39.0) |
| Triglycerides, median (IQR) | 117.0 (87.0:154.0) | 119.0 (89.0:153.0) |
| HDL-C | 59.9 (19.2) | 54.3 (16.1) |
| Apolipoprotein A-1 | 163.8 (33.4) | 153.1 (29.2) |

| | | |
|---|---|---|
| A total of 4 patients were identified as having diabetes mellitus at screening (3 in the mAb316P arm and 1 in the ezetimibe arm). There were no clinically or statistically significant between group differences. To convert glucose measurements to mmol/L, multiply by 0.0555; to convert cholesterol measurements to mmol/L, multiply by 0.02586; to convert triglycerides measurements to mmol/L, multiply by 0.01129. BMI, body mass index; HbA1c, glycated hemoglobin; HDL-C, high-density lipoprotein cholesterol; IQR, interquartile range; LDL-C, low-density lipoprotein cholesterol; Q2W, every 2 weeks; SCORE, Systemic Coronary Risk estimation; SD, standard deviation. | | |

**Table 2. Lipid Parameters at Baseline According to Up-titration Status (Patients from Safety Population with at Least One Injection Post-Week 12)**

| Lipid parameter (mg/dL) | Not up-titrated in mAb316P group | Up-titrated in mAb316P group |
|---|---|---|
| | (N=32) | (N=14) |
| LDL-C | | |
| Mean (SD) | 134.7 (26.7) | 153.2 (24.6)* |
| Range (Min : Max) | 77 : 206 | 122 : 207 |
| HDL-C | | |
| Mean (SD) | 51.3 (16.3) | 60.3 (15.2)^{†} |
| Range (Min : Max) | 30 : 98 | 33 : 96 |
| Total cholesterol | | |
| Mean (SD) | 215.1 (32.9) | 236.6 (30.6)^{‡} |
| Range (Min : Max) | 159 : 289 | 184 : 296 |
| Non-HDL-C | | |
| Mean (SD) | 163.7 (32.5) | 176.4 (24.0)^{§} |
| Range (Min : Max) | 100: 233 | 144: 231 |
| Fasting triglycerides | | |
| Mean (SD) | 145.3 (80.5) | 115.4 (34.1)^{∥} |
| Range (Min : Max) | 36 : 373 | 62 : 183 |

| | | |
|---|---|---|
| *P*-values versus patients not up-titrated: **P*=0.0436; ^{†}*P*=0.0280; ^{‡}*P*=0.0546; ^{§}*P*=0.2989, ^{∥}*P*=0.3455. P-values were not adjusted for multiplicity and are for descriptive purposes only. HDL-C, high-density lipoprotein cholesterol; LDL-C, low-density lipoprotein cholesterol; SD, standard deviation. | | |

### Efficacy results

For the primary efficacy analysis (ITT analysis), least-squares (LS) mean (SE) percent reductions in LDL-C from baseline to week 24 were 47 (3)% in the mAb316P group versus 16 (3)% in the ezetimibe group, with a statistically significant LS mean (SE) difference between groups of-32 (4)% (P<0.0001) **(Table** 3). Results from the on-treatment analysis were similar to those from the ITT analysis: LS mean (SE) LDL-C reductions from baseline to week 24 were 54 (2)% versus 17 (2)% (*P*<0.0001), with mAb316P and ezetimibe, respectively **(Table** 3).

At week 12, when all patients in the mAb316P arm were receiving 75 mg Q2W, LDL-C levels were reduced by 48 (3)% with mAb316P versus 20 (3)% with ezetimibe in the ITT analysis, with a between-group LS mean (SE) difference of-28 (4)% (*P*<0.0001). Corresponding LDL-C reductions in the on-treatment analysis at week 12 were 53 (2)% with mAb316P versus 20 (2)% with ezetimibe, with a between-group LS mean (SE) difference of -33 (3)%.

**Figure 3** shows the time-course of changes in LDL-C levels over the study period for patients treated with mAb316P and ezetimibe. Here is shown the on-treatment values since the purpose is to understand the durability of drug effect without any confounding by drop out. There was a dramatic drop in LDL-C from baseline to week 4 in the patients who received mAb316P, with robust LDL-C reductions maintained from week 4 to end of the treatment period at week 24. Statistical analysis of the interaction between treatment and time point in the MMRM model was not significant, suggesting stability of LDL-lowering effect of mAb316P versus ezetimibe over time (as illustrated in **Figure 3**).

The estimated proportions of patients with LDL-C reductions ≥50% at week 12, before up-titration, were 58% in the mAb316P arm, compared with 3% of patients in the ezetimibe arm (ITT). Corresponding values in the on-treatment analysis were 65% in the mAb316P arm and 2% in the ezetimibe arm. All patients responded to mAb316P while exposed to treatment (on-treatment population) (**Figure 4**).

To estimate the impact of the up-titration based on LDL-C ≥70 mg/dL instead of ≥100 mg/dL on the primary efficacy parameter, an additional analysis was performed excluding LDL-C values post up-titration for the 13 patients who were up-titrated despite having LDL-C values <100 mg/dL; this analysis gave results similar to the overall ITT analysis (**Table 4**).

Percent reductions from baseline in Apo B, total cholesterol, and non-HDL-C were significantly greater for mAb316P versus ezetimibe at week 24 and similar in the ITT and on-treatment analyses (**Table 5**). Moderate reductions in Lp(a), TGs and increases in HDL-C were observed following both of the study treatments, with no differences between mAb316P and ezetimibe arms (**Table 5**).

Subgroup analyses suggested no major differences on mAb316P efficacy versus ezetimibe in the ITT population for various parameters (**Figure 5**).

**Table 3. Percent Change in LDL-C from Baseline to Week 24 (ITT or On-Treatment analysis as Indicated)**

| | | | MAb316P versus ezetimibe | | |
|---|---|---|---|---|---|
| LDL-C | Ezetimibe 10 mg | MAb316P 75 mg Q2W | LS mean difference (SE) % | 95% Cl | *P*-value |
| ITT | N=51 | N=52 | | | |
| _S mean (SE) change from baseline (%) | -15.6(3.1) | -47.2 (3.0) | -31.6(4.3) | -40.2 to - 23.0 | <0.0001* |
| On-treatment^{†} | N=50 | N=51 | | | |
| Baseline LDL-C, mean (SD), mg/dL | 137.5 (24.1) | 141.1 (27.4) | | | |
| Min : Max | 73 : 186 | 77 : 207 | | | |
| _S mean (SE) change from baseline (%) | -17.2 (2.0) | -54.1 (2.0) | -36.9 (2.9) | -42.7 to - 31.2 | <0.0001^{‡} |

| | | | | | |
|---|---|---|---|---|---|
| *Statistically significant according to the fixed hierarchical approach used to control overall type-I error rate. ^{†}Includes all patients in the ITT population with at least 1 calculated LDL-C value at one planned time point between the first dose of study treatment and up to 21 days after last injection or 3 days after last capsule intake, whichever came first. ^{‡} *P*-value is shown for descriptive purposes only. Cl, confidence intervals; ITT, intent-to-treat; LDL-C, low-density lipoprotein cholesterol; LS, least squares; Q2W, every 2 weeks; SD, standard deviation; SE, standard error. | | | | | |

**Table 4. Percent Change in LDL-C from Baseline to Week 24 Excluding Post Up-titration Data from Patients Who Were Up-Titrated from MAb316P 75 mg to 150 mg Q2W Having LDL-C <100 mg/dL (Intent-to-Treat Population)**

| | | | MAb316P versus ezetimibe | | |
|---|---|---|---|---|---|
| LDL-C, LS mean (SE) | Ezetimibe 10 mg (N=51) | MAb316P 75 mg Q2W (N=52) | LS mean difference (SE) % | 95% Cl | P-value |
| Week 24 change from baseline (%) | -15.6 (3.2) | -44.3(3.4) | -28.7 (4.6) | -37.9 to - 19.5 | <0.0001^{‡} |

| | | | | | |
|---|---|---|---|---|---|
| ^{‡} *P*-value is shown for descriptive purposes only. Cl, confidence interval; LDL-C, low-density lipoprotein cholesterol; LS, least squares; Q2W, every 2 weeks. | | | | | |

**Table 5. Percent Change from Baseline in Secondary Lipid Parameters (ITT or On-Treatment analysis as indicated)**

| | | | MAb316P versus ezetimibe | | |
|---|---|---|---|---|---|
| LS mean (SE) % change from baseline to week 24 | Ezetimibe 10mg | MAb316P 75 mg Q2W | LS mean difference (SE) % | 95% Cl | *P*-value |
| ITT | N=51 | N=52 | | | |
| Apo B | -11.0 (2.4) | -36.7 (2.3) | -25.8 (3.3) | -32.3 to -19.2 | <0.0001* |
| Non-HDL-C | -15.1 (2.9) | -40.6 (2.8) | -25.5 (4.1) | -33.5 to -17.4 | <0.0001* |
| Total cholesterol | -10.9 (2.2) | -29.6 (2.1) | -18.7 (3.0) | -24.7 to -12.7 | <0.0001* |
| Lp(a)^{†} | -12.3 (3.8) | -16.7 (3.7) | -4.4 (5.3) | -14.8 to 5.9 | 0.4013 |
| TGs^{†} | -10.8 (4.3) | -11.9 (4.2) | -1.2 (5.9) | -12.7 to 10.3 | 0.8433^{‡} |
| HDL-C | 1.6 (1.9) | 6.0 (1.9) | 4.4 (2.7) | -1.0 to 9.8 | 0.1116^{‡} |
| Apo A-1 | -0.6 (1.6) | 4.7 (1.6) | 5.3 (2.2) | 0.9 to 9.8 | 0.0196^{‡} |
| On-treatment | N=50 | N=51 | | | |
| Apo B | -11.5 (1.9) | -40.8 (1.9) | -29.2 (2.6) | -34.4 to -24.0 | <0.0001^{§} |
| Non-HDL-C | -16.6 (1.9) | -47.1 (1.9) | -30.5 (2.7) | -35.9 to -25.1 | <0.0001^{§} |
| Total cholesterol | -12.0 (1.6) | -34.2 (1.6) | -22.2 (2.3) | -26.7 to -17.7 | <0.0001^{§} |
| Lp(a)^{†} | -12.3 (4.0) | -17.7 (4.1) | -5.4 (5.7) | -16.6 to 5.9 | 0.3506^{§} |
| TGs^{†} | -12.7 (4.2) | -14.7 (4.4) | -1.9 (6.0) | -13.7 to 9.8 | 0.7452^{§} |
| HDL-C | 1.7 (1.9) | 8.0 (1.9) | 6.2 (2.7) | 0.8 to 11.6 | 0.0241^{§} |
| Apo A-1 | -0.7 (1.6) | 5.3 (1.6) | 6.1 (2.3) | 1.6 to 10.6 | 0.0084^{§} |

| | | | | | |
|---|---|---|---|---|---|
| *Statistically significant according to the fixed hierarchical approach used to control overall type-I error rate. ^{†}Combined estimate for adjusted mean (SE) percent changes are shown for Lp(a) and TGs. ^{‡}As the difference in Lp(a) at week 24 was not significant for mAb316P versus ezetimibe, no further significance testing was performed as per the fixed hierarchical approach. *P*-values for TGs, HDL-C and Apo A-1 are shown for descriptive purposes only. ^{§}*P*-values are shown for descriptive purposes only. Apo, apolipoprotein; Cl, confidence intervals; HDL-C, high-density lipoprotein cholesterol; ITT, intention-to-treat; Lp(a), lipoprotein(a); LS, least squares; Q2W, every 2 weeks; SE, standard error; TGs, triglycerides. | | | | | |

### Safety results

The overall percentage of patients who experienced at least one TEAE was 69% in the mAb316P arm and 78% in the ezetimibe arm (**Table 6**). There were no deaths. Two SAEs were reported during the TEAE period: one patient, who had received mAb316P 75 mg Q2W for 3 months and had a history of atrial fibrillation and chronic obstructive pulmonary disorder, experienced a pulmonary embolism; study treatment was discontinued and the patient was hospitalized, where he recovered. One patient in the ezetimibe arm with a medical history of arthritis experienced glenoid erosion and was hospitalized for surgery (shoulder arthroplasty). The patient recovered in hospital and completed the study. Neither of the SAEs were considered by the investigator to be related to the study treatment. TEAEs occurring in 5% or more patients in either treatment arms are shown in **Table 6.**

Nine patients prematurely discontinued study treatment following one or more TEAEs (5 [10%] patients in the mAb316P arm and 4 [8%] in the ezetimibe arm). In the ezetimibe group, the TEAEs leading to discontinuation were gout in 1 patient, fatigue, back pain, and frequent urination in 1 patient, abdominal cramping and injection site reaction in 1 patient, and vivid dreams in 1 patient. In the mAb316P group, TEAEs leading to discontinuation were pulmonary embolism in 1 patient, nausea, fatigue, headache, and flushing in 1 patient, arthralgia (generalized aching) in 1 patient, injection site reaction in 1 patient and diarrhea in 1 other patient.

The most common class of TEAE was infection (42.3% mAb316P vs 39.2% ezetimibe), mostly respiratory. Muscle-related TEAEs occurred in 2 (4%) of mAb316P patients and 2 (4%) of ezetimibe patients. Elevated creatine kinase levels over 10 times the upper limit of normal were reported in 1 patient in the ezetimibe group **(Table** 6). Three patients experienced a local injection site reaction (1 [2%] patient in the mAb316P group and 2 [4%] in the ezetimibe group). These events were of mild intensity. The patient in the mAb316P arm experienced 3 episodes of local injection site reaction following consecutive injections. Three patients who were treated with mAb316P 75 mg Q2W experienced at least 1 LDL-C value <25 mg/dL; no particular safety concern associated with the low LDL-C levels was observed with these 3 patients.

Few (2 or less) patients in the ezetimibe group and no patients in the mAb316P group presented abnormalities in vital signs (blood pressure, heart rate). In addition, there were no increases over 3 times the upper limit of normal in alanine aminotransferase or aspartate aminotransferase (**Table 6**). More patients had blood glucose ≥126 mg/dL (7 mmol/L) in the mAb316P arm than in the ezetimibe arm (6 patients versus 1 patient; **Table 6**). The 6 patients in the mAb316P arm who experienced high blood glucose during the treatment period had abnormal fasting glucose at screening or baseline and no pattern was observed in changes in either blood glucose or HbA1c from screening to week 24 (**Table 7**).

Treatment emergent anti-drug antibodies were found in 6 (12%) patients in the mAb316P arm and were not observed in patients in the ezetimibe arm. For all anti-drug antibody-positive patients, titers were low (≤240 in the assay used) and no neutralizing anti-drug antibody which may impact mAb316P pharmacokinetics, LDL-C effects, or safety were detected.

**Table 6. TEAEs and Laboratory Parameters (Safety Population)**

| AE category or lab parameter, n (%) | Ezetimibe 10 mg | MAb316P 75 mg Q2W |
|---|---|---|
| | (n=51) | (n=52) |
| Patients with any TEAE | 40 (78.4) | 36 (69.2) |
| Patients with any treatment emergent SAE | 1 (2.0) | 1 (1.9) |
| Patients with any TEAE leading to death | 0 | 0 |
| Patients with any TEAE leading to treatment discontinuation | 4 (7.8) | 5 (9.6) |
| TEAEs occurring in ≥5% of patients in either group | | |
| Nasopharyngitis | 8 (15.7) | 12 (23.1) |
| Diarrhea | 2 (3.9) | 6 (11.5) |
| Influenza | 3 (5.9) | 6 (11.5) |
| Arthralgia | 2 (3.9) | 3 (5.8) |
| Headaches | 2 (3.9) | 3 (5.8) |
| Nausea | 3 (5.9) | 3 (5.8) |
| Upper respiratory tract infection | 5 (9.8) | 2 (3.8) |
| Back pain* | 3 (5.9) | 1 (1.9) |
| Dizziness | 3 (5.9) | 1 (1.9) |
| Urinary tract infection | 3 (5.9) | 0 |
| Patients with TEAEs of interest | | |
| Musculoskeletal and connective tissue disorders | 11 (21.6) | 8(15.4) |
| Muscle disorders | 2 (3.9) | 2 (3.8) |
| Myalgia | 1 (2.0) | 2 (3.8) |
| | | |
| Muscle spasms | 1 (2.0) | 0 |
| Musculoskeletal and connective tissue disorders NEC | 5 (9.8) | 2 (3.8) |
| Musculoskeletal pain | 1 (2.0) | 1 (1.9) |
| General disorders and administration site conditions | 5 (9.8) | 5 (9.6) |
| Injection site reaction | 2 (3.9) | 1 (1.9) |
| **Laboratory parameters n/N (%)** | | |
| Alanine aminotransferase (ALT) | | |
| ≥3× ULN (if baseline ALT <ULN) or ≥2× the baseline value (if baseline ALT ≥ULN) | 0/51 | 0/52 |
| >3× ULN | 0/51 | 0/52 |
| Aspartate aminotransferase | | |
| >3× ULN | 0/51 | 0/52 |
| Glucose | | |
| ≤70 mg/dL (3.9 mmol/L) and <LLN | 0/50 | 0/52 |
| ≥126 mg/dL (7 mmol/L) (fasted) | 1/50 (2.0) | 6/51 (11.8)** |
| Albumin | | |
| ≤25 g/L | 0/50 | 0/51 |
| Creatine kinase | | |
| >3× ULN | 1/50 (2.0) | 0/51 |
| >10× ULN | 1/50 (2.0) | 0/51 |

| | | |
|---|---|---|
| *Back pain was also counted as a TEAE of special interest (musculoskeletal and connective tissue disorders NEC). ** Three of these patients were identified as having diabetes mellitus at screening and all 6 patients had abnormal fasting blood glucose at screening or baseline; no pattern was observed in changes in blood glucose over time (See Supplementary Table 6). TEAEs are adverse events that developed or worsened or became serious during the TEAE period (defined as the time from the first dose of double-blind study treatment to the last injection plus 70 days [10 weeks], as residual effect of mAb316P was expected until 10 weeks after last injection. LLN, lower limit of normal; NEC, not elsewhere classified; Q2W, every 2 weeks; SAE, serious adverse event; TEAE, treatment-emergent adverse event; and ULN, upper limit of normal. | | |

**Table 7. Blood Glucose Levels for Patients in the MAb316P Arm Who Reported High Blood Glucose During the Study (n=6)**

| Patient (arbitrary number) | Timepoint | Fasting blood glucose (mg/dL) | HbA1c (%) |
|---|---|---|---|
| 1 | Screening (Week -2) | 121 | 5.7 |
| | Baseline (Week 0) | 115 | |
| | Week 24 | 112 | 5.9 |
| 2 | Screening (Week-2) | 128 | 6.2 |
| | Baseline (Week 0) | 119 | |
| | Week 24 | 121 | 6.5 |
| 3* | Screening (Week -2) | 126 | 6.2 |
| | Week 24 | 112 | 5.9 |
| 4* | Screening (Week -2) | 105 | 7.0 |
| | Baseline (Week 0) | 97 | |
| | Week 24 | 70 | 6.2 |
| 5* | Screening (Week -2) | 164 | 7.5 |
| | Baseline (Week 0) | 142 | |
| | Week 24 | 183 | 7.7 |
| 6 | Screening (Week -2) | 125 | 6.7 |
| | Baseline (Week 0) | 129 | |
| | Week 12 | 135 | 6.3 |

| | | | |
|---|---|---|---|
| Values were not available at all timepoints for all patients. *Identified as having diabetes mellitus at screening. | | | |

This was the first Phase 3 study of mAb316P, the first monotherapy study of mAb316P, and the first study to use the 75 mg Q2W dosing regimen. MAb316P demonstrated superior efficacy in monotherapy compared with ezetimibe over 24 weeks of treatment. LDL-C reductions of 48% were observed at week 12 (before up-titration) in patients who received mAb316P 75 mg Q2W, compared with 20% for ezetimibe 10 mg daily, in the ITT analysis. Corresponding reductions for patients who were on-treatment were 53% with mAb316P 75 mg Q2W versus 20% with ezetimibe.

MAb316P efficacy was consistent across baseline parameters including LDL-C and PCSK9 levels, and demographics. Indeed, all mAb316P-treated patients responded to 75 mg Q2W in monotherapy in the on-treatment analysis. MAb316P monotherapy showed a sustained LDL-lowering effect from weeks 4 to 24.

Consistent with the marked reductions in LDL-C, mAb316P also provided robust reductions in total cholesterol, Apo B and non-HDL-C. MAb316P resulted in an 18% reduction in Lp(a) in the current study, compared with 12% in the ezetimibe arm.

MAb316P demonstrated tolerability and safety comparable with ezetimibe. Muscle-related AEs occurred in a similar frequency in both treatment arms (4% of mAb316P and 4% of ezetimibe patients). Furthermore, development of anti-drug antibodies in mAb316P treated patients was low.

To summarize, this is the first 6-month duration, Phase 3, blinded assessment of an anti-PCSK9 antibody. A reduction in LDL-C of -50% was observed in the mAb316P 75mg Q2W arm at 12 weeks in a monotherapy population, which was substantially greater than what was observed in the ezetimibe arm (20%). This study extends the safety observations from the Phase 2 trials, with no evidence of safety signals that appear to limit use and tolerability. This was also the first randomized, controlled trial of an injectable monoclonal antibody to PCSK9 utilizing a disposable autoinjector, which resulted in few injection related AEs (<2% of mAb316P and <4% ezetimibe patients).

### Pharmacokinetic Results

The relationship between mAb316P, free PCSK9, and LDL-C concentrations in patients receiving mAb316P monotherapy was assessed. Changes in free PCSK9 and LDL-C were also assessed in the ezetimibe arm.

Serum mAb316P and free PCSK9 levels were determined using validated enzyme-linked immunosorbent assays.

Pharmacokinetic analyses were conducted in 46 patients in the mAb316P treatment arm who had not discontinued prior to Week 12 (**Table 8**). Of the 46 mAb316P-treated patients, 32 patients achieved LDL-C <70 mg/dL at Week 8 and were maintained on mAb316P 75 mg Q2W, and 14 patients were uptitrated to mAb316P 150 mg Q2W.

Baseline LDL-C levels were greater in uptitrated mAb316P patients compared with non-uptitrated mAb316P patients (P=0.044) (**Table 9**). In the non-uptitrated group, LDL-C reductions observed at Week 4 were sustained until Week 24 (**Figure 6A**)**.** In the uptitrated group, LDL-C levels were also reduced by Week 4, but to a lesser relative extent than the non-uptitrated group (**Figure 6A**). Reductions in LDL-C with mAb316P were similar at Weeks 12 and 24, regardless of whether mAb316P dose was uptitrated at Week 12 (**Figure 6A**), and were consistently greater than with ezetimibe (**Table 9**).

Serum mAb316P levels were similar in the uptitrated and non-uptitrated groups during the first 12 weeks of the study when all patients received the 75 mg dose, and approximately doubled in patients who were uptitrated at Week 12 (**Figure 6B**; **Table 9**).

Baseline free and total PCSK9 levels were slightly greater in the uptitrated group compared with the non-uptitrated and ezetimibe groups (**Table 9**). Free PCSK9 levels reached the lowest point at Week 4 in the non-uptitrated group; free PCSK9 levels were also reduced at Weeks 4-12 in the uptitrated group but were 2-3 times higher than in the non-uptitrated group (**Figure 6C**; Table 9). Uptitration to mAb316P 150 mg Q2W at Week 12 was associated with additional suppression of free PCSK9 levels; reductions were maintained to Week 24 (**Figure 6C****; Table 9**). Free PCSK9 levels remained relatively unchanged from baseline to Week 12 in the ezetimibe group, but were reduced at Week 24 (**Table 9**). Total PCSK9 levels increased following treatment with mAb316P but not ezetimibe (**Table 9**), possibly because the antibody:PCSK9 complex had a longer half-life than free PCSK9.

In patients maintained on mAb316P 75 mg Q2W monotherapy until Week 24, free PCSK9 levels were maximally reduced by Week 4; LDL-C lowering efficacy was maintained from Week 4 to 24. In patients uptitrated to mAb316P 150 mg Q2W at Week 12, mAb316P serum concentrations increased as expected and there was an additional decrease in free PCSK9 levels, but there was little further effect on LDL-C. The results suggest that monotherapy with mAb316P 75 mg Q2W is sufficient to block free PCSK9 and maintain LDL-C lowering efficacy over 24 weeks in most patients not receiving a statin or other lipid modifying therapies. As target-mediated clearance of mAb316P is accelerated by its binding to free PCSK9, higher doses of mAb316P may be required in the clinical setting where patients are receiving concomitant statin or other lipid modifying agents increasing free PCSK9.

The present invention is not to be limited in scope by the specific embodiments described herein. Indeed, various modifications of the invention in addition to those described herein will become apparent to those skilled in the art from the foregoing description and the accompanying figure. Such modifications are intended to fall within the scope of the appended claims.

**Table 8. Mean (SD) baseline characteristics**

| | **MAb316P** | | |
|---|---|---|---|
| **Characteristic** | **Uptitrated* at Week 12 (n=14)** | **Non-uptitrated at Week 12 (n=32)** | **Ezetimibe (n=51)** |
| Age, years | 61.8 (3.9) | 60.2 (5.1) | 59.6(5.3) |
| Male gender, n (%) | 3 (21.4) | 22 (68.8) | 27 (52.9) |
| Race, white, n (%) | 13 (92.9) | 28 (87.5) | 47 (92.2) |
| BMI, kg/m² | 31.8 (6.4) | 29.8 (5.8) | 28.4 (6.7) |
| Type 2 diabetes, n (%) | 0 | 3 (9.4) | 1 (2.0) |
| 10-year risk of fatal CVD (SCORE), % | 3.2 (1.2) | 2.9 (1.4) | 2.68 (1.1) |

| | | | |
|---|---|---|---|
| *MAb316P dose was uptitrated in a blinded manner to 150 mg Q2W at Week 12 if Week 8 LDL-C was ≥70 mg/dL (∼1.8 mmol/L) BMI, body mass index; CVD, cardiovascular disease; SCORE, systematic coronary risk estimation; SD, standard deviation | | | |

**Table 9. Mean (SD) serum mAb316P, total and free PCSK9, and LDL-C concentrations**

| | **MAb316P** | | |
|---|---|---|---|
| | **Uptitrated* at Week 12 (n=14)** | **Non-uptitrated at Week 12 (n=32)** | **Ezetimibe (n=51)** |
| **LDL-C (ontreatment), mg/dL** | | | |
| Baseline | 153.2 (24.6) | 134.7 (26.7) | 137.5 (24.1) |
| Week 4 | 83.5 (23.6) | 59.8 (13.6) | 109.9 (22.4) |
| Week 12 | 78.3 (22.8) | 58.8 (16.8) | 109.8 (25.2) |
| Week 24 | 74.8 (19.5) | 57.8 (16.2) | 113.4 (25.0) |

| **MAb316P, ng/mL**** | | | |
|---|---|---|---|
| Week 4 | 4533.9 (3996.5) | 5784.7 (2456.4) | - |
| Week 12 | 5702.9 (4816.4) | 5979.0 (2780.3) | - |
| Week 24 | 14772.2 (10201.7) | 6991.4 (4418.4) | - |

| **Total PCSK9, ng/mL** | | | |
|---|---|---|---|
| Baseline | 551.6 (129.5) | 470.3 (144.7) | 496.1 (161.0) |
| Week 4 | 2948.2 (1284.8) | 3186.9 (634.1) | 508.8 (244.1) |
| Week 12 | 3762.3 (1588.3) | 3204.2 (988.8) | 645.5 (694.2) |
| Week 24 | 4680.8 (1297.2) | 3408.2 (969.7) | 482.0 (128.5) |

| **Free PCSK9, ng/mL** | | | |
|---|---|---|---|
| Baseline | 213.8 (38.3) | 178.4 (53.6) | 181.5 (61.5) |
| Week 4 | 65.9 (80.2) | 22.8 (37.4) | 180.1 (57.4) |
| Week 12 | 59.3 (76.9) | 40.4 (53.4) | 191.0 (65.0) |
| Week 24 | 14.3 (37.1) | 36.9 (55.0) | 138.5 (56.4) |

| | | | |
|---|---|---|---|
| To convert LDL-C mg/dL to mmol/L, multiply by 0.02586 *MAb316P dose was uptitrated in a blinded manner to 150 mg Q2W at Week 12 if Week 8 LDL-C was ≥70 mg/dL (∼1.8 mmol/L) **C_{trough} values were taken 14±6 days after previous injection C, concentration | | | |

### Example 3: A Randomized, Double-Blind Study of the Efficacy and Safety of an Anti-PCSK9 Antibody ("mAb316P") in Patients with Primary Hypercholesterolemia Who are Intolerant to Statins

### INTRODUCTION

The objective of the present study was to evaluate the ability of an anti-PCSK9 antibody ("mAb316P," also known as REGN727 or alirocumab) to reduce LDL-C in comparison with ezetimibe (EZE) in patients with primary hypercholesterolemia (heFH and non-FH) who are intolerant to statins.

Muscle-related symptoms in clinical trials, which involve highly selected patient groups with high treatment adherence and statin tolerance, may not reflect the true prevalence of myalgia in the clinic, as evidenced by findings in observational studies. The discrepancy of the incidence of muscle-related adverse events (AEs) may be secondary to some patients experiencing AEs with multiple medications, and this may not represent true intolerance to statins. Therefore, to account for patients who may have AEs on multiple medications and not just on statins, a sufficient number of patients were screened and enrolled in the single-blind placebo run-in period of the present study to ensure the double-blind treatment period sample size of 250 patients (100:100:50; mAb316P:EZE:atorvastatin).

The sample size of 250 patients (100:100:50; mAb316P:EZE:atorvastatin), with a double-blind study treatment duration of 24 weeks, was intended to obtain information on efficacy and to gain descriptive experience with safety events in general, all skeletal muscle-related events, and skeletal muscle event-related withdrawals in particular. Statin intolerant patients with moderate, high, or very high CV risk were included in this study, as a population in which LDL-C goal attainment is more difficult to reach when using only non-statin alternatives. The definition of CV risk is based on existing guidelines (See, *e.g*., The Task Force for the management of dyslipidaemias of the European Society of Cardiology [ESC] and the European Atherosclerosis Society [EAS], ESC/EAS Guidelines for the management of dislipidaemias. European Heart Journal 2011; 32:1769-1818); Expert Panel on Detection, Evaluation, and Treatment of High Blood Cholesterol in Adults. Executive summary of the Third Report of the National Cholesterol Education Program [NCEP] Expert Panel on Detection, Evaluation, and Treatment of High Blood Cholesterol in Adults [Adult Treatment Panel III], JAMA 2001; 285:2486-2497).

Use of a 2-week washout period ensured that there was no carry over effect of the background therapy on the comparative arms.

The 4-week placebo run-in period was used to eliminate patients who may develop muscle-related symptoms on other medications, including placebo.

Preliminary pharmacokinetic data from phase 2 studies of mAb316P showed that exposure to mAb316P declined during the 8-week follow-up period that followed the double-blind treatment period, with total serum concentrations of mAb316P still detectable, but at very low levels. Therefore, to ensure sufficient low, non-effective serum mAb316P concentrations, patients were followed during a follow-up period of 8 weeks (*i*.*e*., 10 weeks after the last dose).

Ezetimibe was used as an active comparator because it is the current standard of care for patients who are unable to tolerate statins, and the primary objective of the study was to evaluate the reduction of LDL-C after 24 weeks for mAb316P compared to EZE. Atorvastatin was also included as a comparator to determine that the population selected in the study is a truly statin intolerant population, by assessing the incidence of and frequency of discontinuation due to skeletal muscle-related AEs (Fung and Crook, Cardiovasc. Ther. 2012 Oct, 30(5):e212-218).

Two doses of mAb316P were used in the present study: 75 mg and 150 mg administered subcutaneously every other week (Q2W). The selection of doses was based on data from the phase 1 and phase 2 programs. The selection of dose, dosing frequency, and the up-titration approach was also based on the LDL-C reduction needed to provide the best benefit in terms of CVD reduction, and potential safety considerations regarding low LDL-C values.

The Q2W dosing regimen is expected to maintain constant LDL-C lowering throughout the inter-dosing interval, with the maximum efficacy at 12 weeks provided by the 150 mg Q2W dose. However, for many patients, the magnitude of effect observed with the 150 mg Q2W dose may not be necessary to achieve the LDL-C goal, and treatment may start with a lower dose. Therefore, in the present study, all patients were initially treated with 75 mg Q2W, and only those patients with high and very high CV risk and an LDL-C ≥70 mg/dL and patients with moderate CV risk and an LDL-C ≥100 mg/dl after 8 weeks of treatment were subjected to dose up-titration to 150 mg Q2W at week 12.

Based on the clinical data available at the time of commencement of the present study, treatment with mAb316P has demonstrated a significant LDL-C lowering effect and was generally well-tolerated in a patient population with non-familial hypercholesterolemia or with heterozygous familial hypercholesterolemia. The efficacy on LDL-C lowering was associated with consistent results in total-C, ApoB, non-HDL-C and ApoB/ApoA-1 ratio and a positive trend for HDL-C, TG and Lp(a). There was no evidence that mAb316P adversely affects other cardiovascular (CV) risk factors, e.g., body weight, blood pressure, glucose, or C-reactive protein.

### STUDY OBJECTIVES

The primary objective of the study was to demonstrate the reduction of LDL-C by mAb316P in comparison with EZE 10 mg orally once per day (PO QD) after 24 weeks in patients with primary hypercholesterolemia (heFH and non-FH) who were intolerant to statins.

The secondary objectives of the study were: (1) To evaluate the effect of mAb316P 75 mg in comparison with EZE on LDL-C after 12 weeks of treatment; (2) To evaluate the effect of mAb316P on other lipid parameters (e.g., ApoB, non-HDL-C, total-C, Lp(a), HDL-C, TG levels, and ApoA-1 levels); (3) To evaluate the safety and tolerability of mAb316P, including the characterization of the incidence rate and treatment withdrawal rate of skeletal muscle-related AEs; and (4) To evaluate the development of anti-mAb316P antibodies.

### STUDY DESIGN

The present study was a randomized, double-blind, double-dummy, active-controlled, parallel-group, multi-national, multi-center study in patients with primary hypercholesterolemia and moderate, high, or very high CV risk who are intolerant to statins. The study design is illustrated in Figure 7.

Statin intolerance was defined as the inability to tolerate at least 2 previous statins at the lowest approved daily dose due to skeletal muscle-related symptoms, other than those due to strain or trauma, such as pain, aches, weakness, or cramping, that began or increased during statin therapy and stopped when statin therapy was discontinued.

The study consisted of 5 periods: screening; washout; single-blind placebo run-in; double-blind treatment; and follow-up.

### (1) Screening:

Screening lasted approximately 1 week (week -7). Patients were requested to be on a stable diet equivalent to the National Cholesterol Education Program Adult Treatment Panel III (NCEP-ATP III) Therapeutic Lifestyle Changes (TLC) diet throughout the duration of the study, starting at screening through the end of treatment visit.

### (2) Washout:

Patients who met all screening inclusion and exclusion criteria entered a 2-week (week -6 to week -4) washout period of EZE, statins (for patients taking a non-approved dose or regimen), and red yeast rice.

### (3) Single-Bind Placebo Run-In:

Following the washout, patients entered a 4-week (week -4 to week 0), single-blind (only patients are blinded to treatment) placebo run-in period consisting of 4 weeks of treatment with placebo for mAb316P Q2W (a total of 2 doses) plus a placebo for EZE/atorvastatin capsule PO QD (28 doses).

Only patients who did not experience skeletal muscle-related AEs, other than those due to strain or trauma, during the single-blind placebo run-in period were eligible to enter the double-blind treatment period, described below.

Patients who experienced skeletal muscle-related AEs, other than those due to strain or trauma during the 4-week single-blind placebo run-in period were instructed to stop therapy and were withdrawn from the study.

At the first scheduled visit of the single-blind placebo run-in period (week -4), patients (or their caregivers) were instructed on the administration of study drug using a single-blind auto-injector containing placebo for mAb316P and self-administered the first dose (at week -4) at the clinic. The second dose of study drug during the single-blind placebo run-in period (at week -2) was administered by the patient or caregiver at home using the second single-blind auto-injector of placebo for mAb316P. Patients took a placebo for EZE/atorvastatin capsule QD for 4 weeks during the placebo run-in period.

### (4) Double-Blind Treatment:

Patients who meet all inclusion criteria, and who meet none of the exclusion criteria, including having experienced skeletal muscle-related AEs, other than those due to strain or trauma, during the 4-week single-blind placebo run-in period, were randomized to receive either: (A) mAb316P 75 mg SC Q2W + a placebo for EZE/atorvastatin PO QD; or (B) EZE 10 mg PO QD + placebo for mAb316P SC Q2W; or (C) Atorvastatin 20 mg PO QD + placebo for mAb316P SC Q2W.

Ezetimibe 10 mg, and atorvastatin 20 mg were be over-encapsulated in a capsule to match the placebo for EZE/atorvastatin to ensure the double-blind. Ezetimibe 10 mg, atorvastatin 20 mg, and placebo were indistinguishable from each other.

Randomization was stratified by a history of documented myocardial infarction (MI) or ischemic stroke, (Yes/No).

The first injection during the double-blind treatment period was administered at the site on the day of randomization (week 0 [day 1] -visit 4) and as soon as possible after the call to the interactive voice response system (IVRS)/interactive web response system (IWRS) for randomization into the study. Subsequent injections were administered by the patient (self-injection) or a designated caregiver (spouse, relative, etc.) at a patient-preferred location (eg, home or place of work). Patients were permitted to choose to return to the site Q2W to have the injection administered by study personnel. Patients randomized to mAb316P received 75 mg of study drug from randomization to week 12 (weeks 0, 2, 4, 6, 8, and 10).

At the week 12 visit, based on their LDL-C at week 8 and baseline CV risk (defined elsewhere herein), patients either continued receiving mAb316P 75 mg Q2W or had their dose up-titrated, as follows:
(A) Patients with very high CV risk, in a blinded manner, either: (i) continued receiving mAb316P 75 mg Q2W from week 12 onwards until the last injection at week 22, if their week 8 LDL-C was <70 mg/dL (1.81 mmol/L); or (ii) received a dose that is up-titrated to mAb316P 150 mg Q2W from week 12 onwards until the last injection at week 22, if their week 8 LDL-C was ≥70 mg/dL (1.81 mmol/L).
(B) Patients with high or moderate CV risk, in a blinded manner, either: (i) continued receiving mAb316P 75 mg Q2W from week 12 onwards until the last injection at week 22, if their week 8 LDL-C was <100 mg/dL (2.59 mmol/L), or (ii) received a dose that is up-titrated to mAb316P 150 mg Q2W from week 12 onwards until the last injection at week 22, if their week 8 LDL-C was ≥100 mg/dL (2.59 mmol/L).

### (5) Follow-Up:

Patients were followed for a period of 8 weeks after the end of the double-blind treatment period, or after premature discontinuation of study treatment.

### Throughout the Study:

Patients were instructed to continue taking their background lipid modifying therapy (LMT) throughout the study (with the exception of EZE, statins, red yeast rice, and fibrates [other than fenofibrate]).

Permitted LMTs, (if applicable), were stable (including dose) from screening through the end of treatment visit, barring exceptional circumstances whereby overriding concerns (including but not limited to, TG alert reported by the central lab) warranted such changes, as per the investigator's judgment.

The maximum study duration per patient was up to approximately 39 weeks (9 months): up to 1 week for screening; 2 weeks for the washout; 4 weeks for the single-blind placebo run-in; 24 weeks for double-blind treatment; and 8 weeks of follow-up. The end of the study was defined as the last patient's last on site visit, as scheduled per protocol.

### PATIENT SELECTION

A sufficient number of patients were screened, and enrolled in the single-blind placebo run-in period to ensure that at least 250 patients (100:100:50; mAb316P:EZE:atorvastatin) were eligible to be randomized in the double-blind treatment period.

Study Population: The study population consisted of patients with hypercholesterolemia (heFH or non-FH) and moderate, high, or very high CV risk who were intolerant to statins. Moderate CV risk was defined as a calculated 10-year fatal CVD risk SCORE ≥1 and <5% (ESC/EAS 2011). High CV risk was defined as a calculated 1 0-year fatal CVD risk SCORE ≥5% (ESC/EAS 2011), moderate chronic kidney disease (CKD), type 1 or type 2 diabetes mellitus without target organ damage, or heFH. Very high CV risk was defined as a history of documented CHD, ischemic stroke, peripheral arterial disease (PAD), transient ischemic attack (TIA), abdominal aortic aneurysm, carotid artery occlusion >50% without symptoms, carotid endarterectomy or carotid artery stent procedure, renal artery stenosis, renal artery stent procedure, type 1 or type 2 diabetes mellitus with target organ damage.

A documented history of CHD was defined as the occurrence of one or more of the following: (i) Acute MI; (ii) Silent MI; (iii) Unstable angina; (iv) Coronary revascularization procedure (e.g., percutaneous coronary intervention [PCI] or coronary artery bypass graft surgery [CABG]); and/or (v) Clinically significant CHD diagnosed by invasive or non-invasive testing (such as coronary angiography, stress test using treadmill, stress echocardiography or nuclear imaging).

Inclusion Criteria: In order to be eligible for the present study the patients must have primary hypercholesterolemia (heFH or non-FH) with moderate, high, or very high cardiovascular risk, and a history of statin intolerance.

Diagnosis of heFH was made either by genotyping or by clinical criteria. For patients who were not genotyped, the clinical diagnosis was required to be a certain/definite diagnosis and could be based on either the Simon Broome criteria or the WHO/Dutch Lipid Network criteria. Moderate, high, and very high CV risk were as defined above.

Definition of statin intolerance: Inability to tolerate at least 2 previous statins at the lowest approved daily dose due to skeletal muscle-related symptoms, other than those due to strain or trauma, such as pain, aches, weakness, or cramping, that began or increased during statin therapy and stopped when statin therapy was discontinued.

Exclusion Criteria: Prospective patients who met any of the following criteria (subcategorized under items A, B, and C, below) were excluded from the study:
A. Exclusion Criteria Related to Study Methodology:
   1. Calculated serum LDL-C <70 mg/dL (1.81 mmol/L) and very high CV risk (as defined in elsewhere herein) at the screening visit (week -7);
   2. Calculated serum LDL-C <100 mg/dL (2.59 mmol/L) and high or moderate CV risk (as defined in elsewhere herein) at the screening visit (week -7);
   3. A 10-year fatal CVD risk SCORE <1% at the screening visit (week -7);
   4. Use of a statin that is at or above the lowest approved daily dose within 4 weeks prior to the screening visit (week -7);
   5. Experienced skeletal muscle-related adverse events (AEs), other than those due to strain or trauma during the 4-week single-blind placebo run-in;
   6. Experiencing a skeletal muscle-related AE(s), other than those due to strain or trauma at the time of screening (week -7), start of single-blind placebo run-in period (week -4), or day 1/week 0;
   7. Not on a stable dose of lipid modifying therapy (LMT) for at least 4 weeks and/or fenofibrate for at least 6 weeks, as applicable, prior to the screening visit (week -7) or from screening to randomization, as applicable;
   8. Use of fibrates, other than fenofibrate, within 6 weeks of the screening visit (week -7);
   9. Use of nutraceuticals or over-the-counter therapies known to affect lipids, at a dose/amount that has not been stable for at least 4 weeks prior to the screening visit (week -7) or between the screening and randomization visits;
   10. Use of red yeast rice from the screening visit (week -7) to the end of study visit (week 32);
   11. Use of analgesics for which the dose is not planned to be stable from the screening visit to the end of study visit (week 32);
   12. Diagnosis of fibromyalgia;
   13. History of severe neuropathic pain;
   14. History of rheumatological disease associated with symptoms that may be confounded with symptoms of statin intolerance (e.g., rheumatoid arthritis);
   15. History of myalgia or myopathy that began or increased during treatment with LMT, other than statin therapy, and stopped when the LMT was discontinued;
   16. Known history of seizure disorder;
   17. History of previous transplant surgery;
   18. Use of medications that require intramuscular administration, or planned intramuscular injections during the study;
   19. Known history of myopathy, other than statin-associated myopathy;
   20. History of rhabdomyolysis (defined as evidence of organ damage with creatine kinase >10,000 IU/L);
   21. Presence of any clinically significant uncontrolled endocrine disease known to influence serum lipids or lipoproteins. [Note: Patients on thyroid replacement therapy were allowed to be included if the dosage of thyroxine was stable for at least 12 weeks prior to screening and the thyroid-stimuiating hormone (TSH) level was within the normal range of the central laboratory at the screening visit (week -7)];
   22. History of bariatric surgery within 12 months prior to the screening visit (week -7);
   23. Unstable weight (variation >5 kg) within 2 months prior to the screening visit (week -7);
   24. Known history of loss of function of PCSK9 (e.g., genetic mutation or sequence variation);
   25. Known history of homozygous FH;
   26. Newly diagnosed (within 3 months prior to randomization visit [week 0/day 1]) diabetes mellitus or poorly controlled (hemoglobin A1c [HbA1c] >8.5%) diabetes;
   27. Use of systemic corticosteroids, unless used as replacement therapy for pituitary/adrenal disease with a stable regimen for at least 6 weeks prior to randomization. Note: Topical, intra-articular, nasal, inhaled and ophthalmic steroid therapies were not considered as "systemic" and were allowed;
   28. Use of estrogen or testosterone therapy unless the regimen had been stable in the 6 weeks prior to the screening visit (week -7), and no plans to change the regimen during the study;
   29. History of undergoing plasmapheresis treatment within 2 months prior to the screening visit (week -7), or plans to undergo plasmapheresis during the study;
   30. Systolic blood pressure >160 mm Hg or diastolic blood pressure >100 mm Hg at the screening visit (week -7) or time of randomization (week 0/day 1);
   31. History of a myocardial infarction (MI), unstable angina leading to hospitalization, coronary artery bypass graft surgery (CABG), percutaneous coronary intervention (PCI), uncontrolled cardiac arrhythmia, carotid surgery or stenting, stroke, transient ischemic attack, carotid revascularization, endovascular procedure or surgical intervention for peripheral vascular disease within 3 months prior to the screening visit (week -7);
   32. Patients currently participating in a rehabilitation or exercise program
   33. History of New York Heart Association Class III or IV heart failure within the past 12 months;
   34. Age <18 years or legal age of majority at the screening visit (week -7), whichever is greater;
   35. Not previously instructed on a cholesterol-lowering diet prior to the screening visit (week -7);
   36. Known history of a hemorrhagic stroke;
   37. History of cancer within the past 5 years, except for adequately treated basal cell skin cancer, squamous cell skin cancer, or in situ cervical cancer;
   38. Known history of HIV positivity;
   39. Use of any active investigational drugs within 1 month or 5 half-lives, whichever is longer;
   40. Previous participation in any clinical trial of mAb316P (alirocumab) or any other anti-PCSK9 monoclonal antibody;
   41. Conditions/situations such as: (A) Any clinically significant abnormality identified at the time of screening that, in the judgment of the investigator or any sub-investigator, would preclude safe completion of the study or constrain endpoints assessment; e.g., major systemic diseases, patients with short life expectancy; (B) Considered by the investigator or any sub-investigator as inappropriate for this study for any reason, e.g.: (i) Deemed unable to meet specific protocol requirements, such as scheduled visits; (ii) Deemed unable to administer or tolerate long-term injections as per the patient or the investigator; (iii) Investigator or any sub-investigator, pharmacist, study coordinator, other study staff or relative thereof directly involved in the conduct of the protocol, etc.; (iv) Presence of any other conditions (eg, geographic or social), either actual or anticipated, that the investigator feels would restrict or limit the patient's participation for the duration of the study;
   42. Laboratory findings during screening period (not including randomization labs): (A) Positive test for hepatitis B surface antigen and/or hepatitis C antibody; (B) Positive serum beta-hCG or urine pregnancy test in women of childbearing potential; (C) TG >400 mg/dL (>3.95 mmol/L) (1 repeat lab is allowed); (D) eGFR <30 mL/min/1.73 m2 according to 4-variable MDRD Study equation (calculated by central lab); (E) Alanine aminotransferase (ALT) or aspartate aminotransferase (AST) >3 x upper limit of normal (ULN) (1 repeat lab is allowed); (F) CPK >2 x ULN; (G) TSH < lower limit of normal (LLN) or > ULN of the central laboratory; (H) Vitamin D3 <20 ng/mL [50 nmol/L];
B. Exclusion Criteria Related to the Active Comparator or Other Study Drugs:
   43. All contraindications to the other study drugs (atorvastatin and EZE) or warnings/precautions of use (when appropriate) as displayed in the respective National Product Labeling;
C. Exclusion Criteria Related to the Active Agent (mAb316P):
   44. Known hypersensitivity to monoclonal antibody therapeutics;
   45. Pregnant or breast-feeding women;
   46. Women of childbearing potential with no effective contraceptive method of birth control and/or who are unwilling or unable to be tested for pregnancy.

Patients prematurely discontinued from the study were not replaced.

### STUDY TREATMENTS

The study treatment was a single subcutaneous (SC) injection of 1 mL for a 75 or 150 mg dose of mAb316P or placebo provided in an auto-injector, administered in the abdomen, thigh, or outer area of the upper arm. During the double-blind treatment period (week 0 to 24), eligible patients were randomized to receive: (1) mAb316P 75 mg SC once every two weeks (Q2W) + placebo for Ezetimibe (EZE)/atorvastatin orally once daily (PO QD); or (2) EZE 10 mg PO QD + placebo for mAb316P SC Q2W; or (3) Atorvastatin 20 mg PO QD + placebo for mAb316P SC Q2W.

Ezetimibe 10 mg and atorvastatin 20 mg were over-encapsulated in a capsule to match the placebo for EZE/atorvastatin to ensure the double-blind. Ezetimibe 10 mg, atorvastatin 20 mg, and placebo were indistinguishable from each other. Study drug was administered by SC injection Q2W, starting at week 0 and continuing up to the last injection (week 22), 2 weeks before the end of the double-blind treatment period.

The first injection of double-blind study drug was administered at the clinical site, as soon as possible after the patient was randomized into the study. The patient was observed at the clinical site for 30 minutes following the first injection. The patient/caregiver administered subsequent injections outside of the clinic according to the dosing schedule. On days where the clinic study visit coincided with dosing, the dose of study drug was administered after all study assessments had been performed and all laboratory samples collected.

Study drug was ideally administered Q2W SC at approximately the same time of the day; however, it was deemed acceptable to have a window period of ± 3 days. The time of day was based on the patient's preference.

In the event an injection was delayed by more than 7 days or was completely missed, the patient was instructed to return to the original schedule of study drug dosing without administering additional injections. If the delay was less than or equal to 7 days from the missed date, the patient was instructed to administer the delayed injection and then resume the original dosing schedule. Patients were permitted to elect to have the study site staff administer the injections and return to the site Q2W for their injections. Placebo for EZE/atorvastatin, EZE 10 mg or atorvastatin 20 mg capsules were taken orally once daily. Detailed instructions for transport, storage, preparation, and administration of study drug will be provided by the site to the patient/caregiver.

### Dose Modification ("up-titration option")

At the week 12 visit, based on their LDL-C at week 8 and baseline CV risk (defined elsewhere herein), patients either continued receiving mAb316P 75 mg Q2W or had their dose up-titrated, as follows:
(A) Patients with very high CV risk, in a blinded manner, either: (1) continued receiving mAb316P 75 mg Q2W from week 12 onwards until the last injection at week 22 if their week 8 LDL-C was <70 mg/dL (1.81 mmol/L); or (2) received a dose that was up-titrated to mAb316P 150 mg Q2W from week 12 onwards until the last injection at week 22 if their week 8 LDL-C was ≥70 mg/dL (1.81 mmol/L).
(B) Patients with high or moderate CV risk, will, in a blinded manner, either: (1) continued receiving mAb316P 75 mg Q2W from week 12 onwards until the last injection at week 22, if their week 8 LDL-C was <100 mg/dL (2.59 mmol/L); or (2) received a dose that was up-titrated to mAb316P 150 mg Q2W from week 12 onwards until the last injection at week 22 if their week 8 LDL-C was ≥100 mg/dL (2.59 mmol/L).

### Injection Training

During the first scheduled visit of the single-blind placebo run-in period (at week -4), patients were instructed on the administration of study drug using a single-blind auto-injector containing placebo for mAb316P and self-administered the first dose at the clinic. The second dose of study drug during the single-blind placebo run-in period (at week -2) was administered by the patient or caregiver at home using the second single-blind auto-injector of placebo for mAb316P.

The first injection during the double-blind treatment period was administered at the site on the day of randomization (week 0 [day 1] - visit 4) and as soon as possible after randomization into the study, using an auto-injector from the kit to which patients were randomized. Subsequent injections were administered by the patient (self-injection) or by a designated caregiver, or patients had the option to return to the site Q2W to have the injection administered by study personnel. All patients and caregivers who planned to inject the study drug were trained by the study staff before administering injections.

### Investigational Treatment

Sterile mAb316P drug product was supplied at a concentration of 75 mg/mL or 150 mg/mL in histidine, pH 6.0, polysorbate 20, and sucrose in an auto-injector. Placebo matching mAb316P was supplied in the same formulation as mAb316P, without the addition of protein, in an auto-injector. Ezetimibe 10 mg and atorvastatin 20 mg were over-encapsulated in a capsule to match the placebo for EZE/atorvastatin to ensure the double-blind. Ezetimibe 10 mg, atorvastatin 20 mg, and placebo were indistinguishable from each other.

### Background Treatment

Patients were required to be on stable LMT for at least 4 weeks (6 weeks for fenofibrate) before the screening visit (week -7). Patients were instructed to continue taking their background LMT throughout the study (other than EZE, statins, red yeast rice, and fibrates [other than fenofibrate]). Lipid profile values from samples obtained after randomization were blinded. No change in a patient's background LMT was made from the screening visit (week -7) until the end of study visit (week 32). No dose adjustment, discontinuation or initiation of other LMT (including prohibited LMT) occurred during this time, barring exceptional circumstances whereby overriding concerns warranted such changes, per the investigator's judgment.

In summary, background LMT was not modified from screening to the follow-up visit. Other background treatments permitted in the study included: Bile acid-binding sequestrants (such as cholestyramine, colestipol, colesevelam); Nicotinic acid; Fenofibrate; and Omega-3 fatty acids (≥1000 mg daily).

### Randomization

Patients were randomized to receive mAb316P, EZE, or atorvastatin during the double-blind study treatment period using a ratio 2:2:1, with permuted-block randomization. Randomization was stratified according to a history of documented MI or ischemic stroke [Yes/No].

### Blinding

Single-Blind Placebo Run-In. For the single-blind placebo run-in and in accordance with the single-blind design, only study patients remained blinded to treatment; investigators were not blinded to study treatment. Placebo for mAb316P was supplied in auto-injectors. Oral placebo for EZE/atorvastatin was supplied in a capsule to preserve the blind.

Double-Blind Treatment Period. For the double-blind treatment period, mAb316P and placebo for mAb316P were provided in identically matched auto-injectors, and packaged and labeled identically to preserve the blind. Ezetimibe and atorvastatin were over-encapsulated in a capsule to match the placebo for EZE/atorvastatin to ensure the double-blind. Ezetimibe 10 mg, atorvastatin 20 mg, and placebo were indistinguishable from each other.

Each double-blind treatment kit was labeled with a number, which was generated by a computer program. The treatment kit numbers were obtained by the investigator at the time of patient randomization and subsequent patient visits scheduled via a centralized treatment allocation system that was available 24 hours-a-day, 7 days-a-week.

In accordance with the double-blind design, study patients, investigators and study site personnel remained blinded to study treatment and did not have access to the randomization (treatment codes) except under specifically defined circumstances.

Lipid parameter values from blood samples obtained after the randomization visit, analyzed by the central lab, were not communicated to the sites so that they were not able to deduce the treatment group of their patients based on LDL-C level attained. The sponsor's operational team did not have access to lipid parameters after randomization and until after the final database lock has occurred. Sites and the sponsor operational team were blinded to dose up-titration from 75 mg to 150 mg, in the event a patient met the criterion for up-titration. Blinded study drug kits coded with a medication numbering system were used. In order to maintain the blind, lists linking these codes with product lot numbers were not accessible to individuals involved in study conduct.

Anti-drug antibody (ADA) results were not communicated to the sites and the sponsor operational team did not have access to results associated with patient identification until after the final database lock. Patients who had titers at or above 240 for anti- mAb316P antibodies at the follow-up visit had additional antibody sample(s) obtained 6 to 12 months after the last dose, and thereafter, about every 3 to 6 months, until titer returned below 240. In order to maintain the blind of the study, the requests for sample collection of post-study anti- mAb316P antibodies were made on patients with titers below 240 at the follow-up visit.

### Concomitant Medications

If considered necessary for the patient's welfare and unlikely to interfere with study drug, concomitant medications (other than those that are prohibited during the study) were permitted to be given at the discretion of the investigator, at a stable dose (when possible). Any other concomitant medication(s) were allowed and were recorded as appropriate.

Nutraceutical products or over-the-counter therapies that may affect lipids were allowed only if they had been used at a stable dose for at least 4 weeks prior to the screening visit (week-7) and maintained from the screening visit until the end of the study (week 32). Examples of such nutraceutical products or over-the-counter therapies include: omega-3 fatty acids at doses <1000 mg, plant stanols such as found in Benecol, flax seed oil, and psyllium.

Prohibited concomitant medications from the initial screening visit until the follow-up visit included the following: statins; fibrates, other than fenofibrate; EZE; and red yeast rice products.

### STUDY ENDPOINTS

Baseline characteristics included standard demography (e.g., age, race, weight, height, etc.), disease characteristics including medical history, and medication history for each patient. Historical information regarding statins, doses, and actual skeletal muscle related events that led to the diagnosis of "statin intolerance" were collected as part of medical/surgical history.

Primary Efficacy Endpoint: The primary efficacy endpoint was the percent change in calculated LDL-C from baseline to week 24, which was defined as: 100x (calculated LDL-C value at week 24 - calculated LDL-C value at baseline)/calculated LDL-C value at baseline. A baseline LDL-C value was needed for each patient as the baseline calculated LDL-C value was the last LDL-C level obtained before the first double-blind study drug injection.

The calculated LDL-C at week 24 was the LDL-C level obtained within the week 24 analysis window and during the main efficacy period. The main efficacy period was defined as the time from the first double-blind study drug injection up to 21 days after the last double-blind study drug injection or up to the upper limit of the week 24 analysis window, whichever occurred first.

All calculated LDL-C values (scheduled or unscheduled, fasting or not fasting) were used to provide a value for the primary efficacy endpoint if appropriate according to above definition. The analysis window used to allocate a time point to a measurement was defined in a statistical analysis plan (SAP).

Secondary Efficacy Endpoints: Secondary endpoints of the present study included the following:
(1) The percent change in calculated LDL-C from baseline to week 12: the calculated LDL-C at week 12 was the LDL-C level obtained within the week 12 analysis window and during the 12-week efficacy period. The 12-week efficacy period is defined as the time from the first injection of double-blind study drug up to the visit 6 contact or up to 21 days after the last double-blind injection of study drug, whichever occurred first.
(2) The percent change in ApoB from baseline to week 24.
(3) The percent change in non-HDL-C from baseline to week 24.
(4) The percent change in total-C from baseline to week 24.
(5) The percent change in ApoB from baseline to week 12.
(6) The percent change in non-HDL-C from baseline to week 12.
(7) The percent change in total-C from baseline to week 12.
(8) The proportion of patients reaching LDL-C goal at week 24; e.g., LDL-C <70 mg/dL (1.81 mmol/L) in case of very high CV risk, or LDL-C <100 mg/dL (2.59 mmol/L) for patients with moderate or high CV risk, defined as: (number of patients whose calculated LDL-C value at week 24 reaches LDL-C goal/number of patients in the modified intent-to-treat [mITT] population)*100, using definition and rules used for the primary endpoint.
(9) The proportion of patients reaching LDL-C <70 mg/dL (1.81 mmol/L) at week 24.
(10) The percent change in Lp(a) from baseline to week 24.
(11) The percent change in HDL-C from baseline to week 24.
(12) The percent change in HDL-C from baseline to week 12.
(13) The percent change in Lp(a) from baseline to week 12.
(14) The percent change in fasting TG from baseline to week 24.
(15) The percent change in fasting TG from baseline to week 12.
(16) The percent change in ApoA-1 from baseline to week 24.
(17) The percent change in ApoA-1 from baseline to week 12.
(18) The proportion of patients reaching LDL-C goal at week 12; e.g., LDL-C <70 mg/dL (1.81 mmol/L) in case of very high CV risk, and LDL-C <100 mg/dL (2.59 mmol/L) in case of moderate or high CV risk, defined as: (number of patients whose calculated LDL-C value at week 12 reaches LDL-C goal/number of patients in the mITT population)*100.
(19) The proportion of patients reaching LDL-C <100 mg/dL (2.59 mmol/L) at week 24.
(20) The proportion of patients reaching LDL-C <100 mg/dL (2.59 mmol/L) at week 12.
(21) The proportion of patients reaching LDL-C <70 mg/dL (1.81 mmol/L) at week 12.
(22) The absolute change in calculated LDL-C (mg/dL and mmol/L) from baseline to weeks 12 and 24.
(23) The change in ratio ApoB/ApoA-1 from baseline to weeks 12 and 24.
(24) The proportion of patients with ApoB <80 mg/dL (0.8mmol/L) at weeks 12 and 24.
(25) The proportion of patients with non-HDL-C <100 mg/dL at weeks 12 and 24.
(26) The proportion of patients with calculated LDL-C <70 mg/dL (1.81 mmol/L) and/or ≥50% reduction in calculated LDL-C (if calculated LDL-C ≥70 mg/dL [1.81 mmol/L]) at weeks 12 and 24.

Other Endpoints: (1) Anti-mAb316P anti-drug-antibody status (positive/negative) and titers assessed throughout the study; (2) The percent change in high-sensitivity C-reactive protein (hs-CRP) from baseline to week 24; (3) The absolute change in HbA1c (%) from baseline to week 24.

### STUDY PROCEDURES

All laboratory samples were collected before the dose of study drug was administered. Blood samples for lipid panels were collected in the morning, in fasting condition (i.e., overnight, at least a 10-hour fast and refrain from smoking) for all clinic visits. Alcohol consumption within 48 hours and intense physical exercise within 24 hours preceding blood sampling were discouraged. Note: if the patient was not in fasting conditions, the blood sample was not collected and a new appointment was scheduled the day after (or as close as possible to this date) with a reminder that the blood sample must be drawn under fasting (at least 10 hours) conditions.

Total-C, HDL-C, TG, ApoB, ApoA-1, and Lp(a) were directly measured by a central laboratory as per a predetermined schedule. Low-density lipoprotein cholesterol was calculated using the Friedewald formula at all visits (except week -15 and the follow-up visit). If TG values exceeded 400 mg/dL (4.52 mmol/L), then the central lab measured (via the beta quantification method) the LDL-C rather than calculate it. Non-HDL-C was calculated by subtracting HDL-C from the total-C. Ratio ApoB/ApoA-1 was calculated.

Lipid Panel (Fasting): Blood samples for the lipid panel (total-C, TG, HDL-C, and calculated LDL-C) were collected after at least a 1 0-hour fast at pre-specified time points.

Specialty Lipid Panel (Fasting): Blood samples for the specialty lipid panel (ApoB, ApoA-1, ApoB/ApoA-1 ratio, and Lp[a]) were collected after at least a 10-hour fast at pre-specified time points.

Blood Pressure and Heart Rate: Blood pressure and heart rate were assessed at pre-specified time points. Blood pressure was preferably measured in sitting position under standardized conditions, approximately at the same time of the day, on the same arm, with the same apparatus (after the patient has rested comfortably in sitting position for at least 5 minutes). At the first screening visit, blood pressure was measured in both arms. The arm with the highest diastolic pressure was determined at this visit, and blood pressure was measured on this arm throughout the study. This highest value was recorded in the electronic case report form (eCRF). Heart rate was measured at the time of the measurement of blood pressure.

Physical Examination: A thorough and complete physical examination, including height and weight, was performed at the screening visit (visit 1). Physical exam with body weight was performed at pre-specified time points.

Body Weight and Height: Body weight were obtained with the patient wearing undergarments or very light clothing and no shoes, and with an empty bladder. The same scale was preferably used throughout the study. The use of calibrated balance scales was recommended, if possible.

Electrocardiogram: Electrocardiograms were performed before blood is drawn during visits that required blood draws. A standard 12-lead ECG was performed at pre-specified time points. The 12-lead ECGs were performed after at least 10 minutes rest and in the supine position. The electrodes were positioned at the same place, as much as possible, for each ECG recording throughout the study. The ECG were interpreted locally by the investigator. Each trace was analyzed in comparison with the screening recorded trace.

Laboratory Testing: All laboratory samples were collected before the dose of study drug was administered. Samples for laboratory testing were collected at pre-specified time points and analyzed by a central laboratory during the study.

### RESULTS

### Subject Disposition

A total of 519 patients were screened for this study, of which 361 (69.6%) patients completed screening and entered the single-blind placebo run-in period. For those patients who entered the single-blind placebo run-in period, 47 (13%) patients prematurely discontinued placebo treatment, among which 29 (8%) patients discontinued due to skeletal muscle related adverse events (*i.e.,* met specified exclusion criteria). Therefore, 314 patients (87%) completed the run-in period and were eligible to be randomized into the double-blind period.

A total of 314 patients were randomized (63 to the atorvastatin group, 125 to the ezetimibe group, and 126 to the mAb316P group), with a single patient in the ezetimibe group randomized but not receiving study treatment due to a reason of "other" (concerns for scheduling the required protocol visits). Therefore, the safety population contained 313 patients. This patient did not return for any assessments afterwards, and hence is not included in the ITT population. Further, 3 more patients were excluded from the ITT population, 1 (in the ezetimibe group) due to a lack of baseline LDL-C value and 2 others (1 in the atorvastatin group and 1 in the ezetimibe group) due to a lack of post-baseline assessments. Finally, 9 additional patients (2 in the atorvastatin group, 4 in the ezetimibe group, and 3 in the mAb316P group) were excluded from the miTT populations due to a lack of on-treatment post-baseline assessments.

As of the first-step data cut-off, the patients' status during the study was as follows:
220 (70.1%) patients completed the 24-week double-blind treatment period: 42 (66.7%) in the atorvastatin group, 82 (65.6%) in the ezetimibe group, and 96 (76.2%) in the mAb316P group.

93 (29.6%) patients prematurely discontinued study treatment before completing the double-blind treatment period: 21 (33.3%) in the atorvastatin group, 42 (33.6%) in the ezetimibe group, and 30 (23.8%) in the mAb316P group. 70 (22.3%) patients prematurely terminated study treatment due to adverse events: 16 (25.4%) in the atorvastatin group, 31 (24.8%) in the ezetimibe group, and 23 (18.3%) in the mAb316P group. 2 (3.2%) patients prematurely terminated study treatment due to poor protocol compliance, and both patients were in the atorvastatin group. 21 (6.7%) patients prematurely terminated study treatments due to various other reasons: 3 (4.8%) in the atorvastatin group, 11 (8.8%) in the ezetimibe group, and 7 (5.6%) in the mAb316P group.

281 (89.5%) patients were administered at least one open-label mAb316P treatment, and therefore, are included in the OLE population.

9 (3.2%) patients terminated study treatment in the OLE period, as of the data cut-off for this KRM, and 8 (2.8%) of those patients terminated due to an adverse event.

The remaining 272 (96.8%) patients in the OLE population are ongoing and receiving study treatment in the OLE period.

Baseline characteristics of the patients enrolled in the study are summarized in Table 10.

**Table 10. Baseline Characteristics**

| | **Atorvastatin** | **Ezetimibe** | **mAb316P** |
|---|---|---|---|
| | **(N = 63)** | **(N = 125)** | **(N = 126)** |
| **Age, years, mean (*SD*)** | 63.4 (8.9) | 62.8 (10.1) | 64.1 (9.0) |
| **Male, % (n)** | 55.6 (35) | 53.6 (67) | 55.6 (70) |
| **Race, white, % (n)** | 98.4 (62) | 92.8 (116) | 92.9 (117) |
| **BMI, kg/m², mean (*SD*)** | 29.7 (5.4) | 28.4 (4.9) | 29.6 (6.6) |
| **HeFH, % (n)** | 12.7 (8) | 20.0 (25) | 11.1 (14) |
| **Hypertension** | 35 (55.6%) | 77 (61.6%) | 85 (67.5%) |
| **Type 2 Diabetes** | 15 (23.8%) | 24 (19.2%) | 36 (28.6%) |
| **CHD history, % (n)** | | | |
| **Current smoker, % (n)** | | | |
| **LLT other than statin/ezetimibe** | 54.0 (34) | 44.0 (55) | 37.3 (47) |

Baseline lipid parameters are summarized in Table 11.

**Table 11. Baseline Lipids**

| **Mean (SD), mg/dL** | **Atorvastatin** | **Ezetimibe** | **mAb316P** |
|---|---|---|---|
| | **(N = 63)** | **(N = 125)** | **(N = 126)** |
| **LDL-C (calculated)** | 187.3 (59.5) | 193.5 (70.9) | 191.1 (72.7) |
| **Non-HDL-C** | 223.8 (64.8) | 229.8 (82.7) | 230.0 (80.4) |
| **Apo B** | 139.1 (34.7) | 138.2 (37.4) | 141.7 (39.5) |
| **Lp(a), median (IQR)** | 12.0 (6.0 : 50.0) | 14.0 (7.0 : 43.0) | 18.0 (8.0 : 47.0) |

### Efficacy Results

The placebo run-in was completed by 87.0% (314/361) patients. In general, demographic characteristics, baseline disease characteristics, statin intolerance questionnaire, baseline efficacy lipid parameters, LMT history and background LMT use were comparable among patients randomized to each of the three study treatment groups. Fifteen percent of patients had heterozygous FH. The mean baseline LDL-C was 187.3 mg/dL in the atorvastatin group, 194.2 mg/dL in the ezetimibe group, and 191.1 mg/dL in the mAb316P group. In total, 89.5% of randomized patients entered the open-label extension.

The double-blind period efficacy endpoint analysis results in the order of statistical hierarchical testing as specified in the above protocol (with multiple testing controlled at the 0.05 significance level) is set forth in Table 12. Primary and key secondary efficacy analyses were performed comparing mAb316P-treated patients to ezetimibe-treated patients. For clarification, the ITT analysis is defined for patients in the ITT population and includes all endpoint assessments in an analysis window, regardless of study treatment dosing status (i.e. includes post-treatment assessments). The on-treatment analysis is defined for patients in the mITT population and includes all endpoint assessments from the first double-blind study drug (capsule or injection, whichever comes first) up to 21 days after the last double-blind study drug injection, or 3 days after the last capsule intake, whichever came first (i.e. includes assessments in the efficacy treatment period). Note: A result is statistically significant if the p-value is ≤ 0.05, in the order of the hierarchical testing.

**Table 12. Summary of Efficacy Analysis Results (percent change from baseline)**

| **Endpoint/Analysis** | **Ezetimibe Result** | **mAb316P Result** | **Comparison** | **P-value** |
|---|---|---|---|---|
| LDL-C at WK24 - ITT analysis | LS mean: -14.6% | LS mean: -45.0 % | Diff: -30.4% | <0.0001 |
| LDL-C at WK24 - on-treatment analysis | LS mean: -17.1% | LS mean: -52.2% | Diff: -35.1 % | < 0.0001 |
| LDL-C at WK12 - ITT analysis | LS mean: -15.6% | LS mean: -47.0% | Diff: -31.5% | < 0.0001 |
| LDL-C at WK1 2 - on-treatment analysis | LS mean: -18.0% | LS mean:-51.2% | Diff: -33.1 % | < 0.0001 |
| Apo B at WK24 - ITT analysis | LS mean: -11.2% | LS mean:-36.3% | Diff: -25.1% | < 0.0001 |
| Apo B at WK24 - on-treatment analysis | LS mean: -14.4% | LS mean:-42.6% | Diff: -28.2% | < 0.0001 |
| Non-HDL-C at WK24 - ITT analysis | LS mean: -14.6% | LS mean:-40.2% | Diff: -25.6% | < 0.0001 |
| Non-HDL-C at WK24 - on-treatment analysis | LS mean: -17.1% | LS mean:-46.9% | Diff: -29.8% | < 0.0001 |
| Total Cholesterol at WK24 - ITT analysis | LS mean: -10.9% | LS mean:-31.8% | Diff: -20.8% | < 0.0001 |
| Apo B at WK12 - ITT analysis | LS mean: -11.6% | LS mean:-36.1% | Diff: -24.5% | < 0.0001 |
| Non-HDL-C at WK12 - ITT analysis | LS mean: -15.8% | LS mean:-25.7% | Diff: -25.7% | < 0.0001 |
| Total Cholesterol at WK12 - ITT analysis | LS mean: -11.6% | LS mean:-32.7% | Diff: -21.1% | < 0.0001 |
| Very High CV LDL-C < 70mg/dL ***OR*** Moderate/High CV LDL-C < 100mg/dL at WK24 - ITT analysis | Proportion=4.1 % | Proportion=41.3% | Odds Ratio=20.2 | < 0.0001 |
| Very High CV LDL-C < 70mg/dL ***OR*** Moderate/High CV LDL-C < 100mg/dL at WK24 - on-treatment analysis | Proportion=4.2% | Proportion=50.4% | Odds Ratio=33.9 | < 0.0001 |
| LDL-C < 70mg/dL at WK24 - ITT analysis | Proportion=0.8% | Proportion=32.5% | Odds Ratio=71.5 | < 0.0001 |
| LDL-C < 70mg/dL at WK24 - on-treatment analysis | Proportion=0.8% | Proportion=39.0% | Odds Ratio=109.8 | < 0.0001 |
| Lp(a) at WK24 - ITT analysis | LS mean: -7.3% | LS mean:-25.9% | Diff: -18.7% | < 0.0001 |
| HDL-C at WK24 - ITT analysis | LS mean: 6.8% | LS mean:7.7% | Diff: 0.9% | 0.6997 |
| Fasting Triglycerides at WK24 - ITT analysis | LS mean: -3.5% | LS mean:-9.2% | Diff: -5.6% | 0.1678 |
| Apo A-1 at WK24 - ITT analysis | LS mean: 2.9% | LS mean:4.8% | Diff: 1.9% | 0.2768 |
| Lp(a) at WK12 - ITT analysis | LS mean: -4.5% | LS mean:-21.7% | Diff: -17.2% | < 0.0001 |
| HDL-C at WK12 - ITT analysis | LS mean: 7.6% | LS mean:9.0% | Diff: 1.4% | 0.4148 |
| Fasting Triglycerides at WK12 - ITT analysis | LS mean: -9.4% | LS mean:-8.1 % | Diff: 1.3% | 0.7152 |
| Apo A-1 at WK12 - ITT analysis | LS mean: 3.9% | LS mean: 5.5% | Diff: 1.6% | 0.2685 |

For patients treated with mAb316P, the LS mean LDL-C value at week 24 was 108.5 mg/dL which represents a change in LDL-C level from baseline of -84 mg/dL (*i.e*., -45.0%). By contrast, for patients treated with EZE, the LS mean LDL-C value at week 24 was 159.9 mg/dL which represents a change in LDL-C level from baseline of -33 mg/dL (*i*.*e*., -14.6%). The LS mean % difference in LDL-C at week 24 for mAb316P-treated patients vs. ezetimibe-treated patients was -30.4% (SE = 3.1, p<0.0001).

Fifty-two mAb316P patients (41.9%) reached LDL-C goal at week 24, whereas only 5 EZE patients (4.4%) reached LDL-C goal at week 24 (p value <0.0001). For purposes of this analysis, LDL-C goal was defined as less than 70 mg/dL for very high risk patients, and less than 100 mg/dL for moderate and high risk patients. In addition, 54/109 mAb316P patients (49.5%) were subjected to an uptitration from 75 mg Q2W to 150 mg Q2W at week 12 (based on week 8 LDL-C level).

A summary of reductions in selected secondary lipid parameters (non-HDL-C, Apo B and Lp(a)) at week 24 is shown in Table 13.

**Table 13. Reductions in Secondary Lipid Parameters at Week 24**

| **LS mean Percent Change From Baseline to Week 24 and LS Mean Difference vs. Ezetimibe** | | | | | |
|---|---|---|---|---|---|
| **Non-HDL-C** | | **ApoB** | | **Lp(a)** | |
| **mAb316P** | **EZE** | **mAb316P** | **EZE** | **mAb316P** | **EZE** |
| -40.2* (1.7) | -14.6* (1.7) | -36.3* (1.7) | -11.2* (1.7) | -25.9* (2.4) | -7.3* (2.5) |
| -25.6^{#} (2.4) | | -25.1^{#} (2.4) | | -18.7^{#} (3.5) | |
| p < 0.0001 | | p < 0.0001 | | p < 0.0001 | |

| | | | | | |
|---|---|---|---|---|---|
| ^{*} LS mean (SE) percent change from baseline ^{#} LS mean difference (SE) vs. ezetimibe | | | | | |

The primary efficacy endpoint and more than two-thirds of the key secondary efficacy endpoints achieved statistically significant benefit in favor of the mAb316P-treated patients according to the hierarchical testing procedure.

### Safety Results

A total of 313 patients were randomized and received at least a partial dose of double-blind study treatment (Safety Population), and 281 patients received OLE study treatment (OLE Population). Treatment-emergent SAEs occurred in a total of 29 patients, specifically, 7 (11.1%) patients in the atorvastatin treatment group, 10 (8.1%) patients in the ezetimibe treatment group, and 12 (9.5%) patients in the mAb316P treatment group. There were no more than 1 report in any event preferred term for each of the three treatment groups, with the single exception of 4 (3.2%) patients reporting non-cardiac chest pain in the ezetimibe treatment group.

A total of 70 (22.4%) patients prematurely discontinued study treatment due to a TEAE. Specifically, 16 (25.4%) patients in the atorvastatin treatment group, 31 (25.0%) patients in the ezetimibe treatment group, and 23 (18.3%) patients in the mAb316P treatment group terminated study treatment early. The most prevalent events causing early termination were contained in the musculoskeletal and connective tissues disorders SOC (14 [22.2%] patients in the atorvastatin group, 26 [21.0%] patients in the ezetimibe group, and 20 [15.9%] patients in the mAb316P group), with the most frequently reported preferred term of myalgia.

No patient deaths were reported at the time of the interim analysis.

TEAEs occurred in 54 (85.7%) patients in the atorvastatin treatment group, 100 (80.6%) patients in the ezetimibe treatment group, and 104 (82.5%) patients in the mAb316P treatment group. The TEAEs that occurred in ≥5% of patients in any treatment group are: - nasopharyngitis (3.2% / 8.1% / 6.3% for atorvastatin /ezetimibe / mAb316P respectively); - upper respiratory tract infection (3.2% / 4.0% / 5.6% for atorvastatin / ezetimibe / mAb316P respectively); - headache (6.3% / 4.8% / 4.8% for atorvastatin / ezetimibe / mAb316P respectively); - paraesthesia (6.3% / 0 / 3.2% for atorvastatin / ezetimibe / mAb316P respectively); - arthralgia (7.9% / 7.3% / 5.6% for atorvastatin / ezetimibe / mAb316P respectively); - back pain (7.9% / 5.6% / 4.0% for atorvastatin / ezetimibe / mAb316Prespectively); - muscle spasms (11.1% / 7.3% / 4.0% for atorvastatin / ezetimibe / mAb316P respectively); - muscular weakness (6.3% / 1.6% / 0.8% for atorvastatin / ezetimibe / mAb316P respectively); - myalgia (27% / 23.4% / 24.6% for atorvastatin / ezetimibe / mAb316P respectively); - and fatigue (7.9% / 3.2% / 4.8% for atorvastatin / ezetimibe / mAb316P respectively).

The SOCs with a patient frequency ≥ 5% in the mAb316P group and a higher frequency in the mAb316P group as compared to both the atorvastatin and EZE treatment groups were: "Psychiatric disorders" occurred in 9 (7.1%) patients in the mAb316P treatment group, vs. 2 (3.2%) in the atorvastatin treatment group and 5 (4.0%) patients in the EZE treatment group. The most common event was 5 (4.0%) reports of insomnia in the mAb316P treatment group vs. 1 (1.6%) such events in the atorvastatin treatment group vs. 2 (1.6%) such reports in the ezetimibe treatment group. "Ear and labyrinth disorders" occurred in 8 (6.3%) patients in the mAb316P treatment group, vs. 1 (1.6%) patient in the atorvastatin treatment group and 4 (3.2%) patients in the EZE treatment group. The most common event was 6 (4.8%) reports of vertigo in the mAb316P treatment group vs. 1 (1.6%) such report in the atorvastatin treatment group vs. 2 (1.6%) such report in the ezetimibe treatment group. "Cardiac disorders" occurred in 10 (7.9%) patients in the mAb316P treatment group, vs. 2 (3.2%) patients in the atorvastatin treatment group and 6 (4.8%) patients in the EZE treatment group. The most common event was 4 (3.2%) reports of palpitations in the mAb316P treatment group vs. 0 such reports in the atorvastatin treatment group vs. 2 (1.6%) such reports in the ezetimibe treatment group. "Investigations" occurred in 9 (7.1%) patients in the mAb316P treatment group, vs. 3 (4.8%) patients in the atorvastatin treatment group and 7 (5.6%) patient in the EZE treatment group. The single SOC with a higher frequency in both the atorvastatin and EZE treatment groups compared to the mAb316P treatment group was musculoskeletal and connective tissue disorders.

For TEAEs of special interest (AESIs), results are presented by pre-defined SMQ or CMQ preferred term groupings: Treatment-emergent injection site reactions (ISRs) occurred in 1 (1.6%) patient in the atorvastatin treatment group, 6 (4.8%) patients in the ezetimibe treatment group, and 6 (4.8%) patients in the mAb316P treatment group. General allergic TEAEs, identified through the MedDRA SMQ of "Hypersensitivity" occurred in 4 (6.3%) patients in the atorvastatin treatment group, 9 (7.3%) patients in the ezetimibe treatment group, and 12 (9.5%) patients in the mAb316P treatment group. Treatment-emergent neurologic AEs occurred in 8 (12.7%) patients in the atorvastatin treatment group, 4 (3.2%) patients in the ezetimibe treatment group, and 11 (8.7%) patients in the mAb316P treatment group. The most common preferred terms were paresthesia (6.3% / 0 / 3.2% for atorvastatin / ezetimibe / mAb316P respectively), and muscular weakness (6.3% / 1.6% / 0.8% for atorvastatin / ezetimibe / mAb316P respectively). Treatment-emergent neurocognitive disorders occurred in zero patients in the atorvastatin treatment group, 2 (1.6%) patients in the ezetimibe treatment group, and 3 (2.4%) patients in the mAb316P treatment group.

For patients with cardiovascular events identified for adjudication, one (0.8%) patient was positively adjudicated for non-fatal MI, and this patient was in the mAb316P treatment group.

With respect to the frequency of patients with 2 consecutive calculated LDL-C measurements below 25 mg/dL, no patients had an occurrence in any of the three treatment groups.

Skeletal muscle-related TEAEs are defined twice for this study, specifically for those events collected on the skeletal muscle-related CRF and again by CMQ (as defined in the protocol appendix). A total of 99 (31.6%) patients reported a CMQ preferred term, specifically 25 (39.7%) patients in the atorvastatin treatment group, 40 (32.3%) patients in the ezetimibe treatment group, and 34 (27.0%) patients in the mAb316P treatment group. The most prevalent CMQ defined preferred terms causing skeletal muscle-related events were myalgia (27% / 23.4% / 24.6% for atorvastatin / ezetimibe / mAb316P respectively), muscle spasms (11.1% / 7.3% / 4.0% for atorvastatin / ezetimibe / mAb316P respectively), and muscular weakness (6.3% / 1.6% / 0.8% for atorvastatin / ezetimibe / mAb316P respectively), all of which are contained in the musculoskeletal and connective tissues disorders SOC.

Treatment-emergent skeletal muscle-related events resulting in early treatment discontinuation was reported in 55 (17.6%) patients, specifically 13 (20.6%) patients in the atorvastatin treatment group, 23 (18.5%) patients in the ezetimibe treatment group, and 19 (15.1%) patients in the mAb316P treatment group. No treatment-emergent serious skeletal muscle-related events were reported in any treatment group. No patient deaths were reported due to TEAE skeletal muscle-related events in any treatment group.

### Conclusions

The present study evaluated patients with a history of intolerance to at least two different statins, including one at the lowest dose, due to muscle-related symptoms. Patients were randomized to receive mAb316P, ezetimibe or atorvastatin 20 mg (a calibrator arm). Patients treated in this study had very high LDL-C levels when they first entered the trial (between 187-193.5 mg/dL on average). In clinical practice, 10-25 percent of patients report intolerance to statins.

The present study achieved the primary efficacy endpoint in the ITT population of a statistically significant reduction in percent change from baseline calculated LDL-C in the mAb316P-treated patients (LS mean = -45.0%), as compared to ezetimibe-treated patients (LS mean = - 14.6%), with an LS mean difference between treatment groups of -30.4%. For more than two-thirds of the key secondary efficacy endpoints, this study achieved statistically significant benefit in the mAb316P-treated patients, as compared to ezetimibe-treated patients. Based on the available data from this study, subcutaneous administration of mAb316P in patients with primary hypercholesterolemia (heFH and non FH) who are intolerant to statins was generally safe and well tolerated. The rate of skeletal muscle-related AEs for the mAb316P-treated patients was less in either control group, and this difference was determined to be statistically significant in patients treated with 20 mg of atorvastatin (as assessed by the time to first skeletal muscle AE, p=0.042). Further, the study withdrawal rate of skeletal muscle-related AEs for the mAb316P-treated patients was less than the two control groups. A similar rate of AEs between all treatment groups (mAb31682.5 percent, ezetimibe 81 percent, atorvastatin 86 percent) were observed in the present study. The most common AEs were myalgia (25 percent mAb316, 23 percent ezetimibe, 27 percent atorvastatin), nasopharyngitis (6 percent mAb316, 8 percent ezetimibe, 3 percent atorvastatin), arthralgia (6 percent mAb316, 7 percent ezetimibe, 8 percent atorvastatin), and upper respiratory tract infection (6 percent mAb316, 4 percent ezetimibe, 3 percent atorvastatin).

The present invention is not to be limited in scope by the specific embodiments described herein. Indeed, various modifications of the invention in addition to those described herein will become apparent to those skilled in the art from the foregoing description and the accompanying figures.

## Claims

1. A pharmaceutical composition comprising 75 mg or 150 mg of an antibody or antigen-binding fragment thereof which specifically binds human PCSK9 for use in the treatment of hypercholesterolemia in a patient who is intolerant to statins or who has a history of adverse reactions to statin therapy, wherein the antibody or antigen binding fragment thereof comprises the heavy and light chain CDRs having SEQ ID NOs:2, 3, 4, 7, 8 and 10, and wherein the antibody or antigen-binding fragment thereof is administered in the absence of statin therapy at a frequency of once every two or four weeks at a dose of 75 mg or of once every two or four weeks at a dose of 150 mg.

2. The pharmaceutical composition for use of claim 1 wherein the patient previously experienced a skeletal muscle-related symptom that began or increased while taking a lowest approved daily dose of one or more statin.

3. The pharmaceutical composition for use of claim 1 or 2, wherein the patient previously experienced a skeletal muscle-related symptom that began or increased while taking at least two separate daily therapeutic statin regimens.

4. The pharmaceutical composition for use of claim 3, wherein at least one of the daily therapeutic statin regimens is
(a) the lowest approved daily dose of a statin; or
(b) selected from the group consisting of: 5 mg rosuvastatin daily, 10 mg atorvastatin daily, 10 mg simvastatin daily, 20 mg lovastatin daily, 40 mg pravastatin daily, 40 mg fluvastatin daily, and 2 mg pitavastatin daily.

5. The pharmaceutical composition for use of any one of claims 1 to 4, wherein the patient, prior to or at the time of administration of the antibody or antigen-binding fragment thereof, exhibits hypercholesterolemia defined as a serum low-density lipoprotein cholesterol (LDL-C) level of greater than about 70 mg/dL or greater than about 100 mg/dL.

6. The pharmaceutical composition for use of any one of claims 1 to 5, wherein the patient has heterozygous Familial Hypercholesterolemia (heFH) or a form of hypercholesterolemia that is not Familial Hypercholesterolemia (non-FH).

7. The pharmaceutical composition for use of any one of claims 1 to 6, wherein the patient has a moderate, high, or very high cardiovascular risk.

8. The pharmaceutical composition for use of claim 7, wherein the patient, prior to or at the time of administration of the antibody or antigen-binding fragment thereof, has
(a) a moderate cardiovascular risk defined as a calculated 10-year fatal cardiovascular disease risk SCORE greater than or equal to 1% and less than 5%;
(b) a high cardiovascular risk defined as a calculated 10-year fatal cardiovascular disease risk SCORE greater than or equal to 5% along with one or more of: (i) moderate chronic kidney disease, (ii) type 1 diabetes mellitus without target organ damage, (iii) type 2 diabetes mellitus without target organ damage, and/or (iv) heFH; or(c) a very high cardiovascular risk defined as one or more of: (i) documented coronary heart disease; (ii) ischemic stroke; (iii) peripheral stroke; (iv) peripheral arterial disease (PAD); (v) transient ischemic attack (TIA); (vi) abdominal aortic aneurysm; (vii) carotid artery occlusion >50% without symptoms; (viii) carotid endarterectomy; (ix) carotid artery stent procedure; (x) renal artery stenosis; (xi) renal artery stent procedure; (xii) type 1 diabetes mellitus with target organ damage; and/or (xiii) type 2 diabetes mellitus with target organ damage.

9. The pharmaceutical composition for use of any one of claims 1 to 8, wherein the antibody or antigen binding fragment thereof comprises a HCVR having the amino acid sequence of SEQ ID NO:1 and an LCVR having the amino acid sequence of SEQ ID NO:6.

10. The pharmaceutical composition for use of any one of claims 1 to 9, wherein five initial doses of the pharmaceutical composition comprising 75 mg of the antibody or antigen-binding fragment thereof are administered every two weeks, and wherein
(a) one or more further doses of the pharmaceutical composition comprising 75 mg of the antibody or antigen-binding fragment thereof are administered every two weeks if the LDL-C level of the patient after the initial doses is lower than 70 mg/dL; or
(b) one or more further doses of the pharmaceutical composition comprising 150 mg of the antibody or antigen-binding fragment thereof are administered every two weeks if the LDL-C level of the patient after the initial doses is greater than or equal to 70 mg/dL.

11. Pharmaceutical composition for use in one of claims 1 to 10, wherein the pharmaceutical composition improves the serum levels of one or more lipid components selected from the group consisting of:
(a) reduction of the patient's low density lipoprotein cholesterol (LDL-C) by at least 35%;
(b) reduction of the patient's apolipoprotein B (ApoB) by at least 25%;
(c) reduction of the patient's non-high density lipoproprotein cholesterol (non-HDL-C) by at least 30%;
(d) reduction of the patient's total cholesterol by at least 20%; and
(e) reduction of the patient's lipoprotein a (Lp(a)) by at least 15%.

## Patentansprüche

1. Pharmazeutische Zusammensetzung, umfassend 75 mg oder 150 mg eines spezifisch an humanes PCSK9 bindenden Antikörpers oder Antigen-bindenden Fragments davon, zur Verwendung in der Behandlung von Hypercholesterinämie in einem Patienten, der intolerant gegenüber Statinen ist oder der eine Historie unerwünschter Wirkungen durch Statin Therapie hat, wobei der Antikörper oder das Antigen-bindende Fragment davon komplementäre Bestimmungsregionen gemäß der SEQ ID NOs: 2, 3, 4, 7, 8 und 10 in einer schweren und leichten Kette umfasst, und wobei der Antikörper oder das Antigen-bindenden Fragment davon in Abwesenheit einer Statin Therapie mit einer Häufigkeit von einmal alle zwei oder vier Wochen in einer Dosis von 75 mg oder mit einer Häufigkeit von einmal alle zwei oder vier Wochen in einer Dosis von 150 mg verabreicht wird.

2. Pharmazeutische Zusammensetzung zur Verwendung gemäß Anspruch 1, wobei der Patient vorher an einem Skelettmuskel-Syndrom litt, welches während der Einnahme einer niedrigsten, zugelassenen, täglichen Dosis eines oder mehrerer Statine begann oder sich verstärkte.

3. Pharmazeutische Zusammensetzung zur Verwendung gemäß Anspruch 1 oder 2, wobei der Patient vorher an einem Skelettmuskel-Syndrom litt, welches während der Einnahme von mindestens zwei separaten, täglichen, therapeutischen Statin Regimen begann oder sich verstärkte.

4. Pharmazeutische Zusammensetzung zur Verwendung gemäß Anspruch 3, wobei mindestens eines der täglichen, therapeutischen Statin Regime wie folgt ist:
(a) die niedrigste, zugelassene, tägliche Dosis eines Statins; oder
(b) ausgewählt aus der Gruppe bestehend aus 5 mg Rosuvastatin täglich, 10 mg Atorvastatin täglich, 10 mg Simvastatin täglich, 20 mg Lovastatin täglich, 40 mg Pravastatin täglich, 40 mg Fluvastatin täglich, und 2 mg Pitavastatin täglich.

5. Pharmazeutische Zusammensetzung zur Verwendung gemäß einem der Ansprüche 1-4, wobei der Patient vor oder während der Verabreichung des Antikörpers oder Antigen-bindenden Fragments davon Hypercholesterinämie aufweist, welche durch ein Serumlevel von low-density lipoprotein Cholesterol (LDL-C) von größer als ungefähr 70 mg/dL oder größer als ungefähr 100 mg/dL definiert ist.

6. Pharmazeutische Zusammensetzung zur Verwendung gemäß einem der Ansprüche 1-5, wobei der Patient heterozygote familiäre Hypercholesterinämie (heFH) oder eine Form der Hypercholesterinämie hat, welche nicht familiäre Hypercholesterinämie (nicht-FH) ist.

7. Pharmazeutische Zusammensetzung zur Verwendung gemäß einem der Ansprüche 1-6, wobei der Patient ein moderates, hohes oder sehr hohes kardiovaskuläres Risiko hat.

8. Pharmazeutische Zusammensetzung zur Verwendung gemäß Anspruch 7, wobei der Patient vor oder während der Verabreichung des Antikörpers oder Antigen-bindenden Fragments davon
(a) ein moderates kardiovaskuläres Risiko hat, welches durch einen berechneten 10 Jahre tödliches kardiovaskuläres Erkrankungsrisiko SCORE größer oder gleich 1% und weniger als 5% definiert ist;
(b) ein hohes kardiovaskuläres Risiko hat, welches durch einen berechneten 10 Jahre tödliches kardiovaskuläres Erkrankungsrisiko SCORE größer oder gleich 5%, zusammen mit einer oder mehrerer Erkrankungen (i) moderate chronische Nierenerkrankung; (ii) Type 1 Diabetes mellitus ohne Zielorganschaden; (iii) Type 2 Diabetes mellitus ohne Zielorganschaden, und/oder (iv) heFH definiert ist; oder
(c) ein sehr hohes kardiovaskuläres Risiko hat, welches durch eine oder mehrere Erkrankungen von (i) dokumentierte koronare Herzkrankheit; (ii) ischämischer Schlaganfall; (iii) peripherer Schlaganfall; (iv) periphere arterielle Erkrankung (PAE); (v) transiente ischämische Attacke (TIA); (vi) abdominales Aortenaneurysma; (vii) karotische arterielle Okklusion >50% ohne Symptome; (viii) Karotisendarteriektomie; (ix) Karotis Stent-Verfahren; (x) Nierenarterienstenose; (xi) Nierenarterienstent-Verfahren; (xii) Type 1 Diabetes mellitus mit Zielorganschaden; und/oder (xiii) Type 2 Diabetes mellitus mit Zielorganschaden definiert ist.

9. Pharmazeutische Zusammensetzung zur Verwendung gemäß einem der Ansprüche 1-8, wobei der Antikörper oder das Antigen-bindende Fragment davon eine variable Region einer schweren Kette gemäß der Aminosäuresequenz SEQ ID NO: 1 und eine variable Region einer leichten Kette gemäß der Aminosäuresequenz SEQ ID NO: 6 umfasst.

10. Pharmazeutische Zusammensetzung zur Verwendung gemäß einem der Ansprüche 1-9, wobei fünf Anfangsdosen der pharmazeutischen Zusammensetzung, welche 75 mg des Antikörpers oder Antigen-bindenden Fragment davon umfasst, alle zwei Wochen verabreicht werden, und wobei
(a) eine oder mehrere weitere Dosen der pharmazeutischen Zusammensetzung, welche 75 mg des Antikörpers oder Antigen-bindenden Fragment davon umfasst, alle zwei Wochen verabreicht werden, wenn das LDL-C Level des Patienten nach der Anfangsdosis niedriger als 70 mg/dL ist; oder
(b) eine oder mehrere weitere Dosen der pharmazeutischen Zusammensetzung, welche 150 mg des Antikörpers oder Antigen-bindenden Fragment davon umfasst, alle zwei Wochen verabreicht werden, wenn das LDL-C Level des Patienten nach der Anfangsdosis größer oder gleich 70 mg/dL ist.

11. Pharmazeutische Zusammensetzung zur Verwendung gemäß einem der Ansprüche 1-10, wobei die pharmazeutische Zusammensetzung die Serumlevel einer oder mehrerer Lipidkomponenten verbessert, welche ausgewählt sind aus der Gruppe bestehend aus:
(a) Reduktion des low-densitiy lipoprotein Cholesterols (LDL-C) des Patienten um mindestens 35%;
(b) Reduktion des Apolipoprotein B (ApoB) des Patienten um mindestens 25%;
(c) Reduktion des non-high densitiy lipoprotein Cholesterols (non HDL-C) des Patienten um mindestens 30%;
(d) Reduktion des Gesamtcholesterols des Patienten um mindestens 20%; und
(e) Reduktion des Lipoprotein a (Lpa) des Patienten um mindestens 15%.

## Revendications

1. Composition pharmaceutique comprenant 75 mg ou 150 mg d'un anticorps ou fragment se liant à l'antigène d'un tel anticorps, qui se lie spécifiquement à la PCSK9 humaine, destinée à l'utilisation dans le traitement de l'hypercholestérolémie chez un patient qui est intolérant aux statines ou qui a des antécédents de réactions indésirables à un traitement par des statines, dans laquelle l'anticorps ou le fragment se liant à l'antigène d'un tel anticorps comprend les CDR de chaîne lourde et de chaîne légère ayant les séquences SEQ ID NO : 2, 3, 4, 7, 8 et 10, et dans laquelle l'anticorps ou le fragment se liant à l'antigène d'un tel anticorps est administré en l'absence de traitement par des statines à une fréquence d'une fois toutes les deux ou quatre semaines à une dose de 75 mg ou d'une fois toutes les deux ou quatre semaines à une dose de 150 mg.

2. Composition pharmaceutique destinée à l'utilisation selon la revendication 1, dans laquelle le patient a éprouvé précédemment un symptôme en relation avec des muscles squelettiques, qui est apparu ou a augmenté lors de la prise d'une dose journalière approuvée la plus faible d'une ou de plusieurs statine(s).

3. Composition pharmaceutique destinée à l'utilisation selon la revendication 1 ou 2, dans laquelle le patient a éprouvé précédemment un symptôme en relation avec des muscles squelettiques, qui est apparu ou a augmenté lors de la prise d'au moins deux schémas thérapeutiques journaliers distincts de statines.

4. Composition pharmaceutique destinée à l'utilisation selon la revendication 3, dans laquelle au moins un des schémas thérapeutiques journaliers de statines est
(a) la plus faible dose journalière approuvée d'une statine ; ou
(b) choisi dans l'ensemble constitué par : 5 mg de rosuvastatine par jour, 10 mg d'atorvastatine par jour, 10 mg de simvastatine par jour, 20 mg de lovastatine par jour, 40 mg de pravastatine par jour, 40 mg de fluvastatine par jour, et 2 mg de pitavastatine par jour.

5. Composition pharmaceutique destinée à l'utilisation selon l'une quelconque des revendications 1 à 4, dans laquelle le patient, avant ou au moment de l'administration de l'anticorps ou du fragment se liant à l'antigène d'un tel anticorps, présente une hypercholestérolémie définie comme un taux sérique de cholestérol lié aux lipoprotéines de basse densité (C-LDL) supérieur à environ 70 mg/dL ou supérieur à environ 100 mg/dL.

6. Composition pharmaceutique destinée à l'utilisation selon l'une quelconque des revendications 1 à 5, dans laquelle le patient à une hypercholestérolémie familiale hétérozygote (heFH) ou une forme d'hypercholestérolémie qui n'est pas une hypercholestérolémie familiale (non-FH).

7. Composition pharmaceutique destinée à l'utilisation selon l'une quelconque des revendications 1 à 6, dans laquelle le patient présente un risque cardiovasculaire modéré, élevé, ou très élevé.

8. Composition pharmaceutique destinée à l'utilisation selon la revendication 7, dans laquelle le patient, avant ou au moment de l'administration de l'anticorps ou du fragment se liant à l'antigène d'un tel anticorps, présente
(a) un risque cardiovasculaire modéré défini comme un SCORE calculé de risque de maladie cardiovasculaire fatale sur 10 ans supérieur ou égal à 1 % et inférieur à 5 % ;
(b) un risque cardiovasculaire élevé défini comme un SCORE calculé de risque de maladie cardiovasculaire fatale sur 10 ans supérieur ou égal à 5 %, conjointement avec un(e) ou plusieurs parmi : (i) une néphropathie chronique modérée, (ii) un diabète sucré de type 1 sans lésion d'organe cible, (iii) un diabète sucré de type 2 sans lésion d'organe cible, et/ou (iv) une heFH ; ou
(c) un risque cardiovasculaire très élevé défini comme un(e) ou plusieurs parmi :
(i) une cardiopathie coronarienne documentée ;
(ii) un accident vasculaire cérébral ischémique ;
(iii) un accident vasculaire périphérique ;
(iv) une artériopathie périphérique (AP) ;
(v) une attaque ischémique transitoire (AIT) ;
(vi) un anévrisme aortique abdominal ;
(vii) une occlusion d'artère carotidienne > 50 % sans symptômes ;
(viii) une endartériectomie carotidienne ;
(ix) la pose d'une endoprothèse d'artère carotidienne ;
(x) une sténose d'artère rénale ;
(xi) la pose d'une endoprothèse d'artère rénale ; (xii) un diabète sucré de type 1 avec lésion d'organe cible ; et/ou un diabète sucré de type 2 avec lésion d'organe cible.

9. Composition pharmaceutique destinée à l'utilisation selon l'une quelconque des revendications 1 à 8, dans laquelle l'anticorps ou le fragment se liant à l'antigène d'un tel anticorps comprend une HCVR ayant la séquence d'acides aminés de la séquence SEQ ID NO : 1 et une LCVR ayant la séquence d'acides aminés de la séquence SEQ ID NO : 6.

10. Composition pharmaceutique destinée à l'utilisation selon l'une quelconque des revendications 1 à 9, dans laquelle cinq doses initiales de la composition pharmaceutique comprenant 75 mg de l'anticorps ou du fragment se liant à l'antigène d'un tel anticorps sont administrées toutes les deux semaines, et dans laquelle
(a) une ou plusieurs dose(s) supplémentaire(s) de la composition pharmaceutique comprenant 75 mg de l'anticorps ou du fragment se liant à l'antigène d'un tel anticorps est/sont administrée(s) toutes les deux semaines si le taux de C-LDL du patient après les doses initiales est inférieur à 70 mg/dL ; ou
(b) une ou plusieurs dose(s) supplémentaire(s) de la composition pharmaceutique comprenant 150 mg de l'anticorps ou du fragment se liant à l'antigène d'un tel anticorps est/sont administrée(s) toutes les deux semaines si le taux de C-LDL du patient après les doses initiales est supérieur ou égal à 70 mg/dL

11. Composition pharmaceutique destinée à l'utilisation selon l'une quelconque des revendications 1 à 10, où la composition pharmaceutique améliore les taux sériques d'un ou de plusieurs composant(s) lipidique(s) choisi(s) dans l'ensemble constitué par :
(a) un abaissement d'au moins 35 % du taux de cholestérol lié aux lipoprotéines de basse densité (C-LDL) du patient ;
(b) un abaissement d'au moins 25 % du taux d'apolipoprotéine B (ApoB) du patient ;
(c) un abaissement d'au moins 30 % du taux de cholestérol non lié aux lipoprotéines de haute densité (C-non-HDL) du patient ;
(d) un abaissement d'au moins 20 % du taux de cholestérol total du patient ; et
(e) un abaissement d'au moins 15 % du taux de lipoprotéine a (Lp(a)) du patient.


### SEQUENCE LISTING

<110> BACCARA-DINET, MARIE BESSAC, LAURENCE CHAUDHARI, UMESH HANOTIN, CORINNE PORDY, ROBERT C. SASIELA, WILLIAM J. SCHWEMMER GIPE, DANIEL A.
<120> USE OF A PCSK9 INHIBITOR TO TREAT HYPERLIPIDEMIA
<130> US2013/257 PCT
<140>
   <141>
<160> 198
<170> PatentIn version 3.5
<210> 1
   <211> 118
   <212> PRT
   <213> Artificial Sequence
<220>
   <223> Description of Artificial Sequence: Synthetic polypeptide
<400> 1
<210> 2
   <211> 8
   <212> PRT
   <213> Artificial Sequence
<220>
   <223> Description of Artificial Sequence: Synthetic peptide
<400> 2
   Gly Phe Thr Phe Asn Asn Tyr Ala
   1               5
<210> 3
   <211> 8
   <212> PRT
   <213> Artificial Sequence
<220>
   <223> Description of Artificial Sequence: Synthetic peptide
<400> 3
   Ile Ser Gly Ser Gly Gly Thr Thr
   1               5
<210> 4
   <211> 11
   <212> PRT
   <213> Artificial Sequence
<220>
   <223> Description of Artificial Sequence: Synthetic peptide
<400> 4
   Ala Lys Asp Ser Asn Trp Gly Asn Phe Asp Leu
   1               5                   10
<210> 5
   <211> 447
   <212> PRT
   <213> Artificial Sequence
<220>
   <223> Description of Artificial Sequence: Synthetic REGN727 heavy chain polypeptide
<400> 5
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Asn Asn Tyr
               20                  25                  30
   Ala Met Asn Trp Val Arg Gln Ala Pro Gly Lys Gly Leu Asp Trp Val
           35                  40                  45
   Ser Thr Ile Ser Gly Ser Gly Gly Thr Thr Asn Tyr Ala Asp Ser Val
       50                  55                  60
   Lys Gly Arg Phe Ile Ile Ser Arg Asp Ser Ser Lys His Thr Leu Tyr
   65                  70                  75                  80
   Leu Gln Met Asn Ser Leu Arg Ala Glu Asp Thr Ala Val Tyr Tyr Cys
                   85                  90                  95
   Ala Lys Asp Ser Asn Trp Gly Asn Phe Asp Leu Trp Gly Arg Gly Thr
               100                 105                 110
   Leu Val Thr Val Ser Ser Ala Ser Thr Lys Gly Pro Ser Val Phe Pro
           115                 120                 125
   Leu Ala Pro Ser Ser Lys Ser Thr Ser Gly Gly Thr Ala Ala Leu Gly
       130                 135                 140
   Cys Leu Val Lys Asp Tyr Phe Pro Glu Pro Val Thr Val Ser Trp Asn
   145                 150                 155                 160
   Ser Gly Ala Leu Thr Ser Gly Val His Thr Phe Pro Ala Val Leu Gln
                   165                 170                 175
   Ser Ser Gly Leu Tyr Ser Leu Ser Ser Val Val Thr Val Pro Ser Ser
               180                 185                 190
   Ser Leu Gly Thr Gln Thr Tyr Ile Cys Asn Val Asn His Lys Pro Ser
           195                 200                 205
   Asn Thr Lys Val Asp Lys Lys Val Glu Pro Lys Ser Cys Asp Lys Thr
       210                 215                 220
   His Thr Cys Pro Pro Cys Pro Ala Pro Glu Leu Leu Gly Gly Pro Ser
   225                 230                 235                 240
   Val Phe Leu Phe Pro Pro Lys Pro Lys Asp Thr Leu Met Ile Ser Arg
                   245                 250                 255
   Thr Pro Glu Val Thr Cys Val Val Val Asp Val Ser His Glu Asp Pro
               260                 265                 270
   Glu Val Lys Phe Asn Trp Tyr Val Asp Gly Val Glu Val His Asn Ala
           275                 280                 285
   Lys Thr Lys Pro Arg Glu Glu Gln Tyr Asn Ser Thr Tyr Arg Val Val
       290                 295                 300
   Ser Val Leu Thr Val Leu His Gln Asp Trp Leu Asn Gly Lys Glu Tyr
   305                 310                 315                 320
   Lys Cys Lys Val Ser Asn Lys Ala Leu Pro Ala Pro Ile Glu Lys Thr
                   325                 330                 335
   Ile Ser Lys Ala Lys Gly Gln Pro Arg Glu Pro Gln Val Tyr Thr Leu
               340                 345                 350
   Pro Pro Ser Arg Asp Glu Leu Thr Lys Asn Gln Val Ser Leu Thr Cys
           355                 360                 365
   Leu Val Lys Gly Phe Tyr Pro Ser Asp Ile Ala Val Glu Trp Glu Ser
       370                 375                 380
   Asn Gly Gln Pro Glu Asn Asn Tyr Lys Thr Thr Pro Pro Val Leu Asp
   385                 390                 395                 400
   Ser Asp Gly Ser Phe Phe Leu Tyr Ser Lys Leu Thr Val Asp Lys Ser
                   405                 410                 415
   Arg Trp Gln Gln Gly Asn Val Phe Ser Cys Ser Val Met His Glu Ala
               420                 425                 430
   Leu His Asn His Tyr Thr Gln Lys Ser Leu Ser Leu Ser Pro Gly
           435                 440                 445
<210> 6
   <211> 113
   <212> PRT
   <213> Artificial Sequence
<220>
   <223> Description of Artificial Sequence: Synthetic polypeptide
<400> 6
<210> 7
   <211> 12
   <212> PRT
   <213> Artificial Sequence
<220>
   <223> Description of Artificial Sequence: Synthetic peptide
<400> 7
   Gln Ser Val Leu Tyr Arg Ser Asn Asn Arg Asn Phe
   1 5 10
<210> 8
   <211> 3
   <212> PRT
   <213> Artificial Sequence
<220>
   <223> Description of Artificial Sequence: Synthetic peptide
<400> 8
   Trp Ala Ser
   1
<210> 9
   <211> 220
   <212> PRT
   <213> Artificial Sequence
<220>
   <223> Description of Artificial Sequence: Synthetic REGN727 light chain polypeptide
<400> 9
   Asp Ile Val Met Thr Gln Ser Pro Asp Ser Leu Ala Val Ser Leu Gly
   1 5 10 15
   Glu Arg Ala Thr Ile Asn Cys Lys Ser Ser Gln Ser Val Leu Tyr Arg
   20 25 30
   Ser Asn Asn Arg Asn Phe Leu Gly Trp Tyr Gln Gln Lys Pro Gly Gln
           35                  40                  45
   Pro Pro Asn Leu Leu Ile Tyr Trp Ala Ser Thr Arg Glu Ser Gly Val
       50                  55                  60
   Pro Asp Arg Phe Ser Gly Ser Gly Ser Gly Thr Asp Phe Thr Leu Thr
   65                  70                  75                  80
   Ile Ser Ser Leu Gln Ala Glu Asp Val Ala Val Tyr Tyr Cys Gln Gln
                   85                  90                  95
   Tyr Tyr Thr Thr Pro Tyr Thr Phe Gly Gln Gly Thr Lys Leu Glu Ile
               100                 105                 110
   Lys Arg Thr Val Ala Ala Pro Ser Val Phe Ile Phe Pro Pro Ser Asp
           115                 120                 125
   Glu Gln Leu Lys Ser Gly Thr Ala Ser Val Val Cys Leu Leu Asn Asn
       130                 135                 140
   Phe Tyr Pro Arg Glu Ala Lys Val Gln Trp Lys Val Asp Asn Ala Leu
   145                 150                 155                 160
   Gln Ser Gly Asn Ser Gln Glu Ser Val Thr Glu Gln Asp Ser Lys Asp
                   165                 170                 175
   Ser Thr Tyr Ser Leu Ser Ser Thr Leu Thr Leu Ser Lys Ala Asp Tyr
               180                 185                 190
   Glu Lys His Lys Val Tyr Ala Cys Glu Val Thr His Gln Gly Leu Ser
           195                 200                 205
   Ser Pro Val Thr Lys Ser Phe Asn Arg Gly Glu Cys
       210                 215                 220
<210> 10
   <211> 9
   <212> PRT
   <213> Artificial Sequence
<220>
   <223> Description of Artificial Sequence: Synthetic peptide
<400> 10
   Gln Gln Tyr Tyr Thr Thr Pro Tyr Thr
   1               5
<210> 11
   <211> 127
   <212> PRT
   <213> Artificial Sequence
<220>
   <223> Description of Artificial Sequence: Synthetic polypeptide
<400> 11
   Glu Met Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser His
               20                  25                  30
   Trp Met Lys Trp Val Arg Gln Ala Pro Gly Lys Gly Leu Glu Trp Val
           35                  40                  45
   Ala Asn Ile Asn Gln Asp Gly Ser Glu Lys Tyr Tyr Val Asp Ser Val
       50                  55                  60
   Lys Gly Arg Phe Thr Ile Ser Arg Asp Asn Ala Lys Asn Ser Leu Phe
   65                  70                  75                  80
   Leu Gln Met Asn Ser Leu Arg Ala Glu Asp Thr Ala Val Tyr Tyr Cys
                   85                  90                  95
   Ala Arg Asp Ile Val Leu Met Val Tyr Asp Met Asp Tyr Tyr Tyr Tyr
               100                 105                 110
   Gly Met Asp Val Trp Gly Gln Gly Thr Thr Val Thr Val Ser Ser
           115                 120                 125
<210> 12
   <211> 8
   <212> PRT
   <213> Artificial Sequence
<220>
   <223> Description of Artificial Sequence: Synthetic peptide
<400> 12
   Gly Phe Thr Phe Ser Ser His Trp
   1               5
<210> 13
   <211> 8
   <212> PRT
   <213> Artificial Sequence
<220>
   <223> Description of Artificial Sequence: Synthetic peptide
<400> 13
   Ile Asn Gln Asp Gly Ser Glu Lys
   1               5
<210> 14
   <211> 20
   <212> PRT
   <213> Artificial Sequence
<220>
   <223> Description of Artificial Sequence: Synthetic peptide
<400> 14
   Ala Arg Asp Ile Val Leu Met Val Tyr Asp Met Asp Tyr Tyr Tyr Tyr
   1               5                   10                  15
   Gly Met Asp Val
               20
<210> 15
   <211> 112
   <212> PRT
   <213> Artificial Sequence
<220>
   <223> Description of Artificial Sequence: Synthetic polypeptide
<400> 15
   Asp Ile Val Met Thr Gln Ser Pro Leu Ser Leu Pro Val Thr Pro Gly
   1               5                   10                  15
   Glu Pro Ala Ser Ile Ser Cys Arg Ser Ser Gln Ser Leu Leu His Ser
               20                  25                  30
   Asn Gly Asn Asn Tyr Leu Asp Trp Tyr Leu Gln Lys Pro Gly Gln Ser
           35                  40                  45
   Pro Gln Leu Leu Ile Tyr Leu Gly Ser Asn Arg Ala Ser Gly Val Pro
       50                  55                  60
   Asp Arg Phe Ser Gly Ser Gly Ser Gly Thr Asp Phe Thr Leu Lys Ile
   65                  70                  75                  80
   Ser Arg Val Glu Ala Glu Asp Val Gly Val Tyr Tyr Cys Met Gln Thr
                   85                  90                  95
   Leu Gln Thr Pro Leu Thr Phe Gly Gly Gly Thr Lys Val Glu Ile Lys
               100                 105                 110
<210> 16
   <211> 11
   <212> PRT
   <213> Artificial Sequence
<220>
   <223> Description of Artificial Sequence: Synthetic peptide
<400> 16
   Gln Ser Leu Leu His Ser Asn Gly Asn Asn Tyr
   1               5                   10
<210> 17
   <211> 3
   <212> PRT
   <213> Artificial Sequence
<220>
   <223> Description of Artificial Sequence: Synthetic peptide
<400> 17
   Leu Gly Ser
   1
<210> 18
   <211> 9
   <212> PRT
   <213> Artificial Sequence
<220>
   <223> Description of Artificial Sequence: Synthetic peptide
<400> 18
   Met Gln Thr Leu Gln Thr Pro Leu Thr
   1 5
<210> 19
   <211> 8
   <212> PRT
   <213> Artificial Sequence
<220>
   <223> Description of Artificial Sequence: Synthetic peptide
<400> 19
   Gly Phe Thr Phe Ser Ser His Trp
   1 5
<210> 20
   <211> 8
   <212> PRT
   <213> Artificial Sequence
<220>
   <223> Description of Artificial Sequence: Synthetic peptide
<400> 20
   Ile Asn Gln Asp Gly Ser Glu Lys
   1 5
<210> 21
   <211> 20
   <212> PRT
   <213> Artificial Sequence
<220>
   <223> Description of Artificial Sequence: Synthetic peptide
<400> 21 Gly Met Asp Val 20
<210> 22
   <211> 11
   <212> PRT
   <213> Artificial Sequence
<220>
   <223> Description of Artificial Sequence: Synthetic peptide
<400> 22
   Gln Ser Leu His His Ser Asn Gly Asn Asn Tyr
   1 5 10
<210> 23
   <211> 3
   <212> PRT
   <213> Artificial Sequence
<220>
   <223> Description of Artificial Sequence: Synthetic peptide
<400> 23
   Leu Gly Ser
   1
<210> 24
   <211> 9
   <212> PRT
   <213> Artificial Sequence
<220>
   <223> Description of Artificial Sequence: Synthetic peptide
<400> 24
   Met Gln Thr Leu Gln Thr Pro Leu Thr
   1 5
<210> 25
   <211> 8
   <212> PRT
   <213> Artificial Sequence
<220>
   <223> Description of Artificial Sequence: Synthetic peptide
<400> 25
   Gly Phe Thr Phe Ser Ser His Trp
   1 5
<210> 26
   <211> 8
   <212> PRT
   <213> Artificial Sequence
<220>
   <223> Description of Artificial Sequence: Synthetic peptide
<400> 26
   Ile Asn Gln Asp Gly Ser Glu Lys
   1 5
<210> 27
   <211> 20
   <212> PRT
   <213> Artificial Sequence
<220>
   <223> Description of Artificial Sequence: Synthetic peptide
<400> 27 Gly Met Asp Val 20
<210> 28
   <211> 11
   <212> PRT
   <213> Artificial Sequence
<220>
   <223> Description of Artificial Sequence: Synthetic peptide
<400> 28
   Gln Ser Leu Leu His Ser Asn Gly Asn Asn Tyr
   1 5 10
<210> 29
   <211> 3
   <212> PRT
   <213> Artificial Sequence
<220>
   <223> Description of Artificial Sequence: Synthetic peptide
<400> 29
   Leu Gly Ser
   1
<210> 30
   <211> 9
   <212> PRT
   <213> Artificial Sequence
<220>
   <223> Description of Artificial Sequence: Synthetic peptide
<400> 30
   Met Gln Thr Leu Gln Thr Pro Leu Thr
   1 5
<210> 31
   <211> 8
   <212> PRT
   <213> Artificial Sequence
<220>
   <223> Description of Artificial Sequence: Synthetic peptide
<400> 31
   Gly Phe Thr Phe Ser Ser His Trp
   1               5
<210> 32
   <211> 8
   <212> PRT
   <213> Artificial Sequence
<220>
   <223> Description of Artificial Sequence: Synthetic peptide
<400> 32
   Ile Asn Gln Asp Gly Ser Glu Lys
   1               5
<210> 33
   <211> 20
   <212> PRT
   <213> Artificial Sequence
<220>
   <223> Description of Artificial Sequence: Synthetic peptide
<400> 33
   Ala Arg Asp Ile Val Leu Met Val Tyr His Met Asp Tyr Tyr Tyr Tyr
   1               5                   10                  15
   Gly Met Asp Val
               20
<210> 34
   <211> 11
   <212> PRT
   <213> Artificial Sequence
<220>
   <223> Description of Artificial Sequence: Synthetic peptide
<400> 34
   Gln Ser Leu His His Ser Asn Gly Asn Asn Tyr
   1               5                   10
<210> 35
   <211> 3
   <212> PRT
   <213> Artificial Sequence
<220>
   <223> Description of Artificial Sequence: Synthetic peptide
<400> 35
   Leu Gly Ser
   1
<210> 36
   <211> 9
   <212> PRT
   <213> Artificial Sequence
<220>
   <223> Description of Artificial Sequence: Synthetic peptide
<400> 36
   Met Gln Thr Leu Gln Thr Pro Leu Thr
   1               5
<210> 37
   <211> 131
   <212> PRT
   <213> Artificial Sequence
<220>
   <223> Description of Artificial Sequence: Synthetic VH; m2CX1D05 polypeptide
<400> 37
   Gln Val Gln Leu Val Gln Ser Gly Ala Glu Val Lys Lys Pro Gly Ser
   1               5                   10                  15
   Ser Val Lys Val Ser Cys Lys Ala Ser Gly Gly Thr Phe Asn Ser His
               20                  25                  30
   Ala Ile Ser Trp Val Arg Gln Ala Pro Gly Gln Gly Leu Glu Trp Met
           35                  40                  45
   Gly Gly Ile Asn Pro Ile Leu Gly Ile Ala Asn Tyr Ala Gln Lys Phe
       50                  55                  60
   Gln Gly Arg Val Thr Ile Thr Ala Asp Glu Ser Thr Ser Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg His Tyr Glu Ile Gln Ile Gly Arg Tyr Gly Met Asn Val Tyr
               100                 105                 110
   Tyr Leu Met Tyr Arg Phe Ala Ser Trp Gly Gln Gly Thr Leu Val Thr
           115                 120                 125
   Val Ser Ser
       130
<210> 38
   <211> 10
   <212> PRT
   <213> Artificial Sequence
<220>
   <223> Description of Artificial Sequence: Synthetic VH CDR1; m2CX1D05 peptide
<400> 38
   Gly Gly Thr Phe Asn Ser His Ala Ile Ser
   1               5                   10
<210> 39
   <211> 20
   <212> PRT
   <213> Artificial Sequence
<220>
   <223> Description of Artificial Sequence: Synthetic VH CDR2; m2CX1D05 peptide
<400> 39
   Trp Met Gly Gly Ile Asn Pro Ile Leu Gly Ile Ala Asn Tyr Ala Gln
   1               5                   10                  15
   Lys Phe Gln Gly
   20
<210> 40
   <211> 22
   <212> PRT
   <213> Artificial Sequence
<220>
   <223> Description of Artificial Sequence: Synthetic VH CDR3; m2CX1D05 peptide
<400> 40
   His Tyr Glu Ile Gln Ile Gly Arg Tyr Gly Met Asn Val Tyr Tyr Leu
   1 5 10 15
   Met Tyr Arg Phe Ala Ser
   20
<210> 41
   <211> 213
   <212> PRT
   <213> Artificial Sequence
<220>
   <223> Description of Artificial Sequence: Synthetic LC; m2CX1D05 polypeptide
<400> 41
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Gly Ile Arg Ser Ala
               20                  25                  30
   Leu Asn Trp Tyr Gln Gln Lys Pro Gly Lys Ala Pro Lys Leu Leu Ile
           35                  40                  45
   Tyr Asn Gly Ser Thr Leu Gln Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Val Tyr Tyr Cys Gln Gln Phe Asp Gly Asp Pro Thr
                   85                  90                  95
   Phe Gly Gln Gly Thr Lys Val Glu Ile Lys Arg Thr Val Ala Ala Pro
               100                 105                 110
   Ser Val Phe Ile Phe Pro Pro Ser Asp Glu Gln Leu Lys Ser Gly Thr
           115                 120                 125
   Ala Ser Val Val Cys Leu Leu Asn Asn Phe Tyr Pro Arg Glu Ala Lys
       130                 135                 140
   Val Gln Trp Lys Val Asp Asn Ala Leu Gln Ser Gly Asn Ser Gln Glu
   145                 150                 155                 160
   Ser Val Thr Glu Gln Asp Ser Lys Asp Ser Thr Tyr Ser Leu Ser Ser
                   165                 170                 175
   Thr Leu Thr Leu Ser Lys Ala Asp Tyr Glu Lys His Lys Val Tyr Ala
               180                 185                 190
   Cys Glu Val Thr His Gln Gly Leu Ser Ser Pro Val Thr Lys Ser Phe
           195                 200                 205
   Asn Arg Gly Glu Ala
       210
<210> 42
   <211> 11
   <212> PRT
   <213> Artificial Sequence
<220>
   <223> Description of Artificial Sequence: Synthetic VL CDR 1; m2CX1D05 peptide
<400> 42
   Arg Ala Ser Gln Gly Ile Arg Ser Ala Leu Asn
   1 5 10
<210> 43
   <211> 11
   <212> PRT
   <213> Artificial Sequence
<220>
   <223> Description of Artificial Sequence: Synthetic VL CDR2; m2CX1D05 peptide
<400> 43
   Leu Leu Ile Tyr Asn Gly Ser Thr Leu Gln Ser
   1 5 10
<210> 44
   <211> 7
   <212> PRT
   <213> Artificial Sequence
<220>
   <223> Description of Artificial Sequence: Synthetic VL CDR3; m2CX1D05 peptide
<400> 44
   Gln Gln Phe Asp Gly Asp Pro
   1 5
<210> 45
   <211> 119
   <212> PRT
   <213> Artificial Sequence
<220>
   <223> Description of Artificial Sequence: Synthetic VH; 1B20 polypeptide
<400> 45
   Gln Val Gln Leu Val Gln Ser Gly Ala Glu Val Lys Lys Pro Gly Glu
   1               5                   10                  15
   Ser Leu Lys Ile Ser Cys Lys Gly Ser Gly Tyr Ser Phe Thr Asn Tyr
               20                  25                  30
   Trp Ile Ser Trp Val Arg Gln Met Pro Gly Lys Gly Leu Glu Trp Met
           35                  40                  45
   Gly Ile Ile Tyr Pro Gly Asp Ser Tyr Thr Asn Tyr Ser Pro Ser Phe
       50                  55                  60
   Gln Gly Gln Val Thr Ile Ser Ala Asp Lys Ser Ile Ser Thr Ala Tyr
   65                  70                  75                  80
   Leu Gln Trp Ser Ser Leu Lys Ala Ser Asp Thr Ala Met Tyr Tyr Cys
                   85                  90                  95
   Ala Arg Asp Tyr Trp Tyr Lys Pro Leu Phe Asp Ile Trp Gly Gln Gly
               100                 105                 110
   Thr Leu Val Thr Val Ser Ser
           115
<210> 46
   <211> 10
   <212> PRT
   <213> Artificial Sequence
<220>
   <223> Description of Artificial Sequence: Synthetic VH CDR1; 1B20 peptide
<400> 46
   Gly Tyr Ser Phe Thr Asn Tyr Trp Ile Ser
   1               5                   10
<210> 47
   <211> 20
   <212> PRT
   <213> Artificial Sequence
<220>
   <223> Description of Artificial Sequence: Synthetic VH CDR2; 1B20 peptide
<400> 47
   Trp Met Gly Ile Ile Tyr Pro Gly Asp Ser Tyr Thr Asn Tyr Ser Pro
   1               5                   10                  15
   Ser Phe Gln Gly
               20
<210> 48
   <211> 10
   <212> PRT
   <213> Artificial Sequence
<220>
   <223> Description of Artificial Sequence: Synthetic VH CDR3; 1B20 peptide
<400> 48
   Asp Tyr Trp Tyr Lys Pro Leu Phe Asp Ile
   1               5                   10
<210> 49
   <211> 220
   <212> PRT
   <213> Artificial Sequence
<220>
   <223> Description of Artificial Sequence: Synthetic LC; 1B20 polypeptide
<400> 49
   Asp Ile Val Met Thr Gln Ser Pro Asp Ser Leu Ala Val Ser Leu Gly
   1               5                   10                  15
   Glu Arg Ala Thr Ile Asn Cys Arg Ser Ser Gln Ser Val Leu Tyr Ser
               20                  25                  30
   Ser Asn Asn Lys Asn Tyr Leu Ala Trp Tyr Gln Gln Lys Pro Gly Gln
           35                  40                  45
   Pro Pro Lys Leu Leu Ile Tyr Trp Ala Ser Thr Arg Glu Ser Gly Val
       50                  55                  60
   Pro Asp Arg Phe Ser Gly Ser Gly Ser Gly Thr Asp Phe Thr Leu Thr
   65                  70                  75                  80
   Ile Ser Ser Leu Gln Ala Glu Asp Val Ala Val Tyr Tyr Cys Gln Gln
                   85                  90                  95
   Tyr Ser Ser Phe Pro Ile Thr Phe Gly Gln Gly Thr Lys Val Glu Ile
   100 105 110
   Lys Arg Thr Val Ala Ala Pro Ser Val Phe Ile Phe Pro Pro Ser Asp
   115 120 125
   Glu Gln Leu Lys Ser Gly Thr Ala Ser Val Val Cys Leu Leu Asn Asn
   130 135 140
   Phe Tyr Pro Arg Glu Ala Lys Val Gln Trp Lys Val Asp Asn Ala Leu
   145 150 155 160
   Gln Ser Gly Asn Ser Gln Glu Ser Val Thr Glu Gln Asp Ser Lys Asp
   165 170 175
   Ser Thr Tyr Ser Leu Ser Ser Thr Leu Thr Leu Ser Lys Ala Asp Tyr
   180 185 190
   Glu Lys His Lys Val Tyr Ala Cys Glu Val Thr His Gln Gly Leu Ser
   195 200 205
   Ser Pro Val Thr Lys Ser Phe Asn Arg Gly Glu Ala
   210 215 220
<210> 50
   <211> 17
   <212> PRT
   <213> Artificial Sequence
<220>
   <223> Description of Artificial Sequence: Synthetic VL CDR1; 1B20 peptide
<400> 50
   Arg Ser Ser Gln Ser Val Leu Tyr Ser Ser Asn Asn Lys Asn Tyr Leu
   1 5 10 15
   Ala
<210> 51
   <211> 11
   <212> PRT
   <213> Artificial Sequence
<220>
   <223> Description of Artificial Sequence: Synthetic VL CDR2; 1B20 peptide
<400> 51
   Leu Leu Ile Tyr Trp Ala Ser Thr Arg Glu Ser
   1 5 10
<210> 52
   <211> 8
   <212> PRT
   <213> Artificial Sequence
<220>
   <223> Description of Artificial Sequence: Synthetic VL CDR3; 1B20 peptide
<400> 52
   Gln Gln Tyr Ser Ser Phe Pro Ile
   1               5
<210> 53
   <211> 120
   <212> PRT
   <213> Artificial Sequence
<220>
   <223> Description of Artificial Sequence: Synthetic variable heavy antibody region polypeptide
<400> 53
   Glu Val Gln Leu Leu Glu Ser Gly Gly Gly Leu Val Gln Pro Gly Gly
   1               5                   10                  15
   Ser Leu Arg Leu Ser Cys Lys Ala Ser Gly Tyr Thr Phe Ser Ser Tyr
               20                  25                  30
   Gly Met Tyr Trp Val Arg Gln Ala Pro Gly Lys Gly Leu Glu Trp Ile
           35                  40                  45
   Gly Trp Ile Asp Pro Gly Ser Gly Gly Thr Lys Tyr Asn Glu Lys Phe
       50                  55                  60
   Lys Gly Lys Ala Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Glu Arg Tyr Gly Tyr Tyr Phe Asp Tyr Trp Gly Gln Gly Thr
               100                 105                 110
   Leu Val Thr Val Ser Ser Ala Ser
           115                 120
<210> 54
   <211> 16
   <212> PRT
   <213> Artificial Sequence
<220>
   <223> Description of Artificial Sequence: Synthetic AX132 heavy chain CDR1 antibody region peptide
<400> 54
   Lys Ala Ser Gly Tyr Thr Phe Ser Ser Tyr Gly Met Tyr Trp Val Arg
   1               5                   10                  15
<210> 55
   <211> 23
   <212> PRT
   <213> Artificial Sequence
<220>
   <223> Description of Artificial Sequence: Synthetic AX132 heavy chain CDR2 antibody region peptide
<400> 55
   Trp Ile Gly Trp Ile Asp Pro Gly Ser Gly Gly Thr Lys Tyr Asn Glu
   1               5                   10                  15
   Lys Phe Lys Gly Lys Ala Thr
               20
<210> 56
   <211> 15
   <212> PRT
   <213> Artificial Sequence
<220>
   <223> Description of Artificial Sequence: Synthetic AX132 heavy chain CDR3 antibody region peptide
<400> 56
   Cys Ala Arg Glu Arg Tyr Gly Tyr Tyr Phe Asp Tyr Trp Gly Gln
   1               5                   10                  15
<210> 57
   <211> 108
   <212> PRT
   <213> Artificial Sequence
<220>
   <223> Description of Artificial Sequence: Synthetic variable light antibody region polypeptide
<400> 57
   Glu Ile Val Leu Thr Gln Ser Pro Ala Thr Leu Ser Leu Ser Pro Gly
   1               5                   10                  15
   Glu Arg Ala Thr Ile Thr Cys Arg Ala Ser Gln Tyr Val Gly Ser Tyr
               20                  25                  30
   Leu Asn Trp Tyr Gln Gln Lys Pro Gly Gln Ala Pro Arg Leu Leu Ile
           35                  40                  45
   Tyr Asp Ala Ser Asn Arg Ala Thr Gly Ile Pro Ala Arg Phe Ser Gly
   50 55 60
   Ser Gly Ser Gly Thr Asp Phe Thr Leu Thr Ile Ser Ser Leu Glu Pro
   65 70 75 80
   Glu Asp Phe Ala Val Tyr Tyr Cys Gln Val Trp Asp Ser Ser Pro Pro
   85 90 95
   Val Val Phe Gly Gly Gly Thr Lys Val Glu Ile Lys
   100 105
<210> 58
   <211> 17
   <212> PRT
   <213> Artificial Sequence
<220>
   <223> Description of Artificial Sequence: Synthetic AX213 and AX132 light chain CDR1 antibody region peptide
<400> 58
   Ile Thr Cys Arg Ala Ser Gln Tyr Val Gly Ser Tyr Leu Asn Trp Tyr
   1 5 10 15
   Gln
<210> 59
   <211> 13
   <212> PRT
   <213> Artificial Sequence
<220>
   <223> Description of Artificial Sequence: Synthetic AX213 and AX132 light chain CDR2 antibody region peptide
<400> 59
   Leu Ile Tyr Asp Ala Ser Asn Arg Ala Thr Gly Ile Pro
   1 5 10
<210> 60
   <211> 16
   <212> PRT
   <213> Artificial Sequence
<220>
   <223> Description of Artificial Sequence: Synthetic AX132 & AX213 light chain CDR3 antibody region peptide
<400> 60
   Tyr Tyr Cys Gln Val Trp Asp Ser Ser Pro Pro Val Val Phe Gly Gly
   1 5 10 15
<210> 61
   <211> 120
   <212> PRT
   <213> Artificial Sequence
<220>
   <223> Description of Artificial Sequence: Synthetic variable heavy antibody region polypeptide
<400> 61
   Glu Val Gln Leu Leu Glu Ser Gly Gly Gly Leu Val Gln Pro Gly Gly
   1               5                   10                  15
   Ser Leu Arg Leu Ser Cys Lys Ala Ser Gly Tyr Thr Phe Ser Arg Tyr
               20                  25                  30
   Gly Ile Asn Trp Val Arg Gln Ala Pro Gly Lys Gly Leu Glu Trp Ile
           35                  40                  45
   Gly Arg Ile Asp Pro Gly Asn Gly Gly Thr Arg Tyr Asn Glu Lys Phe
       50                  55                  60
   Lys Gly Lys Ala Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Ala Asn Asp Gly Tyr Ser Phe Asp Tyr Trp Gly Gln Gly Thr
               100                 105                 110
   Leu Val Thr Val Ser Ser Ala Ser
           115                 120
<210> 62
   <211> 16
   <212> PRT
   <213> Artificial Sequence
<220>
   <223> Description of Artificial Sequence: Synthetic AX213 heavy chain CDR1 antibody region peptide
<400> 62
   Lys Ala Ser Gly Tyr Thr Phe Ser Arg Tyr Gly Ile Asn Trp Val Arg
   1               5                   10                  15
<210> 63
   <211> 23
   <212> PRT
   <213> Artificial Sequence
<220>
   <223> Description of Artificial Sequence: Synthetic AX213 heavy chain CDR2 antibody region peptide
<400> 63
   Trp Ile Gly Arg Ile Asp Pro Gly Asn Gly Gly Thr Arg Tyr Asn Glu
   1               5                   10                  15
   Lys Phe Lys Gly Lys Ala Thr
               20
<210> 64
   <211> 15
   <212> PRT
   <213> Artificial Sequence
<220>
   <223> Description of Artificial Sequence: Synthetic AX213 heavy chain CDR3 antibody region peptide
<400> 64
   Cys Ala Arg Ala Asn Asp Gly Tyr Ser Phe Asp Tyr Trp Gly Gln
   1               5                   10                  15
<210> 65
   <211> 108
   <212> PRT
   <213> Artificial Sequence
<220>
   <223> Description of Artificial Sequence: Synthetic variable light antibody region polypeptide
<400> 65
   Glu Ile Val Leu Thr Gln Ser Pro Ala Thr Leu Ser Leu Ser Pro Gly
   1               5                   10                  15
   Glu Arg Ala Thr Ile Thr Cys Arg Ala Ser Gln Tyr Val Gly Ser Tyr
               20                  25                  30
   Leu Asn Trp Tyr Gln Gln Lys Pro Gly Gln Ala Pro Arg Leu Leu Ile
           35                  40                  45
   Tyr Asp Ala Ser Asn Arg Ala Thr Gly Ile Pro Ala Arg Phe Ser Gly
       50                  55                  60
   Ser Gly Ser Gly Thr Asp Phe Thr Leu Thr Ile Ser Ser Leu Glu Pro
   65                  70                  75                  80
   Glu Asp Phe Ala Val Tyr Tyr Cys Gln Val Trp Asp Ser Ser Pro Pro
                   85                  90                  95
   Val Val Phe Gly Gly Gly Thr Lys Val Glu Ile Lys
               100                 105
<210> 66
   <211> 17
   <212> PRT
   <213> Artificial Sequence
<220>
   <223> Description of Artificial Sequence: Synthetic AX213 and AX132 light chain CDR1 antibody region peptide
<400> 66
   Ile Thr Cys Arg Ala Ser Gln Tyr Val Gly Ser Tyr Leu Asn Trp Tyr
   1               5                   10                  15
   Gln
<210> 67
   <211> 13
   <212> PRT
   <213> Artificial Sequence
<220>
   <223> Description of Artificial Sequence: Synthetic AX213 and AX132 light chain CDR2 antibody region peptide
<400> 67
   Leu Ile Tyr Asp Ala Ser Asn Arg Ala Thr Gly Ile Pro
   1               5                   10
<210> 68
   <211> 16
   <212> PRT
   <213> Artificial Sequence
<220>
   <223> Description of Artificial Sequence: Synthetic AX132 & AX213 light chain CDR3 antibody region peptide
<400> 68
   Tyr Tyr Cys Gln Val Trp Asp Ser Ser Pro Pro Val Val Phe Gly Gly
   1               5                   10                  15
<210> 69
   <211> 119
   <212> PRT
   <213> Artificial Sequence
<220>
   <223> Description of Artificial Sequence: Synthetic AX1 VH antibody sequence polypeptide
<400> 69
   Glu Val Gln Leu Leu Glu Ser Gly Gly Gly Leu Val Gln Pro Gly Gly
   1               5                   10                  15
   Ser Leu Arg Leu Ser Cys Lys Ala Ser Gly Phe Thr Phe Thr Ser Tyr
               20                  25                  30
   Tyr Met His Trp Val Arg Gln Ala Pro Gly Lys Gly Leu Glu Trp Ile
           35                  40                  45
   Gly Arg Ile Asn Pro Asp Ser Gly Ser Thr Lys Tyr Asn Glu Lys Phe
       50                  55                  60
   Lys Gly Arg Ala Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Gly Gly Arg Leu Ser Trp Asp Phe Asp Val Trp Gly Gln Gly
               100                 105                 110
   Thr Leu Val Thr Val Ser Ser
           115
<210> 70
   <211> 16
   <212> PRT
   <213> Artificial Sequence
<220>
   <223> Description of Artificial Sequence: Synthetic AX1 VH CDR1 antibody sequence peptide
<400> 70
   Lys Ala Ser Gly Phe Thr Phe Thr Ser Tyr Tyr Met His Trp Val Arg
   1 5 10 15
<210> 71
   <211> 23
   <212> PRT
   <213> Artificial Sequence
<220>
   <223> Description of Artificial Sequence: Synthetic AX1 VH CDR2 antibody sequence peptide
<400> 71
   Trp Ile Gly Arg Ile Asn Pro Asp Ser Gly Ser Thr Lys Tyr Asn Glu
   1 5 10 15
   Lys Phe Lys Gly Arg Ala Thr
   20
<210> 72
   <211> 16
   <212> PRT
   <213> Artificial Sequence
<220>
   <223> Description of Artificial Sequence: Synthetic AX1 VH CDR3 antibody sequence peptide
<400> 72
   Cys Ala Arg Gly Gly Arg Leu Ser Trp Asp Phe Asp Val Trp Gly Gln
   1 5 10 15
<210> 73
   <211> 109
   <212> PRT
   <213> Artificial Sequence
<220>
   <223> Description of Artificial Sequence: Synthetic AX1 VL antibody sequence polypeptide
<400> 73
   Asp Ile Gln Met Thr Gln Ser Pro Ser Ser Leu Ser Ala Ser Val Gly
   1 5 10 15
   Asp Arg Val Thr Ile Thr Cys Arg Ala Ser Gln Asp Ile Ser Arg Tyr
   20 25 30
   Leu Ala Trp Tyr Gln Gln Lys Pro Gly Lys Ala Pro Lys Leu Leu Ile
   35 40 45
   Tyr Ala Ala Ser Ser Leu Gln Ser Gly Val Pro Ser Arg Phe Ser Gly
   50 55 60
   Ser Gly Ser Gly Thr Asp Phe Thr Leu Thr Ile Ser Ser Leu Gln Pro
   65 70 75 80
   Glu Asp Phe Ala Thr Tyr Tyr Cys Ala Ala Tyr Asp Tyr Ser Leu Gly
   85 90 95
   Gly Tyr Val Phe Gly Asp Gly Thr Lys Val Glu Ile Lys
   100 105
<210> 74
   <211> 11
   <212> PRT
   <213> Artificial Sequence
<220>
   <223> Description of Artificial Sequence: Synthetic AX1 VL CDR1 antibody sequence peptide
<400> 74
   Arg Ala Ser Gln Asp Ile Ser Arg Tyr Leu Ala
   1 5 10
<210> 75
   <211> 7
   <212> PRT
   <213> Artificial Sequence
<220>
   <223> Description of Artificial Sequence: Synthetic AX1 AX9 AX189 VL CDR2 antibody sequence peptide
<400> 75
   Ala Ala Ser Ser Leu Gln Ser
   1 5
<210> 76
   <211> 11
   <212> PRT
   <213> Artificial Sequence
<220>
   <223> Description of Artificial Sequence: Synthetic AX1 VL CDR3 antibody sequence peptide
<400> 76
   Ala Ala Tyr Asp Tyr Ser Leu Gly Gly Tyr Val
   1               5                   10
<210> 77
   <211> 121
   <212> PRT
   <213> Artificial Sequence
<220>
   <223> Description of Artificial Sequence: Synthetic AX9 AX189 VH antibody sequence polypeptide
<400> 77
   Glu Val Gln Leu Leu Glu Ser Gly Gly Gly Leu Val Gln Pro Gly Gly
   1               5                   10                  15
   Ser Leu Arg Leu Ser Cys Lys Ala Ser Gly Tyr Thr Phe Ser Ser Tyr
               20                  25                  30
   Trp Met His Trp Val Arg Gln Ala Pro Gly Lys Gly Leu Glu Trp Ile
           35                  40                  45
   Gly Arg Ile Asp Pro Tyr Asn Gly Gly Thr Lys Tyr Asn Glu Lys Phe
       50                  55                  60
   Lys Gly Lys Ala Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Tyr Gly Tyr Tyr Leu Gly Ser Tyr Ala Met Asp Tyr Trp Gly
               100                 105                 110
   Gln Gly Thr Leu Val Thr Val Ser Ser
                       115                 120
<210> 78
   <211> 16
   <212> PRT
   <213> Artificial Sequence
<220>
   <223> Description of Artificial Sequence: Synthetic AX9 AX189 VH CDR1 antibody sequence peptide
<400> 78
   Lys Ala Ser Gly Tyr Thr Phe Ser Ser Tyr Trp Met His Trp Val Arg
   1 5 10 15
<210> 79
   <211> 23
   <212> PRT
   <213> Artificial Sequence
<220>
   <223> Description of Artificial Sequence: Synthetic AX9 AX189 VH CDR2 antibody sequence peptide
<400> 79
   Trp Ile Gly Arg Ile Asp Pro Tyr Asn Gly Gly Thr Lys Tyr Asn Glu
   1 5 10 15
   Lys Phe Lys Gly Lys Ala Thr
   20
<210> 80
   <211> 18
   <212> PRT
   <213> Artificial Sequence
<220>
   <223> Description of Artificial Sequence: Synthetic AX9 AX189 VH CDR3 antibody sequence peptide
<400> 80
   Cys Ala Arg Tyr Gly Tyr Tyr Leu Gly Ser Tyr Ala Met Asp Tyr Trp
   1 5 10 15
   Gly Gln
<210> 81
   <211> 109
   <212> PRT
   <213> Artificial Sequence
<220>
   <223> Description of Artificial Sequence: Synthetic AX189 VL antibody sequence polypeptide
<400> 81
   Asp Ile Gln Met Thr Gln Ser Pro Ser Ser Leu Ser Ala Ser Val Gly
   1 5 10 15
   Asp Arg Val Thr Ile Thr Cys Arg Ala Ser Gln Asp Val Ser Arg Tyr
   20 25 30
   Leu Thr Trp Tyr Gln Gln Lys Pro Gly Lys Ala Pro Lys Leu Leu Ile
   35 40 45
   Tyr Ala Ala Ser Ser Leu Gln Ser Gly Val Pro Ser Arg Phe Ser Gly
   50 55 60
   Ser Gly Ser Gly Thr Asp Phe Thr Leu Thr Ile Ser Ser Leu Gln Pro
   65 70 75 80
   Glu Asp Phe Ala Thr Tyr Tyr Cys Gln Ala Tyr Asp Tyr Ser Leu Ser
   85 90 95
   Gly Tyr Val Phe Gly Gly Gly Thr Lys Val Glu Ile Lys
   100 105
<210> 82
   <211> 11
   <212> PRT
   <213> Artificial Sequence
<220>
   <223> Description of Artificial Sequence: Synthetic AX189 VL CDR1 antibody sequence peptide
<400> 82
   Arg Ala Ser Gln Asp Val Ser Arg Tyr Leu Thr
   1 5 10
<210> 83
   <211> 7
   <212> PRT
   <213> Artificial Sequence
<220>
   <223> Description of Artificial Sequence: Synthetic AX1 AX9 AX189 VL CDR2 antibody sequence peptide
<400> 83
   Ala Ala Ser Ser Leu Gln Ser
   1 5
<210> 84
   <211> 11
   <212> PRT
   <213> Artificial Sequence
<220>
   <223> Description of Artificial Sequence: Synthetic AX189 VL CDR3 antibody sequence peptide
<400> 84
   Gln Ala Tyr Asp Tyr Ser Leu Ser Gly Tyr Val
   1               5                   10
<210> 85
   <211> 115
   <212> PRT
   <213> Homo sapiens
<400> 85
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Leu Thr Ser Tyr
               20                  25                  30
   Gly Ile Ser Trp Val Arg Gln Ala Pro Gly Gln Gly Leu Glu Trp Met
           35                  40                  45
   Gly Trp Val Ser Phe Tyr Asn Gly Asn Thr Asn Tyr Ala Gln Lys Leu
       50                  55                  60
   Gln Gly Arg Gly Thr Met Thr Thr Asp Pro Ser Thr Ser Thr Ala Tyr
   65                  70                  75                  80
   Met Glu Leu Arg Ser Leu Arg Ser Asp Asp Thr Ala Val Tyr Tyr Cys
                   85                  90                  95
   Ala Arg Gly Tyr Gly Met Asp Val Trp Gly Gln Gly Thr Thr Val Thr
               100                 105                 110
   Val Ser Ser
           115
<210> 86
   <211> 5
   <212> PRT
   <213> Homo sapiens
<400> 86
   Ser Tyr Gly Ile Ser
   1               5
<210> 87
   <211> 17
   <212> PRT
   <213> Homo sapiens
<400> 87
   Trp Val Ser Phe Tyr Asn Gly Asn Thr Asn Tyr Ala Gln Lys Leu Gln
   1               5                   10                  15
   Gly
<210> 88
   <211> 6
   <212> PRT
   <213> Homo sapiens
<400> 88
   Gly Tyr Gly Met Asp Val
   1               5
<210> 89
   <211> 109
   <212> PRT
   <213> Homo sapiens
<400> 89
   Gln Ser Ala Leu Thr Gln Pro Ala Ser Val Ser Gly Ser Pro Gly Gln
   1               5                   10                  15
   Ser Ile Thr Ile Ser Cys Thr Gly Thr Ser Ser Asp Val Gly Gly Tyr
               20                  25                  30
   Asn Ser Val Ser Trp Tyr Gln Gln His Pro Gly Lys Ala Pro Lys Leu
           35                  40                  45
   Met Ile Tyr Glu Val Ser Asn Arg Pro Ser Gly Val Ser Asn Arg Phe
       50                  55                  60
   Ser Gly Ser Lys Ser Gly Asn Thr Ala Ser Leu Thr Ile Ser Gly Leu
   65                  70                  75                  80
   Gln Ala Glu Asp Glu Ala Asp Tyr Tyr Cys Asn Ser Tyr Thr Ser Thr
                   85                  90                  95
   Ser Met Val Phe Gly Gly Gly Thr Lys Leu Thr Val Leu
               100                 105
<210> 90
   <211> 14
   <212> PRT
   <213> Homo sapiens
<400> 90
   Thr Gly Thr Ser Ser Asp Val Gly Gly Tyr Asn Ser Val Ser
   1 5 10
<210> 91
   <211> 7
   <212> PRT
   <213> Homo sapiens
<400> 91
   Glu Val Ser Asn Arg Pro Ser
   1 5
<210> 92
   <211> 9
   <212> PRT
   <213> Homo sapiens
<400> 92
   Asn Ser Tyr Thr Ser Thr Ser Met Val
   1 5
<210> 93
   <211> 123
   <212> PRT
   <213> Homo sapiens
<400> 93
   Glu Val Gln Leu Val Glu Ser Gly Gly Gly Leu Val Lys Pro Gly Gly
   1 5 10 15
   Ser Leu Arg Leu Ser Cys Ala Ala Ser Gly Phe Thr Phe Ser Ser Tyr
   20 25 30
   Ser Met Asn Trp Val Arg Gln Ala Pro Gly Lys Gly Leu Glu Trp Val
   35 40 45
   Ser Ser Ile Ser Ser Ser Ser Ser Tyr Ile Ser Tyr Ala Asp Ser Val
   50 55 60
   Lys Gly Arg Phe Thr Ile Ser Arg Asp Asn Ala Lys Asn Ser Leu Tyr
   65 70 75 80
   Leu Gln Met Asn Ser Leu Arg Ala Glu Asp Thr Ala Val Tyr Phe Cys
   85 90 95
   Ala Arg Asp Tyr Asp Phe Trp Ser Ala Tyr Tyr Asp Ala Phe Asp Val
   100 105 110
   Trp Gly Gln Gly Thr Met Val Thr Val Ser Ser
   115 120
<210> 94
   <211> 10
   <212> PRT
   <213> Homo sapiens
<400> 94
   Gly Phe Thr Phe Ser Ser Tyr Ser Met Asn
   1 5 10
<210> 95
   <211> 17
   <212> PRT
   <213> Homo sapiens
<400> 95
   Ser Ile Ser Ser Ser Ser Ser Tyr Ile Ser Tyr Ala Asp Ser Val Lys
   1 5 10 15
   Gly
<210> 96
   <211> 14
   <212> PRT
   <213> Homo sapiens
<400> 96
   Asp Tyr Asp Phe Trp Ser Ala Tyr Tyr Asp Ala Phe Asp Val
   1               5                   10
<210> 97
   <211> 111
   <212> PRT
   <213> Homo sapiens
<400> 97
   Gln Ser Val Leu Thr Gln Pro Pro Ser Val Ser Gly Ala Pro Gly Gln
   1               5                   10                  15
   Arg Val Thr Ile Ser Cys Thr Gly Ser Ser Ser Asn Ile Gly Ala Gly
               20                  25                  30
   Tyr Asp Val His Trp Tyr Gln Gln Leu Pro Gly Thr Ala Pro Lys Leu
           35                  40                  45
   Leu Ile Ser Gly Asn Ser Asn Arg Pro Ser Gly Val Pro Asp Arg Phe
       50                  55                  60
   Ser Gly Ser Lys Ser Gly Thr Ser Ala Ser Leu Ala Ile Thr Gly Leu
   65                  70                  75                  80
   Gln Ala Glu Asp Glu Ala Asp Tyr Tyr Cys Gln Ser Tyr Asp Ser Ser
                   85                  90                  95
   Leu Ser Gly Ser Val Phe Gly Gly Gly Thr Lys Leu Thr Val Leu
               100                 105                 110
<210> 98
   <211> 14
   <212> PRT
   <213> Homo sapiens
<400> 98
   Thr Gly Ser Ser Ser Asn Ile Gly Ala Gly Tyr Asp Val His
   1               5                   10
<210> 99
   <211> 7
   <212> PRT
   <213> Homo sapiens
<400> 99
   Gly Asn Ser Asn Arg Pro Ser
   1               5
<210> 100
   <211> 11
   <212> PRT
   <213> Homo sapiens
<400> 100
   Gln Ser Tyr Asp Ser Ser Leu Ser Gly Ser Val
   1 5 10
<210> 101
   <211> 114
   <212> PRT
   <213> Homo sapiens
<400> 101
   Gln Val Gln Leu Val Glu Ser Gly Gly Gly Val Ala Gln Pro Gly Arg
   1 5 10 15
   Ser Leu Arg Leu Ser Cys Ala Ala Ser Gly Phe Thr Phe Ser Ser Tyr
   20 25 30
   Gly Met His Trp Val Arg Gln Ala Pro Gly Lys Gly Leu Glu Trp Val
   35 40 45
   Ala Val Ile Tyr Tyr Asp Gly Ile Asn Lys His Tyr Ala Asp Ser Val
   50 55 60
   Lys Gly Arg Phe Thr Ile Ser Arg Asp Asn Ser Lys Asn Thr Leu Tyr
   65 70 75 80
   Leu Gln Met Asn Ser Leu Arg Ala Glu Asp Thr Ala Val Tyr Tyr Cys
   85 90 95
   Ala Arg Asp Arg Gly Leu Asp Trp Gly Gln Gly Thr Leu Val Thr Val
   100 105 110
   Ser Ser
<210> 102
   <211> 10
   <212> PRT
   <213> Homo sapiens
<400> 102
   Gly Phe Thr Phe Ser Ser Tyr Gly Met His
   1 5 10
<210> 103
   <211> 17
   <212> PRT
   <213> Homo sapiens
<400> 103
   Val Ile Tyr Tyr Asp Gly Ile Asn Lys His Tyr Ala Asp Ser Val Lys
   1               5                   10                  15
   Gly
<210> 104
   <211> 5
   <212> PRT
   <213> Homo sapiens
<400> 104
   Asp Arg Gly Leu Asp
   1               5
<210> 105
   <211> 113
   <212> PRT
   <213> Homo sapiens
<400> 105
   Asp Ile Val Met Thr Gln Ser Pro Asp Ser Leu Ala Val Ser Leu Gly
   1               5                   10                  15
   Glu Arg Ala Thr Ile Asn Cys Lys Ser Ser Gln Ser Val Leu Tyr Ser
               20                  25                  30
   Ser Asn Ser Lys Asn Tyr Leu Val Trp Tyr Gln Gln Lys Pro Gly Gln
           35                  40                  45
   Pro Pro Lys Leu Leu Ile Tyr Trp Ala Ser Thr Arg Glu Ser Gly Val
       50                  55                  60
   Pro Asp Arg Phe Ser Gly Ser Gly Ser Gly Thr Asp Phe Thr Leu Thr
   65                  70                  75                  80
   Ile Ser Ser Leu Gln Ala Glu Asp Val Ala Val Tyr Tyr Cys Gln Gln
                   85                  90                  95
   Tyr Tyr Ser Thr Pro Trp Thr Phe Gly Gln Gly Thr Lys Val Glu Ile
               100                 105                 110
   Lys
<210> 106
   <211> 17
   <212> PRT
   <213> Homo sapiens
<400> 106
   Lys Ser Ser Gln Ser Val Leu Tyr Ser Ser Asn Ser Lys Asn Tyr Leu
   1               5                   10                  15
   Val
<210> 107
   <211> 7
   <212> PRT
   <213> Homo sapiens
<400> 107
   Trp Ala Ser Thr Arg Glu Ser
   1               5
<210> 108
   <211> 9
   <212> PRT
   <213> Homo sapiens
<400> 108
   Gln Gln Tyr Tyr Ser Thr Pro Trp Thr
   1               5
<210> 109
   <211> 118
   <212> PRT
   <213> Artificial Sequence
<220>
   <223> Description of Artificial Sequence: Synthetic anti-PCSK9 monoclonal antibody pJG04 (clones LGT-209 and LGT-210) Vh heavy chain variable region (FR1-FR4) polypeptide
<400> 109
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Ser Thr Met
               20                  25                  30
   Tyr Met Ser Trp Val Arg Gln Ala Pro Gly Gln Gly Leu Glu Trp Met
   35 40 45
   Gly Arg Ile Asp Pro Ala Asn Glu His Thr Asn Tyr Ala Gln Lys Phe
   50 55 60
   Gln Gly Arg Val Thr Met Thr Arg Asp Thr Ser Ile Ser Thr Ala Tyr
   65 70 75 80
   Met Glu Leu Ser Arg Leu Thr Ser Asp Asp Thr Ala Val Tyr Tyr Cys
   85 90 95
   Ala Arg Ser Tyr Tyr Tyr Tyr Asn Met Asp Tyr Trp Gly Gln Gly Thr
   100 105 110
   Leu Val Thr Val Ser Ser
   115
<210> 110
   <211> 5
   <212> PRT
   <213> Artificial Sequence
<220>
   <223> Description of Artificial Sequence: Synthetic anti-PCSK9 monoclonal antibody clones LGT-209, LGT-210 and LGT-211 heavy chain CDR1 peptide
<400> 110
   Thr Met Tyr Met Ser
   1 5
<210> 111
   <211> 17
   <212> PRT
   <213> Artificial Sequence
<220>
   <223> Description of Artificial Sequence: Synthetic anti-PCSK9 monoclonal antibody clones LGT-209, LGT-210 and LGT-211 heavy chain CDR2 peptide
<400> 111
   Arg Ile Asp Pro Ala Asn Glu His Thr Asn Tyr Ala Gln Lys Phe Gln
   1 5 10 15
   Gly
<210> 112
   <211> 9
   <212> PRT
   <213> Artificial Sequence
<220>
   <223> Description of Artificial Sequence: Synthetic anti-PCSK9 monoclonal antibody pJG04(clones LGT-209 and LGT-210) Vh heavy chain complementarity determining region 3 (CDR3) peptide
<400> 112
   Ser Tyr Tyr Tyr Tyr Asn Met Asp Tyr
   1               5
<210> 113
   <211> 106
   <212> PRT
   <213> Artificial Sequence
<220>
   <223> Description of Artificial Sequence: Synthetic anti-PCSK9 monoclonal antibody pJG10(clones LGT-209 and LGT-211) Vk light chain variable region (FR1-FR4) polypeptide
<400> 113
   Gln Ile Val Leu Thr Gln Ser Pro Ala Thr Leu Ser Val Ser Pro Gly
   1               5                   10                  15
   Glu Arg Ala Thr Leu Ser Cys Arg Ala Ser Gln Ser Val Ser Tyr Met
               20                  25                  30
   His Trp Tyr Gln Gln Lys Pro Gly Gln Ala Pro Arg Leu Leu Ile Tyr
           35                  40                  45
   Gly Val Phe Arg Arg Ala Thr Gly Ile Pro Asp Arg Phe Ser Gly Ser
       50                  55                  60
   Gly Ser Gly Thr Asp Phe Thr Leu Thr Ile Gly Arg Leu Glu Pro Glu
   65                  70                  75                  80
   Asp Phe Ala Val Tyr Tyr Cys Leu Gln Trp Ser Ser Asp Pro Pro Thr
                   85                  90                  95
   Phe Gly Gln Gly Thr Lys Leu Glu Ile Lys
               100                 105
<210> 114
   <211> 10
   <212> PRT
   <213> Artificial Sequence
<220>
   <223> Description of Artificial Sequence: Synthetic anti-PCSK9 monoclonal antibody clones LGT-209, LGT-210 and LGT-211 light chain CDR1 peptide
<400> 114
   Arg Ala Ser Gln Ser Val Ser Tyr Met His
   1 5 10
<210> 115
   <211> 7
   <212> PRT
   <213> Artificial Sequence
<220>
   <223> Description of Artificial Sequence: Synthetic anti-PCSK9 monoclonal antibody clones LGT-209, LGT-210 and LGT-211 light chain CDR1 peptide
<400> 115
   Gly Val Phe Arg Arg Ala Thr
   1 5
<210> 116
   <211> 9
   <212> PRT
   <213> Artificial Sequence
<220>
   <223> Description of Artificial Sequence: Synthetic mouse anti-PCSK9 monoclonal antibody LFU720 and anti-PCSK9 monoclonal antibody clones LGT-209, LGT-210 and LGT-211 light chain CDR3 peptide
<400> 116
   Leu Gln Trp Ser Ser Asp Pro Pro Thr
   1 5
<210> 117
   <211> 118
   <212> PRT
   <213> Homo sapiens
<400> 117
   Gln Val Gln Leu Val Gln Ser Gly Ala Glu Val Lys Lys Pro Gly Ala
   1 5 10 15
   Ser Val Lys Val Ser Cys Lys Ala Ser Gly Tyr Thr Phe Thr Ser Tyr
   20 25 30
   Tyr Met His Trp Val Arg Gln Ala Pro Gly Gln Gly Leu Glu Trp Met
   35 40 45
   Gly Glu Ile Ser Pro Phe Gly Gly Arg Thr Asn Tyr Asn Glu Lys Phe
   50 55 60
   Lys Ser Arg Val Thr Met Thr Arg Asp Thr Ser Thr Ser Thr Val Tyr
   65 70 75 80
   Met Glu Leu Ser Ser Leu Arg Ser Glu Asp Thr Ala Val Tyr Tyr Cys
   85 90 95
   Ala Arg Glu Arg Pro Leu Tyr Ala Ser Asp Leu Trp Gly Gln Gly Thr
   100 105 110
   Thr Val Thr Val Ser Ser
   115
<210> 118
   <211> 7
   <212> PRT
   <213> Artificial Sequence
<220>
   <223> Description of Artificial Sequence: Synthetic peptide
<400> 118
   Gly Tyr Thr Phe Thr Ser Tyr
   1 5
<210> 119
   <211> 6
   <212> PRT
   <213> Artificial Sequence
<220>
   <223> Description of Artificial Sequence: Synthetic peptide
<400> 119
   Ser Pro Phe Gly Gly Arg
   1 5
<210> 120
   <211> 9
   <212> PRT
   <213> Artificial Sequence
<220>
   <223> Description of Artificial Sequence: Synthetic variable heavy chain CDR peptide
<400> 120
   Glu Arg Pro Leu Tyr Ala Ser Asp Leu
   1               5
<210> 121
   <211> 107
   <212> PRT
   <213> Homo sapiens
<400> 121
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Gly Ile Ser Ser Ala
               20                  25                  30
   Leu Ala Trp Tyr Gln Gln Lys Pro Gly Lys Ala Pro Lys Leu Leu Ile
           35                  40                  45
   Tyr Ser Ala Ser Tyr Arg Tyr Thr Gly Val Pro Ser Arg Phe Ser Gly
       50                  55                  60
   Ser Gly Ser Gly Thr Asp Phe Thr Phe Thr Ile Ser Ser Leu Gln Pro
   65                  70                  75                  80
   Glu Asp Ile Ala Thr Tyr Tyr Cys Gln Gln Arg Tyr Ser Leu Trp Arg
                   85                  90                  95
   Thr Phe Gly Gln Gly Thr Lys Leu Glu Ile Lys
               100                 105
<210> 122
   <211> 11
   <212> PRT
   <213> Artificial Sequence
<220>
   <223> Description of Artificial Sequence: Synthetic variable light chain CDR peptide
<400> 122
   Arg Ala Ser Gln Gly Ile Ser Ser Ala Leu Ala
   1               5                   10
<210> 123
   <211> 7
   <212> PRT
   <213> Artificial Sequence
<220>
   <223> Description of Artificial Sequence: Synthetic variable light chain CDR peptide
<400> 123
   Ser Ala Ser Tyr Arg Tyr Thr
   1 5
<210> 124
   <211> 9
   <212> PRT
   <213> Artificial Sequence
<220>
   <223> Description of Artificial Sequence: Synthetic variable light chain CDR peptide
<400> 124
   Gln Gln Arg Tyr Ser Leu Trp Arg Thr
   1 5
<210> 125
   <211> 118
   <212> PRT
   <213> Homo sapiens
<400> 125
   Gln Val Gln Leu Val Gln Ser Gly Ala Glu Val Lys Lys Pro Gly Ala
   1               5                   10                  15
   Ser Val Lys Val Ser Cys Lys Ala Ser Gly Tyr Thr Phe Thr Ser Tyr
               20                  25                  30
   Tyr Met His Trp Val Arg Gln Ala Pro Gly Gln Gly Leu Glu Trp Met
           35                  40                  45
   Gly Glu Ile His Pro Ser Gly Gly Arg Thr Asn Tyr Asn Glu Lys Phe
       50                  55                  60
   Lys Ser Arg Val Thr Met Thr Arg Asp Thr Ser Thr Ser Thr Val Tyr
   65                  70                  75                  80
   Met Glu Leu Ser Ser Leu Arg Ser Glu Asp Thr Ala Val Tyr Tyr Cys
                   85                  90                  95
   Ala Arg Glu Arg Pro Leu Tyr Ala Met Asp Tyr Trp Gly Gln Gly Thr
               100                 105                 110
   Thr Val Thr Val Ser Ser
           115
<210> 126
   <211> 10
   <212> PRT
   <213> Homo sapiens
<400> 126
   Gly Tyr Thr Phe Thr Ser Tyr Tyr Met His
   1               5                   10
<210> 127
   <211> 17
   <212> PRT
   <213> Homo sapiens
<400> 127
   Glu Ile His Pro Ser Gly Gly Arg Thr Asn Tyr Asn Glu Lys Phe Lys
   1               5                   10                  15
   Ser
<210> 128
   <211> 9
   <212> PRT
   <213> Homo sapiens
<400> 128
   Glu Arg Pro Leu Tyr Ala Met Asp Tyr
   1 5
<210> 129
   <211> 107
   <212> PRT
   <213> Homo sapiens
<400> 129
<210> 130
   <211> 11
   <212> PRT
   <213> Homo sapiens
<400> 130
   Lys Ala Ser Gln Asp Val His Thr Ala Val Ala
   1 5 10
<210> 131
   <211> 7
   <212> PRT
   <213> Homo sapiens
<400> 131
   His Ala Ser Tyr Arg Tyr Thr
   1 5
<210> 132
   <211> 9
   <212> PRT
   <213> Homo sapiens
<400> 132
   Gln Gln Arg Tyr Ser Leu Trp Arg Thr
   1               5
<210> 133
   <211> 118
   <212> PRT
   <213> Homo sapiens
<400> 133
<210> 134
   <211> 10
   <212> PRT
   <213> Homo sapiens
<400> 134
   Gly Tyr Thr Phe Thr Ser Tyr Tyr Met His
   1 5 10
<210> 135
   <211> 17
   <212> PRT
   <213> Homo sapiens
<400> 135
   Glu Ile His Pro Ser Gly Gly Arg Thr Asn Tyr Asn Glu Lys Phe Lys
   1 5 10 15
   Ser
<210> 136
   <211> 9
   <212> PRT
   <213> Homo sapiens
<400> 136
   Glu Arg Pro Leu Tyr Ala Ser Asp Leu
   1 5
<210> 137
   <211> 107
   <212> PRT
   <213> Homo sapiens
<400> 137
<210> 138
   <211> 11
   <212> PRT
   <213> Homo sapiens
<400> 138
   Lys Ala Ser Gln Asp Val His Thr Ala Val Ala
   1 5 10
<210> 139
   <211> 7
   <212> PRT
   <213> Homo sapiens
<400> 139
   His Ala Ser Tyr Arg Tyr Thr
   1 5
<210> 140
   <211> 9
   <212> PRT
   <213> Homo sapiens
<400> 140
   Gln Gln Arg Tyr Ser Leu Trp Arg Thr
   1 5
<210> 141
   <211> 118
   <212> PRT
   <213> Mus musculus
<400> 141
   Gln Val Gln Leu Gln Gln Pro Gly Ala Glu Leu Val Lys Pro Gly Ala
   1 5 10 15
   Ser Val Lys Leu Ser Cys Lys Ala Ser Gly Tyr Thr Phe Thr Ser Tyr
   20 25 30
   Trp Met His Trp Val Lys Gln Arg Pro Gly Gln Gly Leu Glu Trp Ile
   35 40 45
   Gly Glu Ile Asn Pro Ser Asn Gly Arg Thr Asn Tyr Asn Glu Lys Phe
   50 55 60
   Lys Ser Lys Ala Thr Leu Thr Val Asp Lys Ser Ser Ser Thr Ala Tyr
   65 70 75 80
   Met Gln Leu Ser Ser Leu Thr Ser Glu Asp Ser Ala Val Tyr Tyr Cys
   85 90 95
   Ala Arg Glu Arg Pro Leu Tyr Ala Met Asp Tyr Trp Gly Gln Gly Thr
   100 105 110
   Ser Val Thr Val Ser Ser
   115
<210> 142
   <211> 7
   <212> PRT
   <213> Mus musculus
<400> 142
   Gly Tyr Thr Phe Thr Ser Tyr
   1 5
<210> 143
   <211> 6
   <212> PRT
   <213> Mus musculus
<400> 143
   Asn Pro Ser Asn Gly Arg
   1 5
<210> 144
   <211> 9
   <212> PRT
   <213> Mus musculus
<400> 144
   Glu Arg Pro Leu Tyr Ala Met Asp Tyr
   1 5
<210> 145
   <211> 108
   <212> PRT
   <213> Mus musculus
<400> 145
   Asp Ile Val Met Thr Gln Ser His Lys Phe Met Ser Thr Ser Val Gly
   1 5 10 15
   Asp Arg Val Ser Ile Thr Cys Lys Ala Ser Gln Asp Val Ser Thr Ala
   20 25 30
   Val Ala Trp Tyr Gln Gln Lys Pro Gly Gln Ser Pro Lys Leu Leu Ile
   35 40 45
   Tyr Ser Ala Ser Tyr Arg Tyr Thr Gly Val Pro Asp Arg Phe Thr Gly
   50 55 60
   Ser Gly Ser Gly Thr Asp Phe Thr Phe Thr Ile Ser Ser Val Gln Ala
   65 70 75 80
   Glu Asp Leu Ala Val Tyr Tyr Cys Gln Gln Arg Tyr Ser Thr Pro Arg
   85 90 95
   Thr Phe Gly Gly Gly Thr Lys Leu Glu Ile Lys Arg
   100 105
<210> 146
   <211> 11
   <212> PRT
   <213> Mus musculus
<400> 146
   Lys Ala Ser Gln Asp Val Ser Thr Ala Val Ala
   1 5 10
<210> 147
   <211> 7
   <212> PRT
   <213> Artificial Sequence
<220>
   <223> Description of Artificial Sequence: Synthetic variable light chain CDR peptide
<400> 147
   Ser Ala Ser Tyr Arg Tyr Thr
   1 5
<210> 148
   <211> 9
   <212> PRT
   <213> Mus musculus
<400> 148
   Gln Gln Arg Tyr Ser Thr Pro Arg Thr
   1 5
<210> 149
   <211> 115
   <212> PRT
   <213> Mus musculus
<400> 149
   Glu Val Gln Leu Gln Gln Ser Gly Pro Glu Leu Val Lys Pro Gly Ala
   1 5 10 15
   Ser Val Lys Ile Ser Cys Lys Ala Ser Gly Tyr Thr Phe Thr Asp Tyr
   20 25 30
   Tyr Met Asn Trp Val Lys Gln Ser His Gly Lys Ser Leu Glu Trp Ile
   35 40 45
   Gly Asp Ile Asn Pro Asn Asn Gly Gly Thr Thr Tyr Asn Gln Lys Phe
   50 55 60
   Lys Gly Lys Ala Thr Leu Thr Val Asp Lys Ser Tyr Ser Thr Ala Tyr
   65 70 75 80
   Met Glu Leu Arg Ser Leu Thr Ser Glu Asp Ser Ala Val Tyr Tyr Cys
   85 90 95
   Ala Arg Trp Leu Leu Phe Ala Tyr Trp Gly Gln Gly Thr Leu Val Thr
   100 105 110
   Val Ser Ala
   115
<210> 150
   <211> 7
   <212> PRT
   <213> Mus musculus
<400> 150
   Gly Tyr Thr Phe Thr Asp Tyr
   1 5
<210> 151
   <211> 6
   <212> PRT
   <213> Mus musculus
<400> 151
   Asn Pro Asn Asn Gly Gly
   1               5
<210> 152
   <211> 6
   <212> PRT
   <213> Mus musculus
<400> 152
   Trp Leu Leu Phe Ala Tyr
   1               5
<210> 153
   <211> 108
   <212> PRT
   <213> Mus musculus
<400> 153
   Asp Ile Val Met Thr Gln Ser Gln Lys Phe Met Ser Thr Ser Val Gly
   1               5                   10                  15
   Asp Arg Val Ser Val Thr Cys Lys Ala Ser Gln Asn Val Gly Thr Asn
               20                  25                  30
   Val Ala Trp Tyr Gln Gln Lys Pro Gly Gln Ser Pro Lys Ala Leu Ile
           35                  40                  45
   Tyr Ser Ala Ser Tyr Arg Tyr Ser Gly Val Pro Asp Arg Phe Thr Gly
       50                  55                  60
   Ser Gly Ser Gly Thr Asp Phe Thr Leu Thr Ile Ser Asn Val Leu Ser
   65                  70                  75                  80
   Glu Asp Leu Ala Glu Tyr Phe Cys Gln Gln Phe Tyr Ser Tyr Pro Tyr
                   85                  90                  95
   Thr Phe Gly Gly Gly Thr Lys Leu Glu Ile Lys Arg
               100                 105
<210> 154
   <211> 11
   <212> PRT
   <213> Mus musculus
<400> 154
   Lys Ala Ser Gln Asn Val Gly Thr Asn Val Ala
   1               5                   10
<210> 155
   <211> 7
   <212> PRT
   <213> Mus musculus
<400> 155
   Ser Ala Ser Tyr Arg Tyr Ser
   1               5
<210> 156
   <211> 9
   <212> PRT
   <213> Mus musculus
<400> 156
   Gln Gln Phe Tyr Ser Tyr Pro Tyr Thr
   1               5
<210> 157
   <211> 123
   <212> PRT
   <213> Mus musculus
<400> 157
   Glu Val Gln Leu Gln Gln Ser Gly Pro Glu Leu Val Lys Pro Gly Ala
   1               5                   10                  15
   Ser Val Lys Ile Ser Cys Lys Ala Ser Gly Tyr Thr Phe Thr Asp Tyr
               20                  25                  30
   Tyr Met Asn Trp Val Lys Gln Ser His Gly Lys Ser Leu Glu Trp Ile
           35                  40                  45
   Gly Asp Ile Asn Pro Asn Asn Gly Gly Thr Ser Tyr Asn Gln Lys Phe
       50                  55                  60
   Lys Gly Lys Ala Thr Leu Thr Val Asp Lys Ser Ser Ser Thr Ala Tyr
   65                  70                  75                  80
   Met Glu Leu Arg Ser Leu Thr Ser Glu Asp Ser Ala Val Tyr Tyr Cys
                   85                  90                  95
   Ala Gly Gly Gly Ile Tyr Tyr Arg Tyr Asp Arg Asn Tyr Phe Asp Tyr
               100                 105                 110
   Trp Gly Gln Gly Thr Thr Leu Thr Val Ser Ser
           115                 120
<210> 158
   <211> 7
   <212> PRT
   <213> Mus musculus
<400> 158
   Gly Tyr Thr Phe Thr Asp Tyr
   1               5
<210> 159
   <211> 6
   <212> PRT
   <213> Mus musculus
<400> 159
   Asn Pro Asn Asn Gly Gly
   1               5
<210> 160
   <211> 14
   <212> PRT
   <213> Mus musculus
<400> 160
   Gly Gly Ile Tyr Tyr Arg Tyr Asp Arg Asn Tyr Phe Asp Tyr
   1               5                   10
<210> 161
   <211> 107
   <212> PRT
   <213> Mus musculus
<400> 161
   Asp Ile Gln Met Thr Gln Thr Thr Ser Ser Leu Ser Ala Ser Leu Gly
   1               5                   10                  15
   Asp Arg Val Thr Ile Ser Cys Ser Ala Ser Gln Gly Ile Ser Asn Tyr
               20                  25                  30
   Leu Asn Trp Tyr Gln Gln Lys Pro Asp Gly Thr Val Lys Leu Leu Ile
           35                  40                  45
   Tyr Tyr Thr Ser Ser Leu His Ser Gly Val Pro Ser Arg Phe Ser Gly
   50 55 60
   Ser Gly Ser Gly Thr Asp Tyr Ser Leu Thr Ile Ser Asn Leu Glu Pro
   65 70 75 80
   Glu Asp Ile Ala Thr Tyr Tyr Cys Gln Gln Tyr Ser Lys Leu Pro Phe
   85 90 95
   Thr Phe Gly Ser Gly Thr Lys Leu Glu Ile Lys
   100 105
<210> 162
   <211> 11
   <212> PRT
   <213> Mus musculus
<400> 162
   Ser Ala Ser Gln Gly Ile Ser Asn Tyr Leu Asn
   1 5 10
<210> 163
   <211> 7
   <212> PRT
   <213> Mus musculus
<400> 163
   Tyr Thr Ser Ser Leu His Ser
   1 5
<210> 164
   <211> 9
   <212> PRT
   <213> Artificial Sequence
<220>
   <223> Description of Artificial Sequence: Synthetic peptide
<400> 164
   Gln Gln Tyr Ser Lys Leu Pro Phe Thr
   1 5
<210> 165
   <211> 117
   <212> PRT
   <213> Mus musculus
<400> 165
   Glu Val Lys Leu Val Glu Ser Glu Gly Gly Leu Val Gln Pro Gly Ser
   1 5 10 15
   Ser Met Lys Leu Ser Cys Thr Ala Ser Gly Phe Thr Phe Ser Asp Tyr
   20 25 30
   Tyr Met Ala Trp Val Arg Gln Val Pro Glu Lys Gly Leu Glu Trp Val
   35 40 45
   Ala Asn Ile Asn Tyr Asp Gly Ser Asn Thr Ser Tyr Leu Asp Ser Leu
   50 55 60
   Lys Ser Arg Phe Ile Ile Ser Arg Asp Asn Ala Lys Asn Ile Leu Tyr
   65 70 75 80
   Leu Gln Met Ser Ser Leu Lys Ser Glu Asp Thr Ala Thr Tyr Tyr Cys
   85 90 95
   Ala Arg Glu Lys Phe Ala Ala Met Asp Tyr Trp Gly Gln Gly Thr Ser
   100 105 110
   Val Thr Val Ser Ser
   115
<210> 166
   <211> 7
   <212> PRT
   <213> Mus musculus
<400> 166
   Gly Phe Thr Phe Ser Asp Tyr
   1 5
<210> 167
   <211> 6
   <212> PRT
   <213> Mus musculus
<400> 167
   Asn Tyr Asp Gly Ser Asn
   1 5
<210> 168
   <211> 8
   <212> PRT
   <213> Mus musculus
<400> 168
   Glu Lys Phe Ala Ala Met Asp Tyr
   1 5
<210> 169
   <211> 108
   <212> PRT
   <213> Mus musculus
<400> 169
   Asp Ile Val Met Thr Gln Ser His Lys Phe Met Ser Thr Ser Phe Gly
   1               5                   10                  15
   Asp Arg Val Ser Ile Thr Cys Lys Ala Ser Gln Asp Val Ser Asn Ala
               20                  25                  30
   Leu Ala Trp Tyr Gln Gln Lys Pro Gly His Ser Pro Lys Leu Leu Ile
           35                  40                  45
   Phe Ser Ala Ser Tyr Arg Tyr Thr Gly Val Pro Asp Arg Phe Thr Gly
       50                  55                  60
   Ser Gly Ser Gly Thr Asp Phe Thr Phe Thr Ile Ser Ser Val Gln Ala
   65                  70                  75                  80
   Glu Asp Leu Ala Val Tyr Tyr Cys Gln Gln His Tyr Ser Thr Pro Trp
                   85                  90                  95
   Thr Phe Gly Gly Gly Thr Lys Leu Glu Ile Lys Arg
               100                 105
<210> 170
   <211> 11
   <212> PRT
   <213> Mus musculus
<400> 170
   Lys Ala Ser Gln Asp Val Ser Asn Ala Leu Ala
   1               5                   10
<210> 171
   <211> 7
   <212> PRT
   <213> Artificial Sequence
<220>
   <223> Description of Artificial Sequence: Synthetic variable light chain CDR peptide
<400> 171
   Ser Ala Ser Tyr Arg Tyr Thr
   1               5
<210> 172
   <211> 9
   <212> PRT
   <213> Mus musculus
<400> 172
   Gln Gln His Tyr Ser Thr Pro Trp Thr
   1 5
<210> 173
   <211> 121
   <212> PRT
   <213> Artificial Sequence
<220>
   <223> Description of Artificial Sequence: Synthetic polypeptide
<400> 173
   Glu Val Gln Leu Val Glu Ser Gly Gly Gly Leu Val Gln Pro Gly Gly
   1 5 10 15
   Ser Leu Arg Leu Ser Cys Ala Ala Ser Gly Phe Thr Phe Thr Arg His
   20 25 30
   Thr Ile His Trp Val Arg Gln Ala Pro Gly Lys Gly Leu Glu Trp Val
   35 40 45
   Ala Arg Ile Ser Pro Ala Asn Gly Asn Thr Asn Tyr Ala Asp Ser Val
   50 55 60
   Lys Gly Arg Phe Thr Ile Ser Ala Asp Thr Ser Lys Asn Thr Ala Tyr
   65 70 75 80
   Leu Gln Met Asn Ser Leu Arg Ala Glu Asp Thr Ala Val Tyr Tyr Cys
   85 90 95
   Ala Arg Trp Ile Gly Ser Arg Glu Leu Tyr Ile Met Asp Tyr Trp Gly
   100 105 110
   Gln Gly Thr Leu Val Thr Val Ser Ser
   115 120
<210> 174
   <211> 10
   <212> PRT
   <213> Artificial Sequence
<220>
   <223> Description of Artificial Sequence: Synthetic peptide
<400> 174
   Gly Phe Thr Phe Thr Arg His Thr Ile His
   1 5 10
<210> 175
   <211> 17
   <212> PRT
   <213> Artificial Sequence
<220>
   <223> Description of Artificial Sequence: Synthetic peptide
<400> 175
   Arg Ile Ser Pro Ala Asn Gly Asn Thr Asn Tyr Ala Asp Ser Val Lys
   1 5 10 15
   Gly
<210> 176
   <211> 12
   <212> PRT
   <213> Artificial Sequence
<220>
   <223> Description of Artificial Sequence: Synthetic peptide
<400> 176
   Trp Ile Gly Ser Arg Glu Leu Tyr Ile Met Asp Tyr
   1 5 10
<210> 177
   <211> 108
   <212> PRT
   <213> Artificial Sequence
<220>
   <223> Description of Artificial Sequence: Synthetic polypeptide
<400> 177
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Asp Val Ser Thr Ala
               20                  25                  30
   Val Ala Trp Tyr Gln Gln Lys Pro Gly Lys Ala Pro Lys Leu Leu Ile
           35                  40                  45
   Tyr Ser Ala Ser Phe Leu Tyr Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Ser Tyr Arg Ile Gln Pro
                   85                  90                  95
   Thr Phe Gly Gln Gly Thr Lys Val Glu Ile Lys Arg
               100                 105
<210> 178
   <211> 11
   <212> PRT
   <213> Artificial Sequence
<220>
   <223> Description of Artificial Sequence: Synthetic peptide
<400> 178
   Arg Ala Ser Gln Asp Val Ser Thr Ala Val Ala
   1               5                   10
<210> 179
   <211> 7
   <212> PRT
   <213> Artificial Sequence
<220>
   <223> Description of Artificial Sequence: Synthetic peptide
<400> 179
   Ser Ala Ser Phe Leu Tyr Ser
   1               5
<210> 180
   <211> 9
   <212> PRT
   <213> Artificial Sequence
<220>
   <223> Description of Artificial Sequence: Synthetic peptide
<400> 180
   Gln Gln Ser Tyr Arg Ile Gln Pro Thr
   1               5
<210> 181
   <211> 121
   <212> PRT
   <213> Artificial Sequence
<220>
   <223> Description of Artificial Sequence: Synthetic polypeptide
<400> 181
   Glu Val Gln Leu Val Glu Ser Gly Gly Gly Leu Val Gln Pro Gly Gly
   1               5                   10                  15
   Ser Leu Arg Leu Ser Cys Ala Ala Ser Gly Phe Thr Phe Ser Ser Thr
               20                  25                  30
   Ala Ile His Trp Val Arg Gln Ala Pro Gly Lys Gly Leu Glu Trp Val
           35                  40                  45
   Ala Arg Ile Ser Pro Ala Asn Gly Asn Thr Asn Tyr Ala Asp Ser Val
       50                  55                  60
   Lys Gly Arg Phe Thr Ile Ser Ala Asp Thr Ser Lys Asn Thr Ala Tyr
   65                  70                  75                  80
   Leu Gln Met Asn Ser Leu Arg Ala Glu Asp Thr Ala Val Tyr Tyr Cys
                   85                  90                  95
   Ala Arg Trp Ile Gly Ser Arg Glu Leu Tyr Ile Met Asp Tyr Trp Gly
               100                 105                 110
   Gln Gly Thr Leu Val Thr Val Ser Ser
           115                 120
<210> 182
   <211> 10
   <212> PRT
   <213> Artificial Sequence
<220>
   <223> Description of Artificial Sequence: Synthetic peptide
<400> 182
   Gly Phe Thr Phe Ser Ser Thr Ala Ile His
   1               5                   10
<210> 183
   <211> 17
   <212> PRT
   <213> Artificial Sequence
<220>
   <223> Description of Artificial Sequence: Synthetic peptide
<400> 183
   Arg Ile Ser Pro Ala Asn Gly Asn Thr Asn Tyr Ala Asp Ser Val Lys
   1               5                   10                  15
   Gly
<210> 184
   <211> 12
   <212> PRT
   <213> Artificial Sequence
<220>
   <223> Description of Artificial Sequence: Synthetic peptide
<400> 184
   Trp Ile Gly Ser Arg Glu Leu Tyr Ile Met Asp Tyr
   1               5                   10
<210> 185
   <211> 108
   <212> PRT
   <213> Artificial Sequence
<220>
   <223> Description of Artificial Sequence: Synthetic polypeptide
<400> 185
   Asp Ile Gln Met Thr Gln Ser Pro Ser Ser Leu Ser Ala Ser Val Gly
   1               5                   10                  15
   Asp Arg Val Thr Ile Thr Cys Arg Ala Ser Gln Asp Val Ser Thr Ala
               20                  25                  30
   Val Ala Trp Tyr Gln Gln Lys Pro Gly Lys Ala Pro Lys Leu Leu Ile
           35                  40                  45
   Tyr Ser Ala Ser Phe Leu Tyr Ser Gly Val Pro Ser Arg Phe Ser Gly
       50                  55                  60
   Ser Gly Ser Gly Thr Asp Phe Thr Leu Thr Ile Ser Ser Leu Gln Pro
   65                  70                  75                  80
   Glu Asp Phe Ala Thr Tyr Tyr Cys Gln Gln Ser Tyr Pro Ala Leu His
                   85                  90                  95
   Thr Phe Gly Gln Gly Thr Lys Val Glu Ile Lys Arg
               100                 105
<210> 186
   <211> 11
   <212> PRT
   <213> Artificial Sequence
<220>
   <223> Description of Artificial Sequence: Synthetic peptide
<400> 186
   Arg Ala Ser Gln Asp Val Ser Thr Ala Val Ala
   1               5                   10
<210> 187
   <211> 7
   <212> PRT
   <213> Artificial Sequence
<220>
   <223> Description of Artificial Sequence: Synthetic peptide
<400> 187
   Ser Ala Ser Phe Leu Tyr Ser
   1               5
<210> 188
   <211> 9
   <212> PRT
   <213> Artificial Sequence
<220>
   <223> Description of Artificial Sequence: Synthetic peptide
<400> 188
   Gln Gln Ser Tyr Pro Ala Leu His Thr
   1               5
<210> 189
   <211> 125
   <212> PRT
   <213> Artificial Sequence
<220>
   <223> Description of Artificial Sequence: Synthetic polypeptide
<400> 189
   Glu Val Gln Leu Val Glu Ser Gly Gly Gly Leu Val Lys Pro Gly Gly
   1               5                   10                  15
   Ser Leu Arg Leu Ser Cys Ala Ala Ser Gly Phe Pro Phe Ser Lys Leu
               20                  25                  30
   Gly Met Val Trp Val Arg Gln Ala Pro Gly Lys Gly Leu Glu Trp Val
           35                  40                  45
   Ser Thr Ile Ser Ser Gly Gly Gly Tyr Thr Tyr Tyr Pro Asp Ser Val
       50                  55                  60
   Lys Gly Arg Phe Thr Ile Ser Arg Asp Asn Ala Lys Asn Ser Leu Tyr
   65                  70                  75                  80
   Leu Gln Met Asn Ser Leu Arg Ala Glu Asp Thr Ala Val Tyr Tyr Cys
                   85                  90                  95
   Ala Arg Glu Gly Ile Ser Phe Gln Gly Gly Thr Tyr Thr Tyr Val Met
               100                 105                 110
   Asp Tyr Trp Gly Gln Gly Thr Leu Val Thr Val Ser Ser
           115                 120                 125
<210> 190
   <211> 10
   <212> PRT
   <213> Artificial Sequence
<220>
   <223> Description of Artificial Sequence: Synthetic peptide
<400> 190
   Gly Phe Pro Phe Ser Lys Leu Gly Met Val
   1 5 10
<210> 191
   <211> 17
   <212> PRT
   <213> Artificial Sequence
<220>
   <223> Description of Artificial Sequence: Synthetic peptide
<400> 191
   Thr Ile Ser Ser Gly Gly Gly Tyr Thr Tyr Tyr Pro Asp Ser Val Lys
   1 5 10 15
   Gly
<210> 192
   <211> 16
   <212> PRT
   <213> Artificial Sequence
<220>
   <223> Description of Artificial Sequence: Synthetic peptide
<400> 192
   Glu Gly Ile Ser Phe Gln Gly Gly Thr Tyr Thr Tyr Val Met Asp Tyr
   1 5 10 15
<210> 193
   <211> 112
   <212> PRT
   <213> Artificial Sequence
<220>
   <223> Description of Artificial Sequence: Synthetic polypeptide
<400> 193
   Asp Ile Val Met Thr Gln Ser Pro Leu Ser Leu Pro Val Thr Pro Gly
   1 5 10 15
   Glu Pro Ala Ser Ile Ser Cys Arg Ser Ser Lys Ser Leu Leu His Arg
   20 25 30
   Asn Gly Ile Thr Tyr Ser Tyr Trp Tyr Leu Gln Lys Pro Gly Gln Ser
   35 40 45
   Pro Gln Leu Leu Ile Tyr Gln Leu Ser Asn Leu Ala Ser Gly Val Pro
   50 55 60
   Asp Arg Phe Ser Gly Ser Gly Ser Gly Thr Asp Phe Thr Leu Lys Ile
   65 70 75 80
   Ser Arg Val Glu Ala Glu Asp Val Gly Val Tyr Tyr Cys Tyr Gln Asn
   85 90 95
   Leu Glu Leu Pro Leu Thr Phe Gly Gln Gly Thr Lys Val Glu Ile Lys
   100 105 110
<210> 194
   <211> 16
   <212> PRT
   <213> Artificial Sequence
<220>
   <223> Description of Artificial Sequence: Synthetic peptide
<400> 194
   Arg Ser Ser Lys Ser Leu Leu His Arg Asn Gly Ile Thr Tyr Ser Tyr
   1 5 10 15
<210> 195
   <211> 7
   <212> PRT
   <213> Artificial Sequence
<220>
   <223> Description of Artificial Sequence: Synthetic peptide
<400> 195
   Gln Leu Ser Asn Leu Ala Ser
   1 5
<210> 196
   <211> 9
   <212> PRT
   <213> Artificial Sequence
<220>
   <223> Description of Artificial Sequence: Synthetic peptide
<400> 196
   Tyr Gln Asn Leu Glu Leu Pro Leu Thr
   1 5
<210> 197
   <211> 2076
   <212> DNA
   <213> Homo sapiens
<400> 197
   atgggcaccg tcagctccag gcggtcctgg tggccgctgc cactgctgct gctgctgctg 60
   ctgctcctgg gtcccgcggg cgcccgtgcg caggaggacg aggacggcga ctacgaggag 120
   ctggtgctag ccttgcgttc cgaggaggac ggcctggccg aagcacccga gcacggaacc 180
   acagccacct tccaccgctg cgccaaggat ccgtggaggt tgcctggcac ctacgtggtg 240
   gtgctgaagg aggagaccca cctctcgcag tcagagcgca ctgcccgccg cctgcaggcc 300
   caggctgccc gccggggata cctcaccaag atcctgcatg tcttccatgg ccttcttcct 360
   ggcttcctgg tgaagatgag tggcgacctg ctggagctgg ccttgaagtt gccccatgtc 420
   gactacatcg aggaggactc ctctgtcttt gcccagagca tcccgtggaa cctggagcgg 480
   attacccctc cacggtaccg ggcggatgaa taccagcccc ccgacggagg cagcctggtg 540
   gaggtgtatc tcctagacac cagcatacag agtgaccacc gggaaatcga gggcagggtc 600
   atggtcaccg acttcgagaa tgtgcccgag gaggacggga cccgcttcca cagacaggcc 660
   agcaagtgtg acagtcatgg cacccacctg gcaggggtgg tcagcggccg ggatgccggc 720
   gtggccaagg gtgccagcat gcgcagcctg cgcgtgctca actgccaagg gaagggcacg 780
   gttagcggca ccctcatagg cctggagttt attcggaaaa gccagctggt ccagcctgtg 840
   gggccactgg tggtgctgct gcccctggcg ggtgggtaca gccgcgtcct caacgccgcc 900
   tgccagcgcc tggcgagggc tggggtcgtg ctggtcaccg ctgccggcaa cttccgggac 960
   gatgcctgcc tctactcccc agcctcagct cccgaggtca tcacagttgg ggccaccaat 1020
   gcccaagacc agccggtgac cctggggact ttggggacca actttggccg ctgtgtggac 1080
   ctctttgccc caggggagga catcattggt gcctccagcg actgcagcac ctgctttgtg 1140
   tcacagagtg ggacatcaca ggctgctgcc cacgtggctg gcattgcagc catgatgctg 1200
   tctgccgagc cggagctcac cctggccgag ttgaggcaga gactgatcca cttctctgcc 1260
   aaagatgtca tcaatgaggc ctggttccct gaggaccagc gggtactgac ccccaacctg 1320
   gtggccgccc tgccccccag cacccatggg gcaggttggc agctgttttg caggactgta 1380
   tggtcagcac actcggggcc tacacggatg gccacagccg tcgcccgctg cgccccagat 1440
   gaggagctgc tgagctgctc cagtttctcc aggagtggga agcggcgggg cgagcgcatg 1500
   gaggcccaag ggggcaagct ggtctgccgg gcccacaacg cttttggggg tgagggtgtc 1560
   tacgccattg ccaggtgctg cctgctaccc caggccaact gcagcgtcca cacagctcca 1620
   ccagctgagg ccagcatggg gacccgtgtc cactgccacc aacagggcca cgtcctcaca 1680
   ggctgcagct cccactggga ggtggaggac cttggcaccc acaagccgcc tgtgctgagg 1740
   ccacgaggtc agcccaacca gtgcgtgggc cacagggagg ccagcatcca cgcttcctgc 1800
   tgccatgccc caggtctgga atgcaaagtc aaggagcatg gaatcccggc ccctcaggag 1860
   caggtgaccg tggcctgcga ggagggctgg accctgactg gctgcagtgc cctccctggg 1920
   acctcccacg tcctgggggc ctacgccgta gacaacacgt gtgtagtcag gagccgggac 1980
   gtcagcacta caggcagcac cagcgaaggg gccgtgacag ccgttgccat ctgctgccgg 2040
   agccggcacc tggcgcaggc ctcccaggag ctccag 2076
<210> 198
   <211> 692
   <212> PRT
   <213> Homo sapiens
<400> 198